# EUROPÄISCHE PATENTSCHRIFT

(11) **EP 1 140 921 B1**
(45) Veröffentlichungstag und Bekanntmachung des Hinweises auf die Patenterteilung: **02.04.2003**
(21) Anmeldenummer: 99959375.9
(22) Anmeldetag: 03.12.1999
(51) Int. Cl.: C07D 413/04, A01N 43/76, C07D 413/14

(54) **4-TRIFLUORMETHYL-3-OXAZOLYLPYRIDINE, VERFAHREN ZU IHRER HERSTELLUNG, SIE ENTHALTENDE MITTEL UND IHRE VERWENDUNG ALS SCHÄDLINGSBEKÄMPFUNGSMITTEL**
4-TRIFLUOROMETHYL-3-OXAZOLYLPYRIDINES, METHOD FOR PRODUCING THEM, PRODUCTS CONTAINING THE SAME, AND THEIR USE AS PESTICIDES
4-TRIFLUOROMETHYL-3-OXAZOLYLPYRIDINES, LEUR PROCEDE DE PRODUCTION, LES PRODUITS LES CONTENANT ET LEUR UTILISATION COMME PESTICIDES

(30) Priorität: 17.12.1998 DE 19858192
(43) Veröffentlichungstag der Anmeldung: 10.10.2001
(73) Patentinhaber: Bayer CropScience GmbH, 65929 Frankfurt/Main (DE)
(72) Erfinder: BASTIAANS, Henricus, Maria, Martinus, D-61250 Usingen (DE); TIEBES, Jörg, D-60320 Frankfurt (DE); JANS, Daniela, D-61348 Bad Homburg v. d. H. (DE); HEMPEL, Waltraud, D-65835 Liederbach (DE); SANFT, Ulrich, D-65817 Eppstein/Ts. (DE); THÖNESSEN, Maria-Theresia, D-55262 Heidesheim (DE)
(86) Internationale Anmeldenummer: EP9909441
(87) Internationale Veröffentlichungsnummer: WO00035912

(56) Entgegenhaltungen:
- WO-A-95/07891
- WO-A-99/65901
- DE-A- 19 725 450

## Beschreibung

Die Erfindung betrifft 4-Trifluormethyl-3-oxazolylpyridine, ein Verfahren zu ihrer Herstellung, sie enthaltende Mittel sowie deren Verwendung zur Bekämpfung von tierischen Schädlingen, insbesondere Insekten, Spinnmilben, Ektoparasiten und Helminthen.

Es ist bereits bekannt, daß geeignet substituierte Pyridine akarizide und insektizide Wirkung zeigen. So sind in WO 95/07891 Pyridine beschrieben, die in 4-Position einen über ein Heteroatom verknüpften Cycloalkylrest und in 3-Position eine Gruppe unterschiedlicher Substituenten tragen. Jedoch ist die gewünschte Wirkung gegenüber Schadorganismen nicht immer ausreichend. Darüber hinaus weisen diese Verbindungen oftmals unerwünschte toxikologische Eigenschaften gegenüber Säugetieren und aquatischen Lebewesen auf.

In der nicht vorveröffentlichten internationalen Anmeldung WO-A 98/57969 sind 4-Haloalkyl-3-heterocylylpyridine und -pyrimidine zur Verwendung als Schädlingsbekämpfungsmittel vorgeschlagen.

Aufgabe der vorliegenden Erfindung war die Bereitstellung von Verbindungen mit guten insektiziden und akariziden Eigenschaften bei gleichzeitig geringer Toxizität gegenüber Säugetieren und aquatischen Lebewesen.

Es wurde nun gefunden, daß Verbindungen der allgemeinen Formel (I), gegebenenfalls auch als Salze, im Vergleich zu den bereits bekannten Verbindungen ein gutes Wirkungsspektrum gegenüber tierischen Schädlingen bei gleichzeitig sehr günstigen toxikologischen Eigenschaften gegenüber Säugetieren und aquatischen Lebewesen aufweisen.

Gegenstand der Erfindung sind daher Verbindungen der Formel (I), 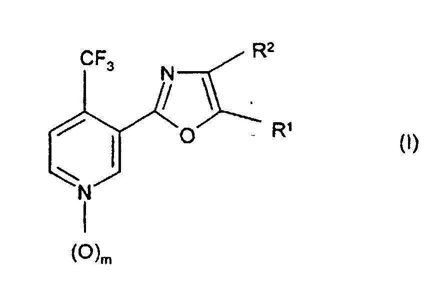 wobei die Symbole und Indizes folgende Bedeutungen haben:
m ist 0 oder 1;
R¹ und R² sind
   a) H, CH₃, -C₂H₅, -CH₂-CH₂-CH₃, -CH(CH₃)₂ oder Cyclopropyl oder
   b) -CH₃, -CH₂XR³, -CHY, -CO₂R⁴ oder -CONR⁵R⁶,
   wobei jeweils einer der Reste R¹, R² aus der Gruppe a und der andere aus der Gruppe b ist;

X ist O, S, SO, SO₂ oder NR⁷ ;
Y ist O, Br₂, -O-(CH₂)₂-O-, ((C₁-C₄)-Alkoxy)₂, ((C₁-C₄)-Alkylthio)₂, V-(CH₂)_{2 oder 3}-V, mit V = O, S, wobei ein H-Atom gegebenenfalls durch (C₁-C₄) Alkyl ersetzt ist;
R³ ist R⁸, COR⁹, CO₂R¹⁰, CONR¹¹R¹² oder, falls X O oder NR⁷ ist, SO₂R¹³;
R⁴, R⁵, R⁶, R⁷, R⁸, R⁹, R¹⁰, R¹¹, R¹², R¹³ sind gleich oder verschieden unabhängig voneinander H, (C₁-C₁₀)-Alkyl, (C₂-C₁₀)-Alkenyl, (C₂-C₁₀)-Alkinyl, (C₃-C₈)-Cycloalkyl, (C₄-C₈)-Cycloalkenyl, (C₆-C₈)-Cycloalkinyl, Aryl oder Heterocyclyl, wobei jede der acht letztgenannten Gruppen gegebenenfalls ein oder mehrfach substituiert ist, und wobei gegebenenfalls jeweils R⁵ und R⁶ sowie R¹¹ und R¹² zusammen -(CH₂)₄-, -(CH₂)₅-, -(CH₂)₂-O-(CH₂)₂- oder -(CH₂)₂-NR⁴-(CH₂)₂ sind; mit der Maßgabe, daß die Verbindungen, in denen
   R¹ = CO₂C₂H₅ und R² = H,
   R¹ = H und R² = CH₂NHC₆H₅,
   R¹ = CH₃ und R² = CO₂H,
   R¹ = CH₃ und R² = CO₂C₂H₅,
   R¹ = CH₃ und R² = CON(CH₃)₂,
   R¹ = CH(CH₃)₂ und R² = CO₂H,
   R¹ = CH(CH₃)₂ und R² = CO₂C₂H₅
bedeuten, ausgenommen sind.

Bevorzugte Verbindungen der Formel (I) sind zum Beispiel
1. Verbindungen, in denen R¹ H und R² einen Rest aus der Gruppe b bedeutet;
2. Verbindungen, in denen R¹ einen Rest aus der Gruppe b und R² H bedeutet,
3. Verbindungen, in denen R¹ einen Rest aus der Gruppe a, außer Wasserstoff, und R² einen Rest aus der Gruppe b bedeutet.

m ist bevorzugt 0.

Im Falle von m = 1 und R¹ oder R² = CH₂S(O)ₙR³ ist n bevorzugt 2.

Aus der Gruppe a sind bevorzugt H, CH₃ und Cyclopropyl. Aus der Gruppe b sind bevorzugt CH₂XR³ und -CONR⁵R⁶.

R^{4... 13} sind bevorzugt
a) H, (C₁-C₁₀)-Alkyl, (C₂-C₁₀)-Alkenyl, (C₂-C₁₀)-Alkinyl, (C₃-C₈)-Cycloalkyl, (C₄-C₈)-Cycloalkenyl, (C₆-C₈)-Cycloalkinyl, wobei die sechs zuletzt genannten Reste gegebenenfalls mit einem oder mehreren Resten aus der Gruppe: Halogen, Cyano, Nitro, Hydroxy, -C(=W)R¹⁴, -C(=NOR¹⁰)R¹⁴, -C(=NNR¹⁴₂)R¹⁴, -C(=W)OR¹⁴, -C(=W)NR¹⁴₂, -OC(=W)R¹⁴, -OC(=W)OR¹⁴, -NR⁵C(=W)R¹⁴, N[C(=W)R¹⁴]₂, -NR¹⁴C(=W)OR¹⁴, -C(=W)NR¹⁴-NR¹⁴₂, -C(=W)NR¹⁴-NR¹⁴[C(=W)R¹⁵], -NR¹⁴-C(=W)NR¹⁴₂, -NR¹⁴-NR¹⁴C(=W)R¹⁴, -NR¹⁴-N[C(=W)R¹⁴]₂, -N[(C=W)R¹⁴]-NR¹⁴₂, -NR¹⁴-NR¹⁴[(C=W)WR¹⁴], -NR¹⁴(C=NR¹⁴)R¹⁴, -NR¹⁴(C=NR¹⁴)NR¹⁴₂, -O-NR¹⁴₂, -O-NR¹⁴(C=W)R¹⁴, -SO₂NR¹⁴₂, -NR¹⁴SO₂R¹⁴, -SO₂OR¹⁴, -OSO₂R¹⁴, -OR¹⁴, -NR¹⁴₂, -SR¹⁴, -SiR¹⁴₃, -SeR¹⁴, -PR¹⁴₂, -P(=W)R¹⁴₂, -SOR¹⁴, -SO₂R¹⁴, -PW₂R¹⁴₂, -PW₃R¹⁴₂, Aryl und Heterocyclyl,
   von denen die beiden letztgenannten Reste gegebenenfalls mit einem oder mehreren Resten aus der Gruppe (C₁-C₆)-Alkyl, (C₂-C₆)-Alkenyl, (C₂-C₆)-Alkinyl, (C₃-C₈)-Cycloalkyl, (C₄-C₈)-Cycloalkenyl, (C₆-C₈)-Cycloalkinyl, (C₁-C₆)-Haloalkyl, (C₂-C₆)-Haloalkenyl, (C₂-C₆)-Haloalkinyl, Halogen, -OR¹⁴, -NR¹⁴₂, -SR¹⁴, -SiR¹⁴₃, -C(=W)R¹⁴, -C(=W)OR¹⁴, -C(=W)NR¹⁴₂, -SOR¹⁴, -SO₂R¹⁴, Nitro, Cyano und Hydroxy substituiert sind,
   substituiert sind,
b) Aryl,
   welches gegebenenfalls mit einem oder mehreren Resten aus der Gruppe (C₁-C₆)-Alkyl, (C₂-C₆)-Alkenyl, (C₂-C₆)-Alkinyl, (C₃-C₈)-Cycloalkyl, (C₄-C₈)-Cycloalkenyl und (C₆-C₈)-Cycloalkinyl,
   wobei diese sechs vorstehend genannten Reste gegebenenfalls durch einen oder mehrere Reste aus der Gruppe Halogen, Cyano, Nitro, -C(=W)R¹⁴, -C(=W)OR¹⁴, -C(=W)NR¹⁴₂, -OR¹⁴, -NR¹⁴₂, -SR¹⁴,, -SOR¹⁴ und -SO₂R¹⁴ substituiert sind,
   Halogen, Cyano, Nitro, -C(=W)R¹⁴, -C(=NOR¹⁰)R¹⁴, -C(=NNR¹⁴₂)R¹⁴, -C(=W)OR¹⁴, -C(=W)NR¹⁴₂, -OC(=W)R¹⁴, -OC(=W)OR¹⁴, -NR¹⁴C(=W)R¹⁴, -[C(=W)R¹⁴]₂, -NR¹⁴C(=W)OR¹⁴, -OR¹⁴, -NR¹⁴₂, -SR¹⁴, -SiR¹⁴₃, -PR¹⁴₂,-SOR¹⁴, -SO₂R¹⁴, -PW₂R¹⁴₂ und -PW₃R¹⁴₂ substituiert ist,
c) Heterocyclyl,
   welches gegebenenfalls mit einem oder mehreren Resten aus der Gruppe (C₁-C₆)-Alkyl, (C₂-C₆)-Alkenyl, (C₂-C₆)-Alkinyl, (C₃-C₈)-Cycloalkyl, (C₄-C₈)-Cycloalkenyl und (C₆-C₈)-Cycloalkinyl,
wobei diese sechs vorstehend genannten Reste gegebenenfalls durch einen oder mehrere Reste aus der Gruppe Halogen, Cyano, Nitro, -C(=W)R¹⁴, -C(=W)OR¹⁴, -C(=W)NR¹⁴₂, -OR¹⁴, -NR¹⁴₂, -SR¹⁴,, -SOR¹⁴ und -SO₂R¹⁴ substituiert sind,
Halogen, Cyano, Nitro, -C(=W)R¹⁴, -C(=NOR¹⁰)R¹⁴, -C(=NNR¹⁴₂)R¹⁴, -C(=W)OR¹⁴, -C(=W)NR¹⁴₂, -OC(=W)R¹⁴, -OC(=W)OR¹⁴, -NR¹⁴C(=W)R¹⁴,-[C(=W)R¹⁴]₂, -NR¹⁴C(=W)OR¹⁴, -OR¹⁴, NR¹⁴₂, -SR¹⁴, SiR¹⁴₃, -PR¹⁴₂, -SOR¹⁴,
-SO₂R¹⁴, -PW₂R¹⁴₂ und -PW₃R¹⁴₂ substituiert ist,
- W ist: O oder S;
- R¹⁴ ist: gleich oder verschieden Wasserstoff,
(C₁-C₆)-Alkyl, (C₂-C₆)-Alkenyl, (C₂-C₆)-Alkinyl, (C₃-C₈)-Cycloalkyl, (C₄-C₈)-Cycloalkenyl, (C₃-C₈)-Cycloalkyl-(C₁-C₄)-Alkyl, (C₄-C₈)-Cycloalkenyl-(C₁-C₄)-Alkyl, (C₃-C₈)-Cycloalkyl-(C₂-C₄)-Alkenyl, (C₄-C₈)-Cycloalkenyl-(C₁-C₄)-Alkenyl, (C₁-C₆)-Alkyl-(C₃-C₈)-Cycloalkyl, (C₂-C₆)-Alkenyl-(C₃-C₈)-Cycloalkyl, (C₂-C₆)-Alkinyl-(C₃-C₈)-Cycloalkyl, (C₁-C₆)-Alkyl-(C₄-C₈)-Cycloalkenyl, (C₂-C₆)-Alkenyl-(C₄-C₈)-Cycloalkenyl, wobei die vierzehn letztgenannten Reste gegebenenfalls mit einem oder mehreren, vorzugsweise 1 bis 3, Resten aus der Gruppe Halogen, Cyano, Nitro, Hydroxy, Thio, Amino, Formyl, (C₁-C₆)-Alkoxy, (C₂-C₆)-Alkenyloxy, (C₂-C₆)-Alkinyloxy, (C₁-C₆)-Haloalkyloxy, (C₂-C₆)-Haloalkenyloxy, (C₂-C₆)-Haloalkinyloxy, (C₃-C₈)-Cycloalkoxy, (C₄-C₈)-Cycloalkenyloxy, (C₃-C₈)-Halocycloalkoxy, (C₄-C₈)-Halocycloalkenyloxy, (C₃-C₈)-Cycloalkyl-(C₁-C₄)-Alkoxy, (C₄-C₈)-Cycloalkenyl-(C₁-C₄)-Alkoxy, (C₃-C₈)-Cycloalkyl-(C₂-C₄)-Alkenyloxy, (C₄-C₈)-Cycloalkenyl-(C₁-C₄)-Alkenyloxy, (C₁-C₆)-Alkyl-(C₃-C₈)-Cycloalkoxy, (C₂-C₆)-Alkenyl-(C₃-C₈)-Cycloalkoxy, (C₂-C₆)-Alkinyl-(C₃-C₈)-Cycloalkoxy, (C₁-C₆)-Alkyl-(C₄-C₈)-Cycloalkenyloxy, (C₂-C₆)-Alkenyl-(C₄-C₈)-Cycloalkenyloxy, (C₁-C₄)-Alkoxy-(C₁-C₆)-Alkoxy, (C₁-C₄)-Alkoxy-(C₂-C₆)-Alkenyloxy, Carbamoyl, (C₁-C₆)-Mono- oder Dialkylcarbamoyl, (C₁-C₆)-Mono- oder Dihaloalkylcarbamoyl, (C₃-C₈)-Monooder Dicycloalkylcarbamoyl, (C₁-C₆)-Alkoxycarbonyl, (C₃-C₈)-Cycloalkoxycarbonyl, (C₁-C₆)-Alkanoyloxy, (C₃-C₈)-Cycloalkanoyloxy, (C₁-C₆)-Haloalkoxycarbonyl, (C₁-C₆)-Haloalkanoyloxy, (C₁-C₆)-Alkanamido, (C₁-C₆)-Haloalkanamido, (C₂-C₆)-Alkenamido, (C₃-C₈)-Cycloalkanamido, (C₃-C₈)-Cycloalkyl-(C₁-C₄)-Alkanamido, (C₁-C₆)-Alkylthio, (C₂-C₆)-Alkenylthio, (C₂-C₆)-Alkinylthio, (C₁-C₆)-Haloalkylthio, (C₂-C₆)-Haloalkenylthio, (C₂-C₆)-Haloalkinylthio, (C₃-C₈)-Cycloalkylthio, (C₄-C₈)-Cycloalkenylthio,
(C₃-C₈)-Halocycloalkthio, (C₄-C₈)-Halocycloalkenylthio, (C₃-C₈)-Cycloalkyl-(C₁-C₄)-Alkylthio, (C₄-C₈)-Cycloalkenyl-(C₁-C₄)-Alkylthio, (C₃-C₈)-Cycloalkyl-(C₂-C₄)-Alkenylthio, (C₄-C₈)-Cycloalkenyl-(C₁-C₄)-Alkenylthio, (C₁-C₆)-Alkyl-(C₃-C₈)-Cycloalkylthio, (C₂-C₆)-Alkenyl-(C₃-C₈)-Cycloalkylthio, (C₂-C₆)-Alkinyl-(C₃-C₈)-Cycloalkylthio, (C₁-C₆)-Alkyl-(C₄-C₈)-Cycloalkenylthio, (C₂-C₆)-Alkenyl-(C₄-C₈)-Cycloalkenylthio, (C₁-C₆)-Alkylsulfinyl, (C₂-C₆)-Alkenylsulfinyl, (C₂-C₆)-Alkinylsulfinyl, (C₁-C₆)-Haloalkylsulfinyl, (C₂-C₆)-Haloalkenylsulfinyl, (C₂-C₆)-Haloalkinylsulfinyl, (C₃-C₈)-Cycloalkylsulfinyl, (C₄-C₈)-Cycloalkenylsulfinyl, (C₃-C₈)-Halocycloalksulfinyl, (C₄-C₈)-Halocycloalkenylsulfinyl, (C₃-C₈)-Cycloalkyl-(C₁-C₄)-Alkylsulfinyl, (C₄-C₈)-Cycloalkenyl-(C₁-C₄)-Alkylsulfinyl, (C₃-C₈)-Cycloalkyl-(C₂-C₄)-Alkenylsulfinyl, (C₄-C₈)-Cycloalkenyl-(C₁-C₄)-Alkenylsulfinyl, (C₁-C₆)-Alkyl-(C₃-C₈)-Cycloalkylsulfinyl, (C₂-C₆)-Alkenyl-(C₃-C₈)-Cycloalkylsulfinyl, (C₂-C₆)-Alkinyl-(C₃-C₈)-Cycloalkylsulfinyl, (C₁-C₆)-Alkyl-(C₄-C₈)-Cycloalkenylsulfinyl, (C₂-C₆)-Alkenyl-(C₄-C₈)-Cycloalkenylsulfinyl, (C₁-C₆)-Alkylsulfonyl, (C₂-C₆)-Alkenylsulfonyl, (C₂-C₆)-Alkinylsulfonyl, (C₁-C₆)-Haloalkylsulfonyl, (C₂-C₆)-Haloalkenylsulfonyl, (C₂-C₆)-Haloalkinylsulfonyl, (C₃-C₈)-Cycloalkylsulfonyl, (C₄-C₈)-Cycloalkenylsulfonyl, (C₃-C₈)-Halocycloalksulfonyl, (C₄-C₈)-Halocycloalkenylsulfonyl, (C₃-C₈)-Cycloalkyl-(C₁-C₄)-Alkylsulfonyl, (C₄-C₈)-Cycloalkenyl-(C₁-C₄)-Alkylsulfonyl, (C₃-C₈)-Cycloalkyl-(C₂-C₄)-Alkenylsulfonyl, (C₄-C₈)-Cycloalkenyl-(C₁-C₄)-Alkenylsulfonyl, (C₁-C₆)-Alkyl-(C₃-C₈)-Cycloalkylsulfonyl, (C₂-C₆)-Alkenyl-(C₃-C₈)-Cycloalkylsulfonyl, (C₂-C₆)-Alkinyl-(C₃-C₈)-Cycloalkylsulfonyl, (C₁-C₆)-Alkyl-(C₄-C₈)-Cycloalkenylsulfonyl, (C₂-C₆)-Alkenyl-(C₄-C₈)-Cycloalkenylsulfonyl, (C₁-C₆)-Alkylamino, (C₂-C₆)-Alkenylamino, (C₂-C₆)-Alkinylamino, (C₁-C₆)-Haloalkylamino, (C₂-C₆)-Haloalkenylamino, (C₂-C₆)-Haloalkinylamino, (C₃-C₈)-Cycloalkylamino, (C₄-C₈)-Cycloalkenylamino, (C₃-C₈)-Halocycloalkamino, (C₄-C₈)-Halocycloalkenylamino, (C₃-C₈)-Cycloalkyl-(C₁-C₄)-Alkylamino, (C₄-C₈)-Cycloalkenyl-(C₁-C₄)-Alkylamino, (C₃-C₈)-Cycloalkyl-(C₂-C₄)-Alkenylamino, (C₄-C₈)-Cycloalkenyl-(C₁-C₄)-Alkenylamino, (C₁-C₆)-Alkyl-(C₃-C₈)-Cycloalkylamino, (C₂-C₆)-Alkenyl-(C₃-C₈)-Cycloalkylamino, (C₂-C₆)-Alkinyl-(C₃-C₈)-Cycloalkylamino, (C₁-C₆)-Alkyl-(C₄-C₈)-Cycloalkenylamino, (C₂-C₆)-Alkenyl-(C₄-C₈)-Cycloalkenylamino, (C₁-C₆)-Trialkylsilyl, Aryl, Aryloxy, Arylthio, Arylamino, Aryl-(C₁-C₄)-Alkoxy, Aryl-(C₂-C₄)-Alkenyloxy, Aryl-(C₁-C₄)-Alkylthio, Aryl-(C₂-C₄)-Alkenylthio, Aryl-(C₁-C₄)-Alkylamino, Aryl-(C₂-C₄)-Alkenylamino, Aryl-(C₁-C₆)-Dialkylsilyl, Diaryl-(C₁-C₆)-Alkylsilyl, Triarylsilyl und 5- oder 6-gliedriges Heterocyclyl,
wobei der cyclische Teil der vierzehn letztgenannten Reste gegebenenfalls durch einen oder mehrere Reste aus der Gruppe Halogen, Cyano, Nitro, Amino, Hydroxy, Thio, (C₁-C₄)-Alkyl, (C₁-C₄)-Haloalkyl, (C₃-C₈)-Cycloalkyl, (C₁-C₄)-Alkoxy, (C₁-C₄)-Haloalkoxy, (C₁-C₄)-Alkylthio, (C₁-C₄)-Haloalkylthio, (C₁-C₄)-Alkylamino, (C₁-C₄)-Haloalkylamino, Formyl und (C₁-C₄)-Alkanoyl substituiert ist;
substituiert sind,

Aryl, 5- oder 6-gliedriger Heteroaromat, wobei die beiden letztgenannten Reste gegebenenfalls durch einen oder mehrere Reste aus der Gruppe
Halogen, Cyano, Nitro, Hydroxy, Thio, Amino, Formyl, (C₁-C₆)-Alkoxy, (C₂-C₆)-Alkenyloxy, (C₂-C₆)-Alkinyloxy, (C₁-C₆)-Haloalkyloxy, (C₂-C₆)-Haloalkenyloxy, (C₂-C₆)-Haloalkinyloxy, (C₃-C₈)-Cycloalkoxy, (C₄-C₈)-Cycloalkenyloxy, (C₃-C₈)-Halocycloalkoxy, (C₄-C₈)-Halocycloalkenyloxy, Carbamoyl, (C₁-C₆)-Mono- oder Dialkylcarbamoyl, (C₁-C₆)-Alkoxycarbonyl, (C₁-C₆)-Alkanoyloxy, (C₁-C₆)-Mono- oder Dihaloalkylcarbamoyl, (C₁-C₆)-Haloalkoxycarbonyl, (C₁-C₆)-Haloalkanoyloxy, (C₁-C₆)-Alkanamido, (C₁-C₆)-Haloalkanamido, (C₂-C₆)-Alkenamido, (C₁-C₆)-Alkylthio, (C₂-C₆)-Alkenylthio, (C₂-C₆)-Alkinylthio, (C₁-C₆)-Haloalkylthio, (C₂-C₆)-Haloalkenylthio, (C₂-C₆)-Haloalkinylthio, (C₃-C₈)-Cycloalkylthio, (C₄-C₈)-Cycloalkenylthio, (C₃-C₈)-Halocycloalkthio, (C₄-C₈)-Halocycloalkenylthio, (C₁-C₆)-Alkylsulfinyl, (C₂-C₆)-Alkenylsulfinyl, (C₂-C₆)-Alkinylsulfinyl, (C₁-C₆)-Haloalkylsulfinyl, (C₂-C₆)-Haloalkenylsulfinyl, (C₂-C₆)-Haloalkinylsulfinyl, (C₃-C₈)-Cycloalkylsulfinyl, (C₄-C₈)-Cycloalkenylsulfinyl, (C₃-C₈)-Halocycloalksulfinyl, (C₄-C₈)-Halocycloalkenylsulfinyl, (C₁-C₆)-Alkylsulfonyl, (C₂-C₆)-Alkenylsulfonyl, (C₂-C₆)-Alkinylsulfonyl, (C₁-C₆)-Haloalkylsulfonyl, (C₂-C₆)-Haloalkenylsulfonyl, (C₂-C₆)-Haloalkinylsulfonyl, (C₃-C₈)-Cycloalkylsulfonyl, (C₄-C₈)-Cycloalkenylsulfonyl, (C₃-C₈)-Halocycloalksulfonyl, (C₄-C₈)-Halocycloalkenylsulfonyl, (C₁-C₆)-Alkylamino, (C₂-C₆)-Alkenylamino, (C₂-C₆)-Alkinylamino, (C₁-C₆)-Haloalkylamino, (C₂-C₆)-Haloalkenylamino, (C₂-C₆)-Haloalkinylamino, (C₃-C₈)-Cycloalkylamino, (C₄-C₈)-Cycloalkenylamino, (C₃-C₈)-Halocycloalkamino und (C₄-C₈)-Halocycloalkenylamino
substituiert sind.
- R^{4....14}: sind besonders bevorzugt gleich oder verschieden H, (C₁-C₆)-Alkyl, (C₂-C₆)-Alkenyl, (C₂-C₆)-Alkinyl, (C₃-C₆)-Cycloalkyl, (C₄-C₆)-Cycloalkenyl, Aryl oder Heterocyclyl,
wobei jede der sieben letztgenannten Gruppen gegebenenfalls durch einen oder mehrere Reste aus der Gruppe Halogen, vorzugsweise F, (C₁-C₄)-Alkoxy, (C₁-C₄)-Alkylthio, NR'-CO-(C₁-C₆)-Alkyl, mit R' = H oder (C₁-C₄)-Alkyl, substituiert ist.

Besonders bevorzugt sind folgende Gruppen von Verbindungen der Formel (I) a - f

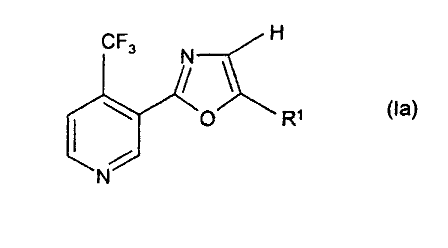

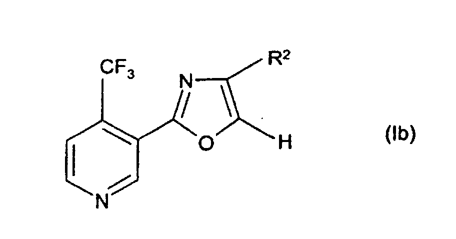

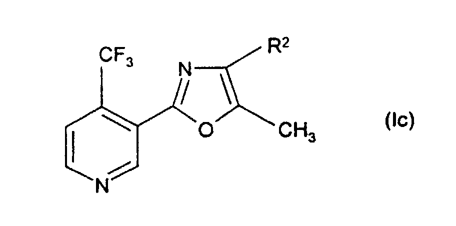

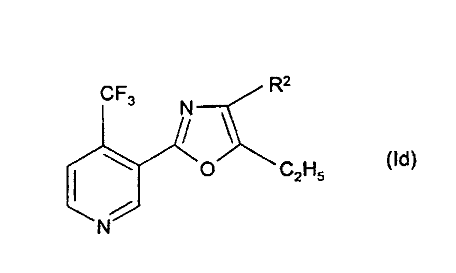

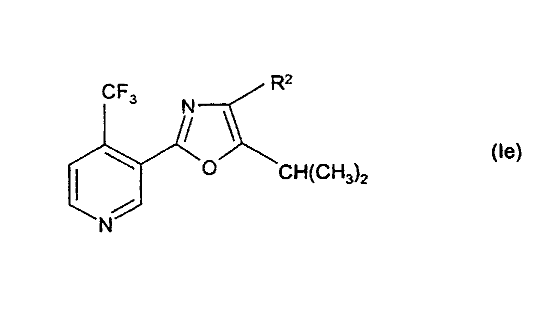

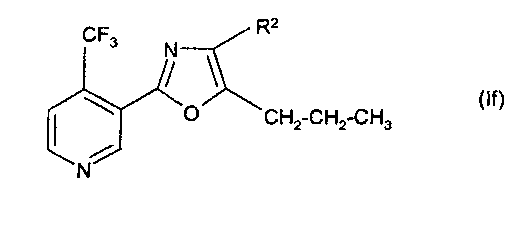

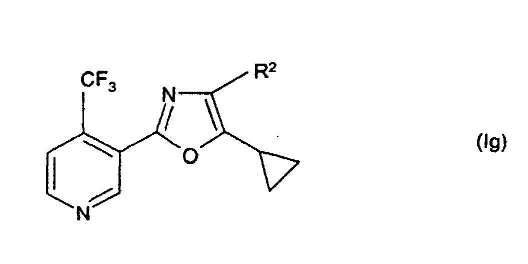

Ganz besonders bevorzugt sind Verbindungen der Formel Ia1- la12

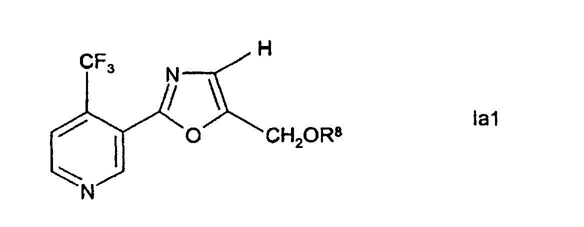

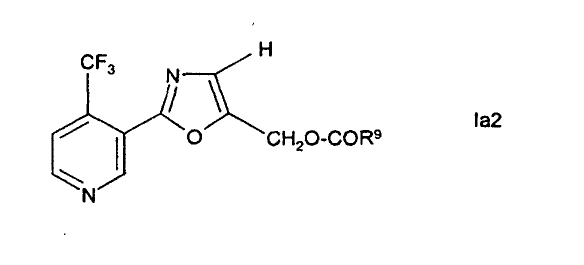

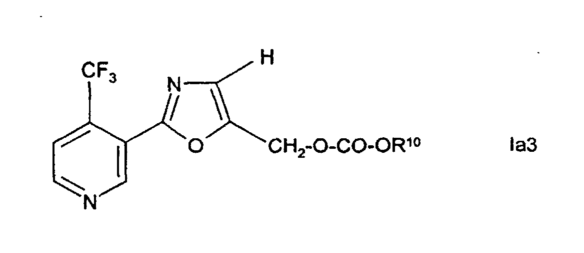

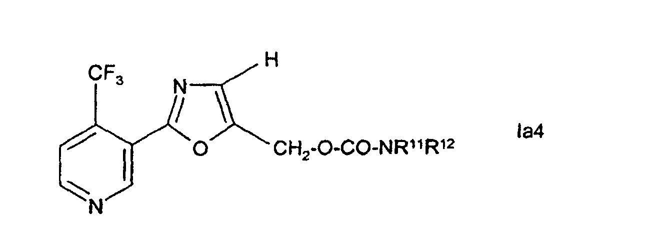

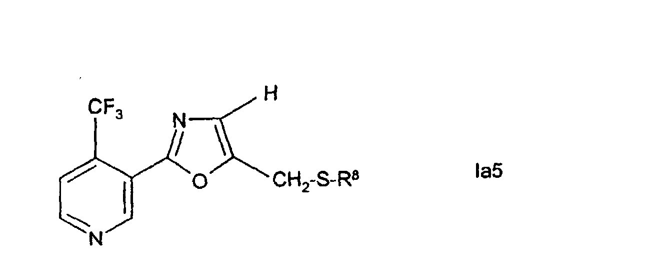

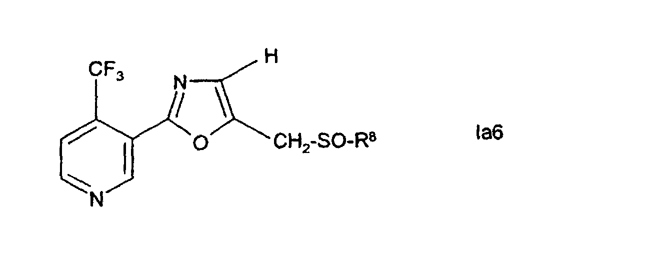

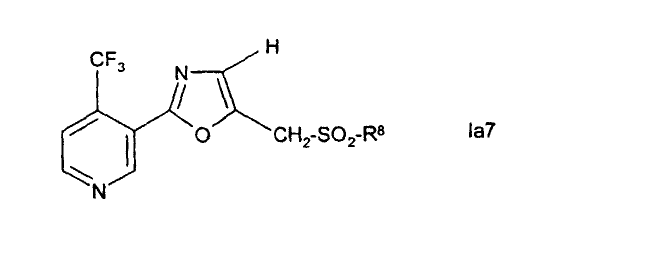

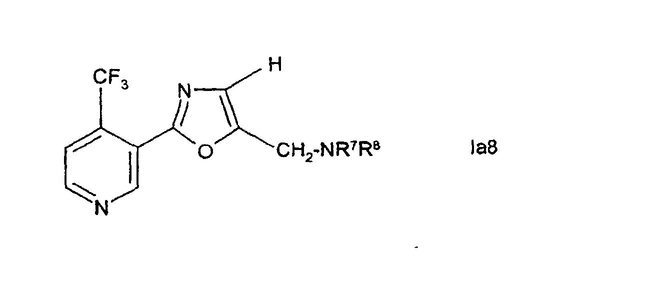

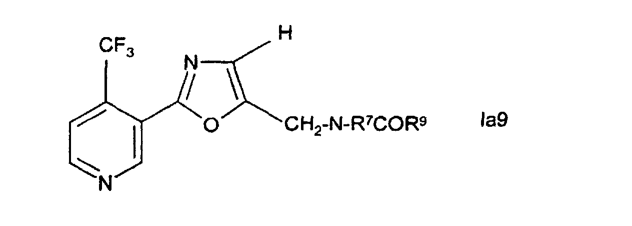

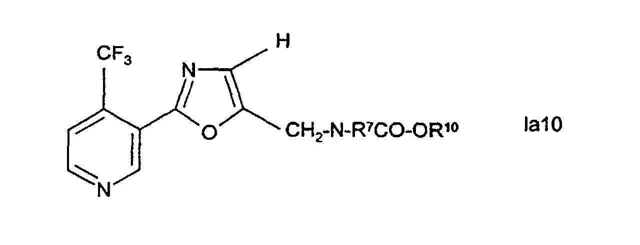

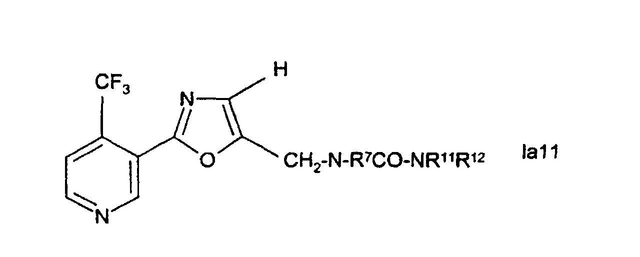

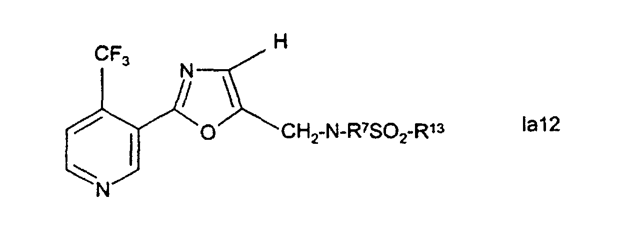

Weiterhin ganz besonders bevorzugt sind Verbindungen der Formel 1b1 bis 1b12:

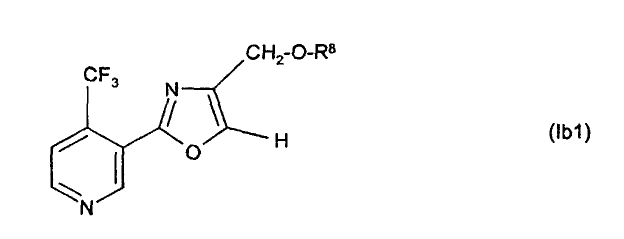

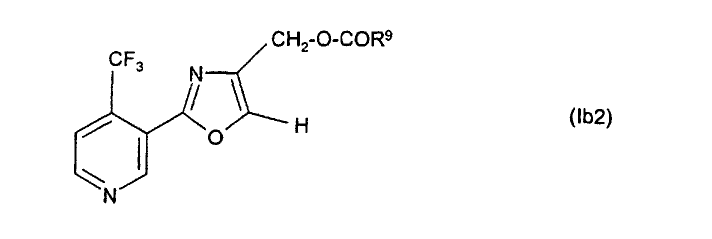

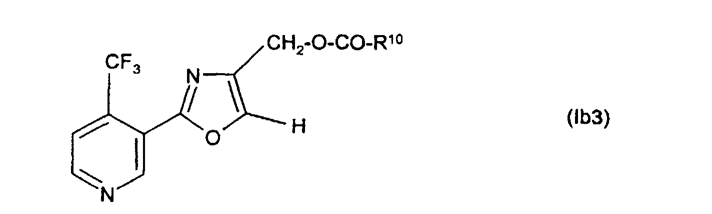

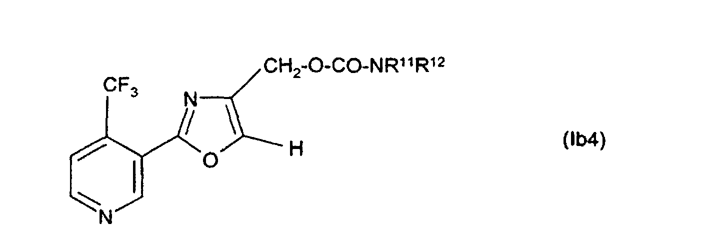

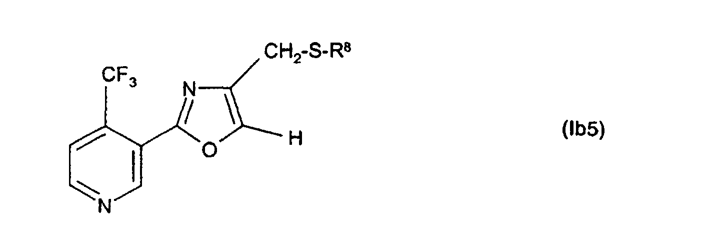

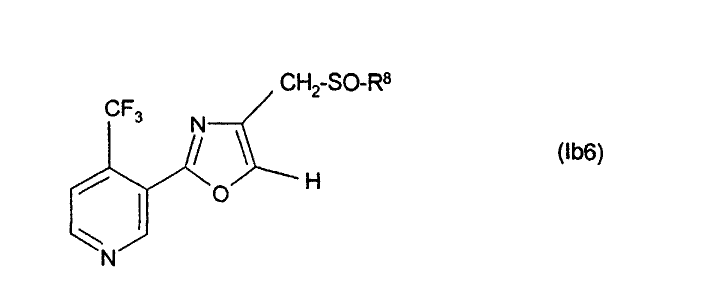

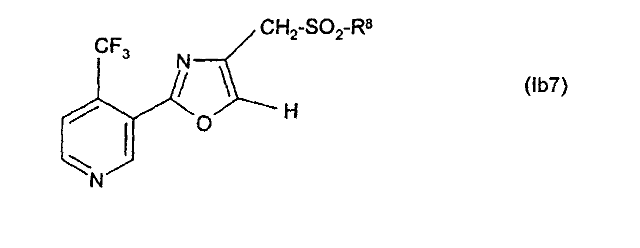

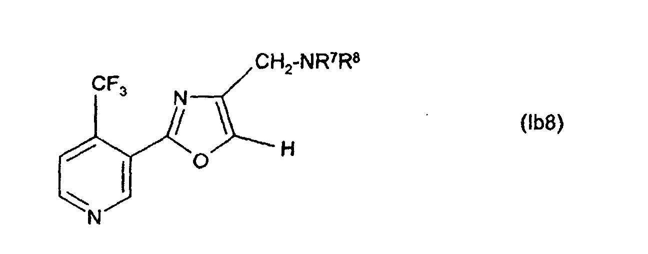

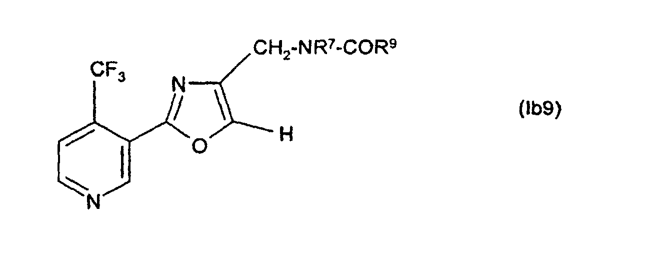

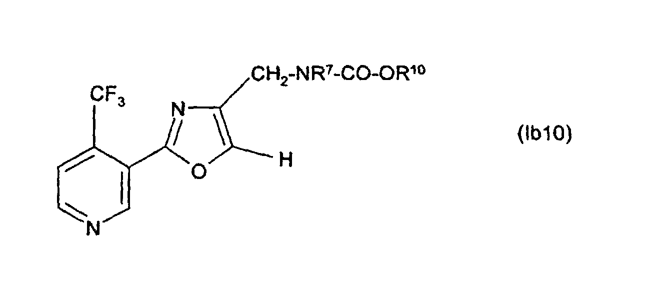

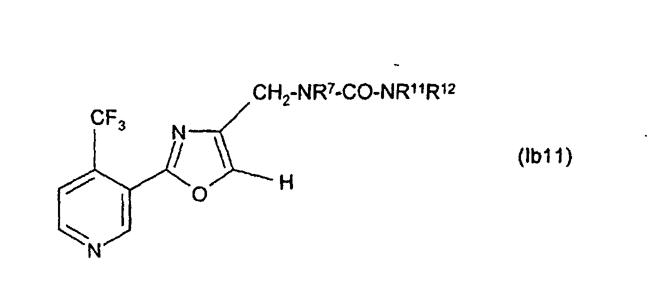

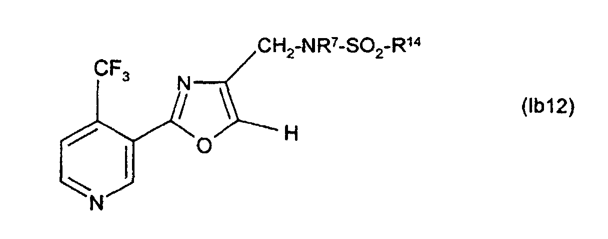

Die Symbole in den Formeln Ia-f bzw. 1a-12 und Ib1-12 haben die oben angegebenen Bedeutungen und Bevorzugungen.

Die Bezeichnung "Halogen" umfaßt Fluor, Chlor, Brom und Iod.

Unter dem Ausdruck "(C₁-C₄)-Alkyl" ist ein unverzweigter oder verzweigter Kohlenwasserstoffrest mit 1, 2, 3 oder 4 Kohlenstoffatomen, wie z.B. der Methyl-, Ethyl-, Propyl-, Isopropyl-, 1-Butyl-, 2-Butyl-, 2-Methylpropyl- oder tert.-Butylrest zu verstehen. Entsprechend ist unter Alkylresten mit einem größeren Bereich an Kohlenstoffatomen ein unverzweigter oder verzweigter gesättigter Kohlenwasserstoffrest zu verstehen, der eine Anzahl an Kohlenstoffatomen enthält, die dieser Bereichsangabe entspricht. Der Ausdruck "(C₁-C₆)-Alkyl" umfaßt demnach die vorgenannten Alkylreste, sowie z.B. den Pentyl-, 2-Methylbutyl-, 1,1-Dimethylpropyl- und Hexyl-Rest. Unter dem Ausdruck "(C₁-C₁₀)-Alkyl" sind die vorgenannten Alkylreste, sowie z.B. der Nonyl-, 1-Decyl- oder 2-Decyl-Rest zu verstehen.

Unter "(C₁-C₄)-Haloalkyl" ist eine unter dem Ausdruck "(C₁-C₄)-Alkyl" genannte Alkylgruppe zu verstehen, in der ein oder mehrere Wasserstoffatome durch die gleiche Anzahl gleicher oder verschiedener Halogenatome, bevorzugt Chlor oder Fluor, ersetzt sind, wie z. B. die Trifluormethyl-, die 1-Fluorethyl-, die 2,2,2-Trifluorethyl-, die Chlormethyl-, Fluormethyl-, die Difluormethyl- und die 1,1,2,2-Tetrafluorethylgruppe.

Unter "(C₁-C₄)-Alkoxy" ist eine Alkoxygruppe zu verstehen, deren Kohlenwasserstoffrest die unter dem Ausdruck "(C₁-C₄)-Alkyl" angegebene Bedeutung hat. Sinngemäß sind die Alkoxygruppen zu verstehen, die einen größeren Bereich an Kohlenstoffatomen umfassen.

Die Bezeichnungen "Alkenyl" und "Alkinyl" mit einer vorangestellten Bereichsangabe von Kohlenstoffatomen bedeuten einen geradkettigen oder verzweigten Kohlenwasserstoffrest mit einer dieser Bereichsangabe entsprechenden Kohlenstoffatomzahl, der mindestens eine Mehrfachbindung beinhaltet, wobei sich diese an beliebiger Position des betreffenden ungesättigten Restes befinden kann. "(C₂-C₄)-Alkenyl" steht demnach z.B. für die Vinyl-, Allyl-, 2-Methyl-2-propenyl- oder 2-Butenyl-Gruppe; "(C₂-C₆)-Alkenyl" steht für die vorstehend genannten Reste sowie z.B. für die Pentenyl-, 2-Methylpentenyl- oder die Hexenyl-Gruppe. "(C₂-C₄)-Alkinyl" steht z.B. für die Ethinyl-, Propargyl-, 2-Methyl-2-propinyl- oder 2-Butinyl-Gruppe. Unter "(C₂-C₆)-Alkinyl" sind die vorstehend genannten Reste sowie z.B. die 2-Pentinyl- oder die 2-Hexinyl-Gruppe und unter "(C₂-C₁₀)-Alkinyl" die vorstehend genannten Reste sowie z.B. die 2-Octinyl- oder die 2-Decinyl-Gruppe zu verstehen.

"(C₃-C₈)-Cycloalkyl" steht für monocyclische Alkylreste, wie den Cyclopropyl-, Cyclobutyl-, Cyclopentyl-, Cyclohexyl-, Cycloheptyl- oder Cyclooctylrest und für bicyclische Alkylreste, wie den Norbomylrest.

Unter dem Ausdruck "(C₃-C₈)-Cycloalkyl-(C₁-C₄)-alkyl" ist beispielsweise der Cyclopropylmethyl-, Cyclopentylmethyl-, Cyclohexylmethyl-, Cyclohexylethyl- und Cyclohexylbutyl-Rest und unter dem Ausdruck "(C₁-C₆)-Alkyl-(C₃-C₈)-cycloalkyl beispielsweise der 1-Methyl-cyclopropyl-, 1-Methyl-cyclopentyl-, 1-Methylcyclohexyl-, 3-Hexyl-cyclobutyl- und 4-tert.-Butyl-cyclohexyl-Rest zu verstehen.

"(C₁-C₄)-Alkoxy-(C₁-C₆)-alkyloxy" bedeutet eine wie vorstehend definierte Alkoxy-Gruppe, die durch eine weitere Alkoxy-Gruppe substituiert ist, wie z.B. 1-Ethoxyethoxy.

Unter" (C₃-C₈)-Cycloalkoxy" oder "(C₃-C₈)-Cycloalkylthio" ist einer der oben angeführten (C₃-C₈)-Cycloalkyl-Reste, der über ein Sauerstoff- oder Schwefelatom verknüpft ist, zu verstehen.

"(C₃-C₈)-Cycloalkyl-(C₁-C₆)-alkoxy bedeutet z.B. die Cyclopropylmethoxy, Cyclobutylmethoxy-, Cyclopentylmethoxy-, Cyclohexylmethoxy-, Cyclohexylethoxyoder die Cyclohexylbutoxy-Gruppe;

Der Ausdruck "(C₁-C₄)-Alkyl-(C₃-C₈)-cycloalkoxy" steht z.B. für die Methylcyclopropyloxy-, Methylcyclobutyloxy- oder die Butylcyclohexyloxy-Gruppe.

"(C₁-C₆)-Alkylthio" steht für eine Alkylthiogruppe, deren Kohlenwasserstoffrest die unter dem Ausdruck "(C₁-C₆)-Alkyl" angegebene Bedeutung hat.

Analog bedeuten "(C₁-C₆)-Alkylsulfinyl" z.B. die Methyl-, Ethyl-, Propyl-, Isopropyl-, Butyl-, Isobutyl-, sek.-Butyl- oder tert.-Butylsulfinyl-Gruppe und "(C₁-C₆)-Alkylsulfonyl" z.B. die Methyl-, Ethyl-, Propyl-, Isopropyl-, Butyl-, Isobutyl-, sek.-Butyl- oder tert.-Butylsulfonyl-Gruppe.

"(C₁-C₆)-Alkylamino" steht für ein Stickstoffatom, das durch einen oder zwei gleiche oder verschiedene Alkylreste der obigen Definition substituiert ist.

Der Ausdruck "(C₁-C₆)-Mono- oder Dialkylcarbamoyl" bedeutet eine Carbamoylgruppe mit einem oder zwei Kohlenwasserstoffresten, die die unter dem Ausdruck "(C₁-C₆-Alkyl)" angegebene Bedeutung haben und die im Fall von zwei Kohlenwasserstoffresten gleich oder verschieden sein können.

Analog bedeutet "(C₁-C₆)-Dihaloalkylcarbamoyl" eine Carbamoylgruppe, die zwei (C₁-C₆)-Haloalkylreste gemäß der obigen Definition oder einen (C₁-C₆)-Haloalkylrest und einen (C₁-C₆)-Alkylrest gemäß der obigen Definition trägt.

"(C₁-C₆)-Alkanoyl" steht z.B. für die Acetyl-, Propionyl-, Butyryl- oder 2-Methylbutyryl-Gruppe;

Unter dem Ausdruck "Aryl" ist ein carbocyclischer aromatischer Rest mit vorzugsweise-6 bis 14, insbesondere 6 bis 12 C-Atomen, beispielsweise Phenyl, Naphthyl oder Biphenylyl, vorzugsweise Phenyl, zu verstehen.

Der Ausdruck "Heterocyclyl" steht vorzugsweise für einen cyclischer Rest, der voll gesättigt, teilweise ungesättigt oder voll ungesättigt sein kann und der durch ein oder mehrere gleiche oder verschiedene Atome aus der Gruppe Stickstoff, Schwefel oder Sauerstoff unterbrochen sein kann, wobei jedoch nicht zwei Sauerstoffatome direkt benachbart sein dürfen und noch mindestens ein Kohlenstoffatom im Ring vorhanden sein muß, z.B. ein Rest von Thiophen, Furan, Pyrrol, Thiazol, Oxazol, Imidazol, Isothiazol, Isoxazol, Pyrazol, 1,3,4-Oxadiazol, 1,3,4-Thiadiazol, 1,3,4-Triazol, 1,2,4-Oxadiazol, 1,2,4-Thiadiazol, 1,2,4-Triazol, 1,2,3-Triazol, 1,2,3,4-Tetrazol, Benzo[b]thiophen, Benzo[b]furan, Indol, Benzo[c]thiophen, Benzo[c]furan, Isoindol, Benzoxazol, Benzothiazol, Benzimidazol, Benzisoxazol, Benzisothiazol, Benzopyrazol, Benzothiadiazol, Benzotriazol, Dibenzofuran, Dibenzothiophen, Carbazol, Pyridin, Pyrazin, Pyrimidin, Pyridazin, 1,3,5-Triazin, 1,2,4-Triazin, 1,2,5-Triazin, Chinolin, Isochinolin, Chinoxalin, Chinazolin, Cinnolin, 1,8-Naphthyridin, 1,5-Naphthyridin, 1,6-Naphthyridin, 1,7-Naphthyridin, Phthalazin, Pyridopyrimidin, Purin, Pteridin 4H-Chinolizin; Piperidin, Pyrrolidin, Oxazolin, Tetrahydrofuran, Tetrahydropyran, Isoxzolidin oder Thiazolidin. Der Ausdruck "Heteroaromat" umfaßt demnach von den vorstehend unter "Heterocycly" genannten Bedeutungen jeweils die voll ungesättigten aromatischen heterocyclischen Verbindungen.

Heterocyclyl bedeutet besonders bevorzugt ein gesättigtes, teilgesättigtes oder aromatisches Ringsystem mit 3 bis 6 Ringgliedern und 1 bis 4 Heteroatomen aus der Gruppe O, S und N.

Ganz besonders bevorzugt bedeutet Heterocyclyl ein Radikal des Pyridin, Pyrimidin, (1,2,4)-Oxadiazol, (1,3,4)-Oxadiazol, Pyrrol, Furan, Thiophen, Oxazol, Thiazol, Imidazol, Pyrazol, Isoxazol, 1,2,4-Triazol, Tetrazol, Pyrazin, Pyridazin, Oxazolin, Thiazolin, Tetrahydrofuran, Tetrahydropyran, Morpholin, Piperidin, Piperazin, Pyrrolin, Pyrrolidin, Oxazolidin, Thiazolidin, Oxiran und Oxetan.

"Aryl-(C₁-C₄)-alkoxy" steht für einen über eine (C₁-C₄)-Alkoxygruppe verknüpften Arylrest, z.B. der Benzyloxy-, Phenylethoxy-, Phenylbutoxy- oder Naphthylmethoxy-Rest.

"Arylthio" bedeutet einen über ein Schwefelatom verknüpften Arylrest, z.B. den Phenylthio- oder die 1- oder 2-Naphthylthio-Rest. Analog bedeutet "Aryloxy" z.B. den Phenoxy- oder 1- oder 2-Naphthyloxy-Rest.

"Aryl-(C₁-C₄)-alkylthio" steht für einen Arylrest, der über einen Alkylthiorest verknüpft ist, z.B. der Benzylthio-, Naphthylmethylthio- oder die Phenylethylthio-Rest.

Der Ausdruck "(C₁-C₆)-Trialkylsilyl" bedeutet ein Siliciumatom, das drei gleiche oder verschiedene Alkylreste gemäß der obigen Definition trägt. Analog steht "Aryl-(C₁-C₆)-Dialkylsilyl" für ein Siliciumatom, das einen Arylrest und zwei gleiche oder verschiedene Alkylreste gemäß der obigen Definition trägt, "Diaryl-(C₁-C₆)-Alkylsilyl" für ein Siliciumatom, das einen Alkylrest und zwei gleiche oder verschiedene Arylreste gemäß der obigen Definition trägt, und "Triarylsilyl" für ein Siliciumatom, das drei gleiche oder verschiedene Arylreste gemäß der obigen Definition trägt.

Je nach Art der oben definierten Substituenten weisen die Verbindungen der allgemeinen Formel (I) saure oder basische Eigenschaften auf und können Salze bilden. Tragen die Verbindungen der allgemeinen Formel (I) beispielsweise Gruppen wie Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkaliund Erdalkalimetalle, insbesondere die von Natrium, Kalium, Magnesium und Calcium, weiterhin Ammoniak, primäre, sekundäre und tertiäre Amine mit (C₁-C₄)-Alkylresten sowie Mono-, Di- und Trialkanolamine von (C₁-C₄)-Alkanolen. Tragen die Verbindungen der allgemeinen Formel (I) beispielsweise Gruppen wie Amino, Alkylamino oder andere, basische Eigenschaften induzierende Gruppen, so können diese Verbindungen mit Säuren zu Salzen umgesetzt werden. Geeignete Säuren sind beispielsweise Mineralsäuren, wie Salz-, Schwefel- und Phosphorsäure, organische Säuren, wie Essigsäure und Oxalsäure, und saure Salze, wie NaHS04 und KHSO4. Die so erhältlichen Salze weisen ebenfalls insektizide, akarizide und nematizide Eigenschaften auf.

Die Verbindungen der allgemeinen Formel (I) können ein oder mehrere asymmetrische Kohlenstoffatome oder Stereoisomere an Doppelbindungen aufweisen. Es können daher Enantiomere oder Diastereomere auftreten. Die Erfindung umfaßt sowohl die reinen Isomeren als auch deren Gemische. Die Gemische von Diastereomeren können nach gebräuchlichen Methoden, z.B. durch selektive Kristallisation aus geeigneten Lösungsmitteln oder durch Chromatographie in die Isomeren aufgetrennt werden. Racemate können nach üblichen Methoden in die Enantiomeren aufgetrennt werden.

Die Herstellung der erfindungsgemäßen Verbindungen erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart, beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch machen.

Die Edukte können gewünschtenfalls auch in situ gebildet werden, und zwar derart, daß man sie aus dem Reaktionsgemisch nicht isoliert, sondern sofort weiter zu den Verbindungen der Formel (I) umsetzt.

Die vorliegende Erfindung betrifft auch Verfahren zur Herstellung von Verbindungen der allgemeinen Formel (I):

Verbindungen der Formel (I) mit R² = H lassen sich beispielsweise durch weitere Funktionalisierung von Verbindung (III) oder (IV) herstellen:

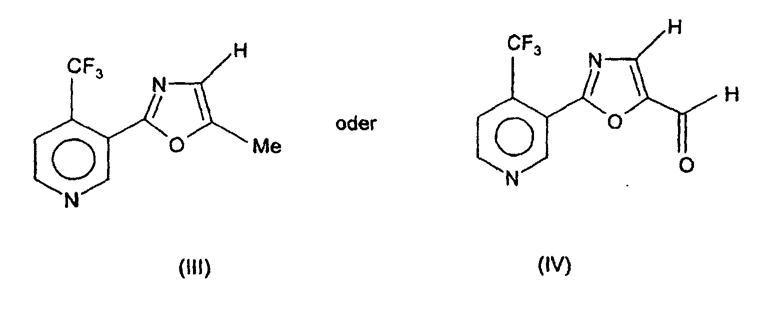

Die Verbindungen (III) und (IV) werden beispielsweise durch Cyclisierung von Verbindungen der Formel (V) erhalten:

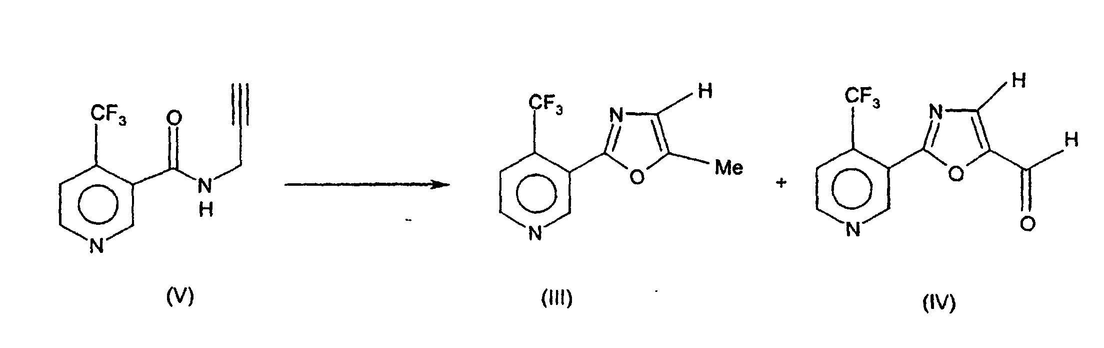

Verschiedene Vorschriften für die Cyclisierung sind aus der Literatur bekannt, z.B.
- KOH / Ethanol / Rückfluß (siehe z.B. J. Reisch et al., Pharmazie 1992, 47, 18-20)
- NaH / THF / 70 °C (siehe z.B. B. M. Nilsson et al., J. Heterocyclic Chem. 1989, 26, 269 - 275)
- Hg (CH₃CO₂)₂ / Eisessig / Rückfluß (siehe z.B. J. Saunders et al., J. Med. Chem. 1990, 33, 1128-1130)

Die Ausgangsverbindung (V) ist aus käuflicher Trifluormethylnicotinsäure und Propargylamin durch Verwendung eines wasserentziehenden Reagenzes, wie Dicyclohexylcarbodiimid, 1-Ethyl-3-(3-dimethylaminopropyl)carbodiimid oder N,N'-Carbonyldiimidazol, direkt zugänglich.

Verbindungen der Formel (I) mit R¹ = H lassen sich beispielsweise durch weitere Funktionalisierung der Verbindung (VI) 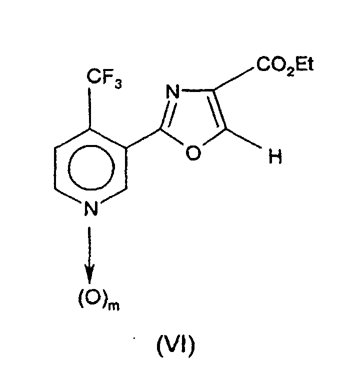 erhalten.

Verbindung (VI) kann beispielsweise in zwei Schritten durch Cyclisierung von (VII) hergestellt werden: 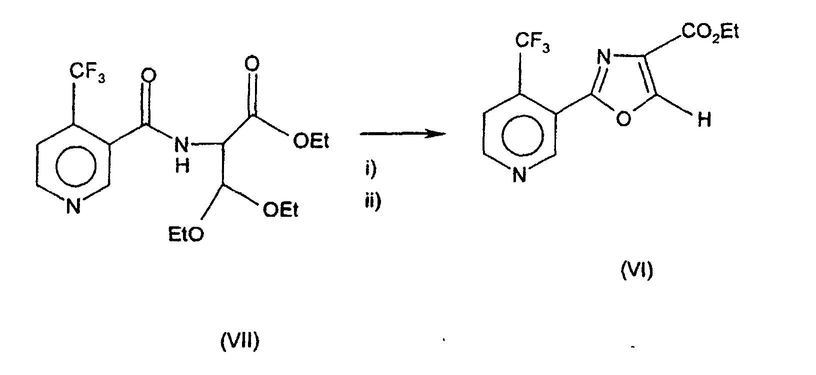 wobei (VII) z.B. zuerst bei 40°C in Gykoldimethylether mit Trimethylsilyttrifluormethylsulfonat behandelt wird und anschließend durch Behandlung mit Kaliumtertiärbutanolat in tert. Butanol bei 0°C die Umsetzung zu (VI) erfolgt (siehe z.B. S. Swaminathan et al., Tetrahedron Lett. 1998, 39, 4769-4472).

Verbindung (VII) läßt sich z.B. durch Acylierung von Ethyl-2-amino-3,3-diethoxypropionat mit 4-Trifluormethylnicotinsäurechlorid unter Standardbedingungen herstellen. Ethyl-2-amino-3,3-diethoxypropionat kann nach literaturbekannten Verfahren hergestellt werden (siehe z.B. S.K. Singh et al., Heterocycles 1997, 44, 379-391 oder T.W. Doyle et al., Can. J. Chem. 1977, 55, 468-483).

Verbindungen in denen R¹ ein Rest aus der Gruppe a und R² aus der Gruppe b ist, lassen sich beispielsweise durch weitere Funktionalisierung von Verbindungen der Formel (VIII) herstellen: 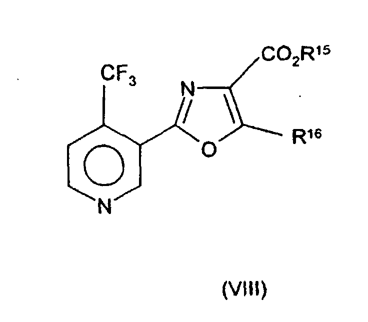 wobei
- R¹⁵: CH₃ oder C₂H₅ und
- R¹⁶: CH₃, C₂H₅, CH₂-CH₂-CH₃, -CH(CH₃)₂ oder Cyclopropyl bedeutet.

(VIII) läßt sich z. B. durch Cyclisierung von Amiden der Formel (IX) herstellen.

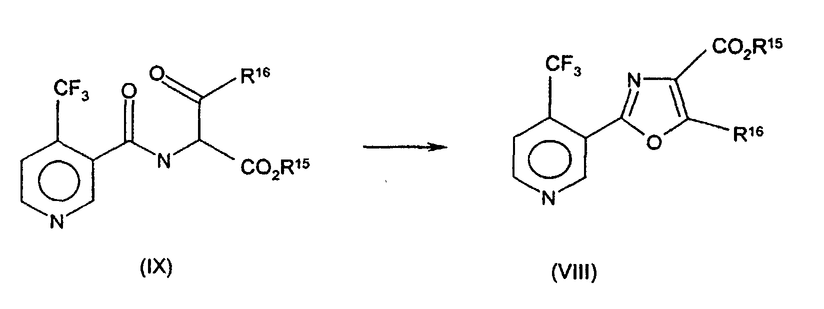

Als wasserentziehendes Reagenz können hierbei beispielsweise anorganische Säurechloride, wie Thionylchlorid oder Phosphorylchlorid, anorganische Säuren, wie Schwefelsäure oder Phosphorsäure, oder ein Gemisch von Acetanhydrid mit einer anorganischen Säure eingesetzt werden (siehe z.B. K. Meguro et al., Chem. Pharm. Bull. 1986, 34, 2840-2851).
Die Verbindungen der Formel (IX) lassen sich beispielsweise in einem Eintopfverfahren aus dem entsprechenden β-Ketoester (X) und
4-Trifluormethylnicotrinsäurechlorid herstellen.

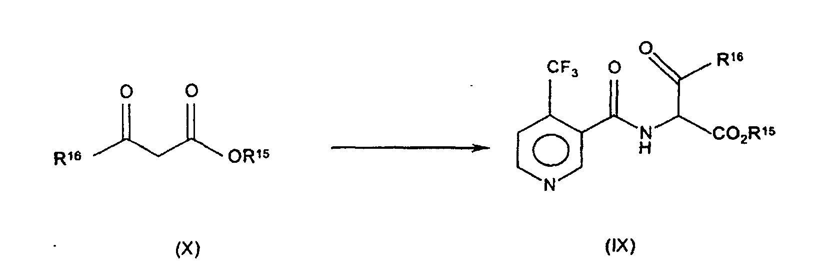

Die Herstellung des Oxims, aus dem β-Ketoester mit Natriumnitrit in Essigsäure, Reduktion mit Zn-Schwefelsäure und anschließende Acylierung ist z.B. bei G. Erhart, Berichte 1949, 82, 60-63 beschrieben.

Nach Aufbau des Oxazolsystems durch Kondensations- und Cyclisierungsreaktionen können die Reste R¹, R² der Verbindungen der Formel (XI) gewünschtenfalls weiter derivatisiert werden, wobei die breite, dem Fachmann geläufige Methodenpalette der organisch-chemischen Synthese zur Verfügung steht.

Ester und Carboxamide der Formel (I) können z.B. aus Verbindungen der Formel (XI) nach literaturbekannten, dem Fachmann geläufigen Methoden, wie Umesterung, Aminolyse oder Amidbildung aus Carbonsäuren und Aminen durch Verwendung eines wasserentziehenden Reagenzes, wie Dicyclohexylcarbodiimid, 1-Ethyl-3-(3-dimethylaminopropyl)carbodiimid, N,N'-Carbonyldiimidazol oder 2-(1 H-Benzotriazol-2-yl)-1,1,3,3-tetramethylammonium-tetrafluoroborat, erhalten werden. 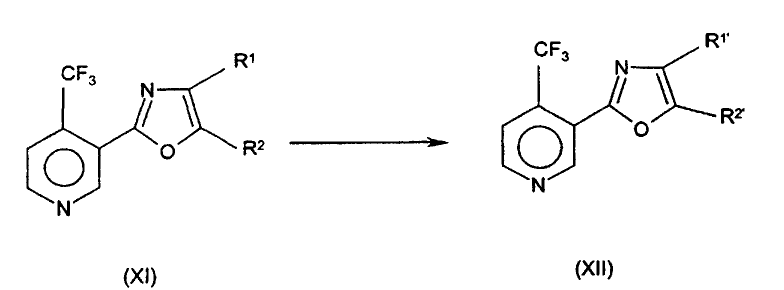 wobei
R¹, R² bzw. R^{1'}, R^{2'} beispielsweise folgende Bedeutungen haben:
R¹ = H; R² = CO₂H R^{1'} = H, R^{2'} = CO₂R⁴, CONR⁵R⁶
R¹ = CO₂Et, CO₂H; R² = H R^{1'} = CO₂R⁴, CONR⁵R⁶; R^{2'} = H
R¹ = CO₂Me, CO₂Et, CO₂H R^{1'} = CO₂R⁴, CONR⁵R⁶,
R² = Me, Et, n-Pr, i-Pr und c-Pr R^{2'} = Me, Et, i-Pr und c-Pr (c = cyclo).
R⁴, R⁵ und R⁶ haben dabei die oben angegebenen Bedeutungen.

Verbindungen der Formel (XI) mit R¹ = CO₂Et, CO₂Me, CO₂H; R² = H, Me, Et, n-Pr, i-Pr und c-Pr können direkt durch die beschriebenen Cyclisierungsreaktionen oder durch Hydrolyse der Ester von Cyclisierungsprodukten erhalten werden.

Verbindung (XI) mit R¹ = H und R² = CO₂H kann z.B. durch Oxidation nach literaturbekannten Verfahren aus Verbindung (III) oder (IV) hergestellt werden.

Ether, Thioether und Amine und weitere Derivate der Formel (XIV) können z.B. aus Verbindungen der Formel (XIII) durch literaturbekannte, dem Fachmann geläufige Umsetzungen mit entsprechenden Nucleophilen erhalten werden. 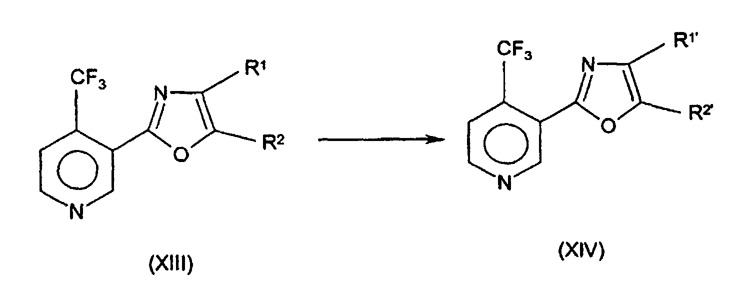
I. R¹ = H; R² = CH₂Br, CH₂I R^{1'} = H; R^{2'} = CH₂XR³
II. R¹ = CH₂Cl, CH₂I; R² = H R^{1'} = CH₂XR³; R^{2'} = H
III. R¹ = CH₂Cl, CH₂l; R^{1'} = CH₂XR³,
   R² = Me, Et; n-Pr, i-Pr und c-Pr; R^{2'} = Me, Et, n-Pr, i-Pr und c-Pr X = O, S, NR⁴
Wobei X, R³ und R⁴ die oben angegebenen Bedeutungen haben.

Verbindungen der Formel (XIII) können nach literaturbekannten, dem Fachmann geläufigen Verfahren aus Verbindungen der Formeln (III), (VI) und (VIII) hergestellt werden, z.B.

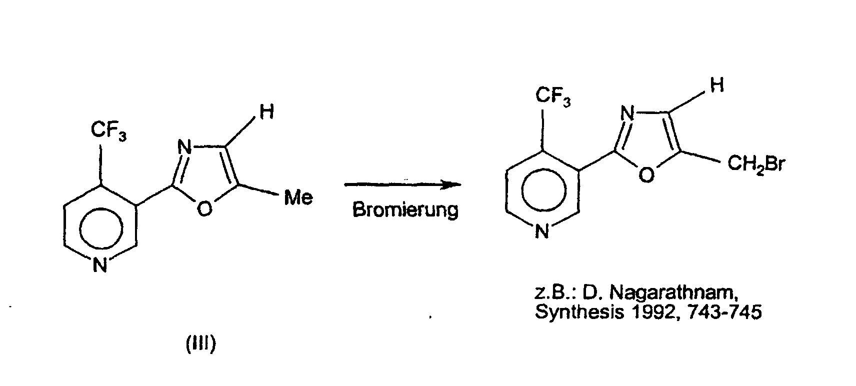

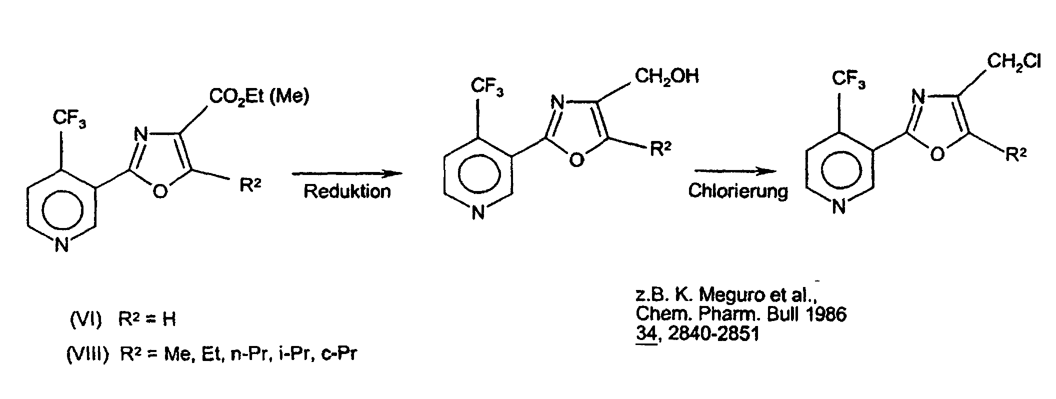

Verbindungen der Formel (XIV), in denen R¹ oder R² CH₂XR³ mit X = S oder NR⁴,
wobei R³ oder R⁴ = H oder R³ = R⁴ = H gilt, bedeutet, können nach literaturbekannten, dem Fachmann geläufigen Methoden z.B. zu Sulfoxiden, Sulfonen, Amiden und Carbamaten umgesetzt werden.

Zum Aufbau von Verbindungen der Formel (I), in der m 1 bedeutet, können Verbindungen der Formel (I), in der m 0 bedeutet, mit einem Oxidationsreagenz, wie meta-Chlorperbenzoesäure, behandelt werden.

Kollektionen aus Verbindungen der Formel (I), die nach oben genannten Schemata synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch S.H. DeWitt in "Annual Reports in Combinatoriäl Chemistry and Molecular Diversity: Automated synthesis", Band 1, Verlag Escom 1997, Seite 69 bis 77 beschrieben ist.

Zur parallelisierten Reaktionsdurchführung und Aufarbeitung können eine Reihe von im Handel erhältlichen Geräten verwendet werden, wie sie beispielsweise von den Firmen Stem Corporation, Woodrolfe road, Tollesbury, Essex, CM9 8SE, England, H+P Labortechnik GmbH, Bruckmannring 28, 85764 Oberschleißheim, Deutschland oder der Firma Radleys, Shirehill, Saffron Walden, Essex, CB - II:3AZ, England angeboten werden. Für die parallelisierte Aufreinigung von Verbindungen der allgemeinen Formel (I) beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständige integrierten Automationssystemen umgangen werden, bei denen die jeweiligen Automationsmodule beispielsweise durch Roboter bedient werden. Derartige Automationssysteme können zum Beispiel von der Firma Zymark Corporation, Zymark Center, Hopkinton, MA 01748, USA bezogen werden.

Neben dem hier beschriebenen kann die Herstellung von Verbindungen der allgemeinen Formel (I) vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen unterstützte Synthesemethoden sind in der Fachliteratur hinreichend beschrieben, z.B. Barry A. Bunin in "The Combinatorial Index", Verlag Academic Press, 1998.
Die Verwendung von Festphasen unterstützten Synthesemethoden erlaubt eine Reihe von literaturbekannten Protokollen, die wiederum manuell oder automatisierten ausgeführt werden können. Zum Beispiel kann die "Teebeutelmethode" (Houghten, US 4,631,211; Houghten et al., Proc. Natl. Acad. Sci, 1985, 82, 5131-5135) mit Produkten der Firma IRORI, 11149 North Torrey Pines Road, La Jolla, CA 92037, USA teilweise automatisiert werden. Die Automatisierung von Festphasen unterstützten Parallelsynthesen gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß der hier beschriebenen Verfahren liefert Verbindungen der Formel (I) in Form von Substanzkollektionen, die Bibliotheken genannt werden. Gegenstand der vorliegenden Erfindung sind auch Bibliotheken, die mindestens zwei Verbindungen der Formel (I) enthalten.

Die Verbindungen der Formel (I) eignen sich bei guter Pflanzenverträglichkeit und günstiger Warmblütertoxizität zur Bekämpfung von tierischen Schädlingen, insbesondere Insekten, Spinnentieren, Helminthen und Mollusken, ganz besonders bevorzugt zur Bekämpfung von Insekten und Spinnentieren, die in der Landwirtschaft, bei der Tierzucht, in Forsten, im Vorrats- und Materialschutz sowie auf dem Hygienesektor vorkommen. Sie sind gegen normal sensible und resistente Arten sowie alle oder einzelne Entwicklungsstadien wirksam. Zu den oben erwähnten Schädlingen gehören:
Aus der Ordnung der Acarina z.B. Acarus siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Eotetranychus spp., Oligonychus spp., Eutetranychus spp.. Aus der Ordnung der Isopoda z.B. Oniscus aselus, Armadium vulgare, Porcellio scaber.
Aus der Ordnung der Diptopoda z.B. Blaniulus guttulatus.
Aus der Ordnung der Chilopoda z.B. Geophilus carpophagus, Scutigera spp..
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria. Aus der Ordnung des Isoptera z.B. Reticulitermes spp..
Aus der Ordnung der Anoplura z.B. Phylloera vastatrix, Pemphigus spp., Pediculus humanus corporis, Haematopinus spp., Linognathus spp..
Aus der Ordnung der Mallophaga z.B. Trichodectes pp., Damalinea spp..
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp..
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylloides chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrynchus assimilis, Hypera postica, Dermestes spp., Trogoderma, Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp..
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hypobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopsis, Ceratophyllus spp.. Aus der Ordnung der Arachnida z.B. Scorpio maurus, Latrodectus mactans.
Aus der Klasse der Helminthen z.B. Haemonchus, Trichostrongulus, Ostertagia, Cooperia, Chabertia, Strongyloides, Oesophagostomum, Hyostrongulus, Ancylostoma, Ascaris und Heterakis sowie Fasciola.

Aus der Klasse der Gastropoda z.B. Deroceras spp., Arion spp., Lymnaea spp., Galba spp., Succinea spp., Biomphataria spp., Bulinus spp., Oncomelania spp..
Aus der Klasse der Bivalva z.B. Dreissena spp..

Zu den pflanzenparasitären Nematoden, die erfindungsgemäß bekämpft werden können, gehören beispielsweise die wurzelparasitären Bodennematoden wie z.B. solche der Gattungen Meloidogyne (Wurzelgallennematoden, wie Meloidogyne incognita, Meloidogyne hapla und Meloidogyne javanica), Heterodera und Globodera (zystenbildende Nematoden, wie Globodera rostochiensis, Globodera pallida, Heterodera trifolii) sowie der Gattungen Radopholus wie Radopholus similis, Pratylenchus wie Pratyglenchus neglectus, Pratylenchus penetrans und Pratylenchus curvitatus;
Tylenchulus wie Tylenchulus semipenetrans, Tylenchorhynchus, wie Tylenchorhynchus dubius und Tylenchorhynchus claytoni, Rotylenchus wie Rotylenchus robustus, Heliocotylenchus wie Haliocotylenchus multicinctus, Belonoaimus wie Belonoaimus longicaudatus, Longidorus wie Longidorus elongatus, Trichodorus wie Trichodorus primitivus und Xiphinema wie Xiphinema index.

Ferner lassen sich mit den erfindungsgemäßen Verbindungen die Nematodengattungen Ditylenchus (Stengelparasiten, wie Ditylenchus dipsaci und Ditylenchus destructor), Aphelenchoides (Blattnematoden, wie Aphelenchoides ritzemabosi) und Anguina (Blütennematoden, wie Anguina tritici) bekämpfen.

Die Erfindung betrifft auch Mittel, insbesondere insektizide und akarizide Mittel, die eine oder mehrere Verbindungen der Formel (I) neben geeigneten Formulierungshilfsmittein enthalten.

Die erfindungsgemäßen Mittel enthalten die Wirkstoffe der Formeln (I) im allgemeinen zu 1 bis 95 Gew.-%. Sie können auf verschiedene Art formuliert
werden, je nachdem, wie es durch die biologischen und/oder chemischphysikalischen Parameter vorgegeben ist. Als Formulierungsmöglichkeiten kommen daher z. B. in Frage:
Spritzpulver (WP), emulgierbare Konzentrate (EC), wäßrige Lösungen (SL), Emulsionen, versprühbare Lösungen, Dispersionen auf Öl- oder Wasserbasis (SC), Suspoemulsionen (SE), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und beispielsweise beschrieben in: Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.
Die notwendigen Formulierungshilfsmittel, d.h. Träger- und/oder oberflächenaktiven Stoffe, wie Inertmaterialien, Tenside, Lösungsmittel und weitere Zusatzstoffe sind ebenfalls bekannt und beispielsweise beschrieben in: Watkins, "Handbook of Insecticide Dust Diluents and Carriers", 2nd Ed., Darland Books, Caldwell N.J.; H. v. Olphen, "Introduction to Clay Colloid Chemistry", 2nd Ed., J. Wiley & Sons, N.Y.; Marsden, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1967; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan-6,6'disulfonsaures Natrium enthalten.
Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calcium-Salze, wie Cadodecylbenzol-sulfonat, oder nichtionische Emulgatoren, wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man z. B. durch Vermahlen des Wirkstoffes mit fein verteilten festen Stoffen, z.B. Talkum, natürlichen Tonen wie Kaolin, Bentonit, Pyrophillit oder Diatomeenerde. Granulate können z. B. entweder durch Verdüsen des Wirkstoffes auf adsorptionsfähiges, granuliertes Inertmaterial oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen, wie Sand, Kaolinite, oder von granuliertem Inertmaterial hergestellt werden. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration üblicherweise etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls jeweils übliche Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser.

Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,0005 und 10,0 kg/ha oder mehr Wirkstoff, vorzugsweise liegt sie jedoch zwischen 0,001 und 5 kg/ha.

Die erfindungsgemäßen Wirkstoffe können in ihren handelsüblichen Formulierungen sowie in den aus diesen Formulierungen bereiteten Anwendungsformen in Mischungen mit anderen Wirkstoffen, wie weiteren Schädlingsbekämpfungsmitteln, z.B. Insektiziden oder Akariziden, Lockstoffen, Sterilantien, Nematiziden, Fungiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen und durch Mikroorganismen hergestellte Stoffe.

Bevorzugte Mischungspartner sind:
1. aus der Gruppe der Phosphorverbindungen
   Acephate, Azamethiphos, Azinphos-ethyl, Azinphos-methyl, Bromophos, Bromophos-ethyl, Cadusafos (F-67825), Chlorethoxyphos, Chlorfenvinphos, Chlormephos, Chlorpyrifos, Chlorpyrifos-methyl, Demeton, Demeton-S-methyl, Demeton-S-methyl sulfon, Dialifos, Diazinon, Dichlorvos, Dicrotophos, Dimethoate, Disulfoton, EPN, Ethion, Ethoprophos, Etrimfos, Famphur, Fenamiphos, Fenitriothion, Fensulfothion, Fenthion, Fonofos, Formothion, Fosthiazate (ASC-66824) Heptenophos, Isazophos, Isothioate, Isoxathion, Malathion, Methacrifos, Methamidophos, Methidathion, Salithion, Mevinphos, Monocrotophos, Naled, Omethoate, Oxydemeton-methyl, Parathion, Parathion-methyl, Phenthoate, Phorate, Phosalone, Phosfolan, Phosphocarb (BAS-301), Phosmet, Phosphamidon, Phoxim, Pirimiphos, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tebupirimfos,Tetrachlorvinphos, Thiometon, Triazophos, Trichlorphon, Vamidothion;
2. aus der Gruppe der Carbamate
   Alanycarb (OK-135), Aldicarb, 2-sec.-Butylphenylmethylcarbamate (BPMC), Carbaryl, Carbofuran, Carbosulfan, Cloethocarb, Benfuracarb, Ethiofencarb, Furathiocarb, HCN-801, Isoprocarb, Methomyl, 5-Methyl-mcumenylbutyryl(methyl)carbamate, Oxamyl, Pirimicarb, Propoxur, Thiodicarb, Thiofanox, 1-Methylthio(ethylideneamino)-N-methyl-N-(morpholinothio)carbamate (UC 51717), Triazamate;
3. aus der Gruppe der Carbonsäureester
   Acrinathrin,Allethrin, Alphametrin, 5-Benzyl-3-furylmethyl-(E)-(1R)-cis-2,2-di-methyl-3-(2-oxothiolan-3-ylidenemethyl)cyclopropanecarboxylate, Beta-Cyfluthrin, Beta-Cypermethrin, Bioallethrin, Bioallethrin((S)-cyclopentylisomer), Bioresmethrin, Bifenthrin, (RS)-1-Cyano-1-(6-phenoxy-2-pyridyl)methyl-(1 RS)-trans-3-(4-tert.butylphenyl)-2,2-dimethylcyclopropanecarboxylate (NCI 85193), Cycloprothrin, Cyfluthrin, Cyhalothrin, Cythithrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-Isomer), Imiprothrin (S-41311), Lambda-Cyhalothrin, Permethrin, Phenothrin ((R)-Isomer), Prallethrin, Pyrethrine (natürliche Produkte), Resmethrin, Tefluthrin, Tetramethrin, Theta-Cypermethrin (TD-2344), Tralomethrin, Transfluthrin, Zeta-Cypermethrin (F-56701);
4. aus der Gruppe der Amidine
   Amitraz, Chlordimeform;
5. aus der Gruppe der Zinnverbindungen
   Cyhexatin, Fenbutatinoxide;
6. Sonstige
   Abamectin, ABG-9008, Acetamiprid, Anagrapha falcitera, AKD-1022, AKD-3059, ANS-118, Bacillus thuringiensis, Beauveria bassianea, Bensultap, Bifenazate (D-2341), Binapacryl, BJL-932, Bromopropylate, BTG-504, BTG-505, Buprofezin, Camphechlor, Cartap, Chlorobenzilate, Chlorfenapyr, Chlorfluazuron, 2-(4-Chlorphenyl)-4,5-diphenylthiophen (UBI-T 930), Chlorfentezine, Chromafenozide (ANS-118), CG-216, CG-217, CG-234, A-184699, Cyclopropancarbonsäure-(2-naphthylmethyl)ester (Ro12-0470), Cyromazin, Diacloden (Thiamethoxam), Diafenthiuron, N-(3,5-Dichlor-4-(1,1,2,3,3,3-hexafluor-1-propyloxy)
   phenyl)carbamoyl)-2-chlorbenzcarboximidsäureethylester, DDT, Dicofol, Diflubenzuron, N-(2,3-Dihydro-3-methyl-1,3-thiazol-2-ylidene)-2,4-xylidine, Dinobuton, Dinocap, Diofenolan, DPX-062, Emamectin-Benzoate (MK-244), Endosulfan, Ethiprole (Sulfethiprole), Ethofenprox, Etoxazole (YI-5301), Fenazaquin, Fenoxycarb, Fipronil, Fluazuron, Flumite (Flufenzine, SZI-121), 2-Fluoro-5-(4-(4-ethoxyphenyl)-4-methyl-1-pentyl)diphenylether (MTI 800), Granulose- und Kempolyederviren, Fenpyroximate, Fenthiocarb, Flubenzimine, Flucycloxuron, Flufenoxuron, Flufenprox (ICI-A5683), Fluproxyfen, Gamma-HCH, Halofenozide (RH-0345), Halofenprox (MTI-732), Hexaflumuron (DE_473), Hexythiazox, HOI-9004, Hydramethylnon (AC 217300), Lufenuron, Imidacloprid, Indoxacarb (DPX-MP062), Kanemite (AKD-2023), M-020, MTI-446, Ivermectin, M-020, Methoxyfenozide (Intrepid, RH-2485), Milbemectin, NC-196, Neemgard, Nitenpyram (Tl-304), 2-Nitromethyl-4,5-dihydro-6H-thiazin (DS 52618), 2-Nitromethyl-3,4-dihydrothiazol (SD 35651), 2-Nitromethylene-1,2-thiazinan-3-ylcarbamaldehyde (WL 108477), Pyriproxyfen (S-71639), NC-196, NC-1111, NNI-9768, Novaluron (MCW-275), OK-9701, OK-9601, OK-9602, Propargite, Pymethrozine, Pyridaben, Pyrimidifen (SU-8801), RH-0345, RH-2485, RYI-210, S-1283, S-1833, SB7242. SI-8601, Silafluofen, Silomadine (CG-177), Spinosad, SU-9118, Tebufenozide, Tebufenpyrad (MK-239), Teflubenzuron, Tetradifon, Tetrasul, Thiacloprid, Thiocyclam, TI-435, Tolfenpyrad (OMI-88), Triazamate (RH-7988), Triflumuron, Verbutin, Vertalec (Mykotal), YI-5301.

Die oben genannten Kombinationspartner sind bekannte Wirkstoffe, die zum großen Teil in Ch.R Worthing, S.B. Walker, The Pesticide Manual, 9. Auflage (1997), British Crop Protection Council beschrieben sind.

Der Wirkstoffgehalt der aus den handelsüblichen Formulierungen bereiteten Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe eignen sich auch zur Bekämpfung von Endo- und Ektoparasiten auf dem veterinärmedizinischen Gebiet bzw. auf dem Gebiet der Tierhaltung. Die Anwendung der erfindungsgemäßen Wirkstoffe geschieht hier in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion.

Die erfindungsgemäßen Verbindungen der Formel (I) können demgemäß auch besonders vorteilhaft in der Viehhaltung (z.B. Rinder, Schafe, Schweine und Geflügel wie Hühner, Gänse usw.) eingesetzt werden. In einer bevorzugten Ausführungsform der Erfindung werden den Tieren die Verbindungen, gegebenenfalls in geeigneten Formulierungen und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die neuen Verbindungen können bei Rindern z.B. in Dosierungen von 0,01 bis 1 mg/kg Körpergewicht eingesetzt werden.

Neben den bisher genannten Applikationsverfahren zeigen die erfindungsgemässen Wirkstoffe der Formel (I) eine hervorragende systemische Wirkung. Die Wirkstoffe können daher auch über Pflanzenteile, unterirdische wie oberirdische (Wurzel, Stengel, Blatt), in die Pflanzen eingebracht werden, wenn die Wirkstoffe in flüssiger oder fester Form in die direkte Umgebung der Pflanze appliziert werden (z.B. Granulate in der Erdapplikation, Applikation in gefluteten Reisfeldern).

Daneben sind die erfindungsgemäßen Wirkstoffe in besonderer Weise zu Behandlung von vegetativen und generativen Vermehrungsmaterial einsetzbar, wie von Saatgut von beispielsweise Getreide, Gemüse, Baumwolle, Reis, Zuckerrübe und anderen Kultur- und Zierpflanzen, von Zwiebeln, Stecklingen und Knollen weiterer vegetativ vermehrter Kultur- und Zierpflanzen. Die Behandlung hierfür kann vor der Saat bzw. dem Pflanzvorgang erfolgen (z.B. durch spezielle Techniken der Saatgutbeizung, durch Beizung in flüssiger oder fester Form oder Seedboxtreatment), während des Saatvorgangs bzw. des Pflanzens oder nach dem Saat- bzw. Pflanzvorgang durch spezielle Applikationstechniken (z.B. Saatreihenbehandlung). Die angewandte Wirkstoffmenge kann entsprechend der Anwendung in einem größerem Bereich schwanken. Im allgemeinen liegen die Aufwandmengen zwischen 1 g und 10 kg Wirkstoff pro Hektar Bodenfläche.

Die Verbindungen der Formel (I) können auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pflanzenschutzmitteln, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung in wirtschaftlich bedeutenden transgenen Kulturen von Nutz- und Zierpflanzen, z.B. von Getreide, wie Weizen, Gerste, Roggen, Hafer, Hirse, Reis, Maniok und Mais, oder auch Kulturen von Zuckerrübe, Baumwolle, Soja, Raps, Kartoffel, Tomate, Erbse und anderen Gemüsesorten.

Bei der Anwendung der in transgenen Kulturen, insbesondere mit Insektenresistenz, treten neben den in anderen Kulturen zu beobachtenden Wirkungen gegenüber Schadorganismen oftmals Wirkungen auf, die für die Applikation in der jeweiligen transgenen Kultur spezifisch sind, beispielsweise ein verändertes oder speziell erweitertes Schädlingsspektrum, das bekämpft werden kann, oder veränderter Aufwandmengen, die für die Applikation eingesetzt werden können.

Gegenstand der Erfindung ist deshalb auch die Verwendung von Verbindungen der Formel (I) zur Bekämpfung von Schadorganismen in transgenen Kulturpflanzen.

Die Anwendung der erfindungsgemäßen Verbindungen beinhaltet neben direkter Applikation auf die Schädlinge jede andere Applikation, bei der Verbindungen der Formel (I) auf die Schädlinge wirken. Solche indirekten Applikationen können beispielsweise in der Anwendung von Verbindungen liegen, die beispielsweise im Boden, der Pflanze oder dem Schädling, zu Verbindungen der Formel (I) zerfallen oder abgebaut werden.

Auf den Inhalt der deutschen Patentanmeldung 198 58 192.0, deren Priorität die vorliegende Anmeldung beansprucht, und der Zusammenfassung wird hiermit ausdrücklich Bezug genommen; er gilt durch Zitat als Bestandteil dieser Beschreibung.

Nachfolgende Beispiele dienen zur Erläuterung der Erfindung, ohne sie damit einzuschränken.

### A. Chemische Beispiele

### Beispiel Nr. 1

### 5-Methyl-2-(4-trifluormethyl-3-pyridyl)-oxazol

4-Trifluormethylnicotinsäurepropionamid (6.2 g) und Quecksilber(ll)acetat (0.6 g) wurden in Essigsäure (250 ml) 3 Stunden lang am Rückfluß gekocht. Anschließend wurde das Reaktionsgemisch eingeengt, der Rückstand in gesättigter Natriumcarbonatlösung aufgenommen und mit Dichlormethan extrahiert. Die chromatographische Reinigung (Kieselgel, Heptan/Essigsäureethylester) des Rohprodukts ergab die gewünschte Verbindung als farbloses Öl.

¹H-NMR (CDCl₃, 300 MHz): 2.45 (s, 3H), 6.98 (s, 1H), 7.65 (d, J= 5 Hz, 1H), 8.84 (d, J = 5 Hz, 1H); 9.32 (s, 1H).

Als Nebenkomponente wurde reines 5-Formyl-2-(4-trifluormethyl-3-pyridyl)-oxazol als farbloses Öl erhalten.

¹H-NMR (CDCl₃, 300 MHz): 7.77 (d, J = 5 Hz, 1H), 8.05 (s, 1H), 9,00 (d, J = 5 Hz, 1H), 9.43 (s, 1H), 9.94 (s, 1H),

### Beispiel Nr. 2

### 5-n-Propylthiomethyl-2-(4-trifluormethyl-3-pyridyl)-oxazol

Zu einer Lösung von 5-Brommethyl-2-(4-trifluormethyl-3-pyridyl)-oxazol (160 mg) und n-Propanthiol (60 µL) in Methanol (7 ml) wurde Natriummethanolat (130 µL, 30 % in Methanol) gegeben und 4 Stunden lang bei Raumtemperatur gerührt. Anschließend wurde Wasser (50 ml) zugesetzt und mit Essigsäureethylester extrahiert. Die organische Phase wurde mit Wasser gewaschen, getrocknet (MgSO₄), filtriert und eingeengt. Auf diese Weise wurde das gewünschte Produkt in reiner Form als schwach gelbes Öl erhalten.

¹H-NMR (CDCl₃, 300 MHz): 1.02 (t, J = 7 Hz, 3H), 1.65 (quin, J = 7 Hz, 2H), 2.57 (t, J = 7 Hz, 2H), 3.81 (s, 2H), 7.16 (s, 1H), 7.70 (d, J = 5 Hz, 1H), 8.88 (d, J = 5 Hz, 1H), 9.35 (s, 1H).

In analoger Weise werden die in den Tabellen dargestellten Thioether hergestellt.

### Beispiel Nr. 3

### 5-Cyclopropyl-4-isopropyloxymethyl-2-(4-trifluormethyl-3-pyridyl)-oxazol

Zu einer Lösung von 4-Chlormethyl-5-cyclopropyl-2-(4-trifluormethyl-3-pyridyl)-oxazol (200 mg) in 2-Propanol (5 ml) wurde eine frisch hergestellte Lösung von Natriumisopropanolat (20 mg Na, 5 ml 2-Propanol) gegeben und 10 Stunden lang unter Rückfluß erhitzt. Anschließend wurde das Reaktionsgemisch eingeengt, mit Wasser versetzt und mit Dichlormethan extrahiert. Die organische Phase wurde mit gesättigter Natriumchloridlösung gewaschen, getrocknet (MgSO₄) und über Kieselgel filtriert. Nach Einengen der organische Phase wurde das Produkt in reiner Form als blaß gelbes Öl erhalten.

¹H-NMR (CDCl₃, 300 MHz): 0.99 - 1.09 (m, 4H), 1,28 (d, J = 7 Hz, 2H), 2.05 - 2.15 (M, 1H), 3.82 (quin, J = 7Hz, 1H), 4.56 (s, 2H), 7.73 (d, J = 5Hz, 1H), 8.80 (d, J = 5 Hz, 1H), 9.39 (s, 1H).

In analoger Weise werden die in den Tabellen dargestellten Ether hergestellt.

### Beispiel Nr. 4

### N-Acetyl, N-methyl-5-aminomethyl-2-(4-trifluormethyl-3-pyridyl)-oxazol

Zu einer Lösung von N-Methyl-5-aminomethyl-2-(4-trifluormethyl-3-pyridyl)-oxazol (150 mg) und Triethylamin (90 µL) in Dichlormethan (5 mL) wurde Acetylchlorid (40 µL) gegeben und eine Stunde bei Raumtemperatur gerührt. Anschließend wurde mit Wasser und gesättigten Natriumhydrogencarbonat gewaschen, getrocknet (MgSO₄), filtriert und eingeengt. Auf diese Weise wurde das reine Produkt als farbloses Öl erhalten. Das ¹H-NMR zeigt ein Gemisch von Rotameren (2:1 Verhältnis).
¹H-NMR (CDCl₃; 300 MHz):
2.15 und 2.28 (s, 3H), 300 und 3.14 (s, 3H), 4.60 und 4.70 (s, 2H), 7.22 (s, 1H), 7.68 und 7.72 (d, J = 5 Hz, 1H), 8.89 und 8.93 (d, J = 5H, 1H), 9.36 (s, 1H).

In analoger Weise werden die in den Tabellen dargestellten Amide, Carbamate, Thiocarbamate, Sulfonamide und Hamstoffderivate hergestellt.

### Beispiel 5

### 4-Ethoxycarbonyl-5-ethyl-2-(4-trifluormethyl-3-pyridyl)-oxazol

Zu einer Lösung von Propionoylessigsäureethylester (7.6 g) in Essigsäure (8.0 mL) wurde eine Lösung von Natriumnitrit (4.0 g) in Wasser (6 mL) gegeben (Temperatur 0 bis 10°C). Nach einer Stunde bei Raumtemperatur wurden 30 %ige Schwefelsäure (66 mL) und Eiswasser (80 ml) zugesetzt. Anschließend wurde bei starker Kühlung und kräftigem Rühren Zinkpulver (62 g) zugegeben. Nach 15 Minuten wurde das überschüssige Zink abfiltriert und das Filtrat unter kräftigem Rühren mit Natriumacetat (48.3 g) und frisch hergestelltem 4-Trifluormethylpyridin-3-carbonsäurechforid (11.5 g) versetzt. Nach 2 Stunden bei Raumtemperatur wurde das Reaktionsgemisch mit Dichlormethan extrahiert. Die organische Phase wurde mit gesättigter Natriumhydrogencarbonatlösung und Wasser gewaschen, getrocknet (MgSO₄), filtriert und eingeengt.
Chromatographische Reinigung (Kieselgel, Heptan/ Essigsäureethylester ergab 2-(4-Trifluormethyl-3-amido)acetessigsäureethylester. Diese Verbindung (13.2 g) wurde in Essigsäureanhydrid (27 mL) mit Schwefelsäure (2,3 mol) versetzt und anschließend eine Stunde bei 90°C gerührt. Nach Abkühlen wurde das Reaktionsgemisch eingeengt, mit einer gesättigter Natriumhydrogencarbonatlösung basisch gestellt, mit Essigsäureethylester extrahiert, getrocknet (MgSO₄), filtriert und eingeengt.
Auf diese Weise wurde das gewünschte Produkt in reiner Form als farblose Kristallen erhalten (Fp. 76-77°C).
¹H-NMR (CDCl₃, 300 MHz): 1.33 (t, J = 7 Hz, 3H), 1.42 (t, J = 7 Hz, 3H), 3.16 (q, J = 7 Hz, 2 H), 4.45 (q, J = 7 Hz, 2H), 7.70 (d, J = 5 Hz, 1H), 8.90 (d, J = 5 Hz, 1H), 9.38 (s, 1H).

In analoger Weise werden die in den Tabellen dargestellten 4-Ethoxycarbonyl-5-alkyl-oxazolderivate hergestellt.

### Beispiel 6

### 5-iso-Propyl-4-dimethylaminocarbonyl-2-(4-trifluormethyl-3-pyridyl)-oxazol

Eine Suspension von 4-Carboxyl(-5-isopropyl-2-(4-trifluormethyl-3-pyridyl)-oxazol (0.8 g) und 1,1'-carbonyldiimidazol (0.5 g) in 1,4-Dioxan (70 mL) wurde 2 Stunden lang bei 80°C gerührt. Anschließend wurde bei 50°C 30 Minuten lang Dimethylamin eingeleitet und eine Stunde nachgerührt. Nach dem Abkühlen wurde Wasser (400 ml) zugegeben und mit Dichlormethan extrahiert. Die organische Phase wurde mit 5 %iger wäßriger Kaliumhydrogensulfat und Wasser gewaschen, getrocknet (MgSO₄) und filtriert. Nach Einengen der organischen Phase wurde das Produkt in reiner Form als farblose Kristallen erhalten (Fp. 91-92°C)
¹H-NMR (CDCl₃, 300 MHz): 1.37 (d, J = 7 Hz, 6H), 3.12 (s 3H), 3.35 (s, 3H), 3.70 (quin, J = 7 Hz, 1H),7.70 (d, J = 5 Hz, 1H), 8.8 g (d, J = 5 Hz, 1H), 9.36 (s, 1H).

In analoger Weise werden die in den Tabellen dargestellten Oxazolcarboxamide hergestellt.

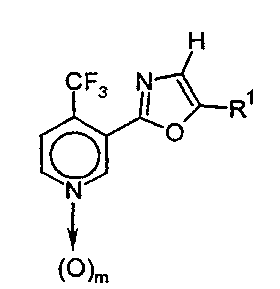

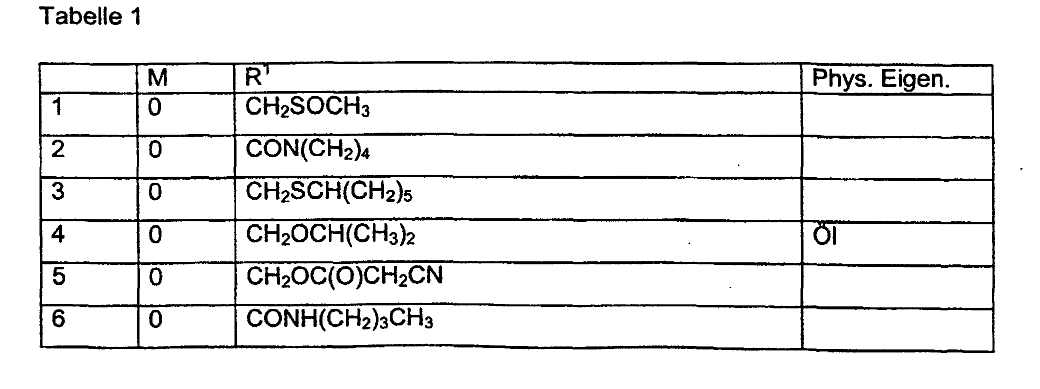

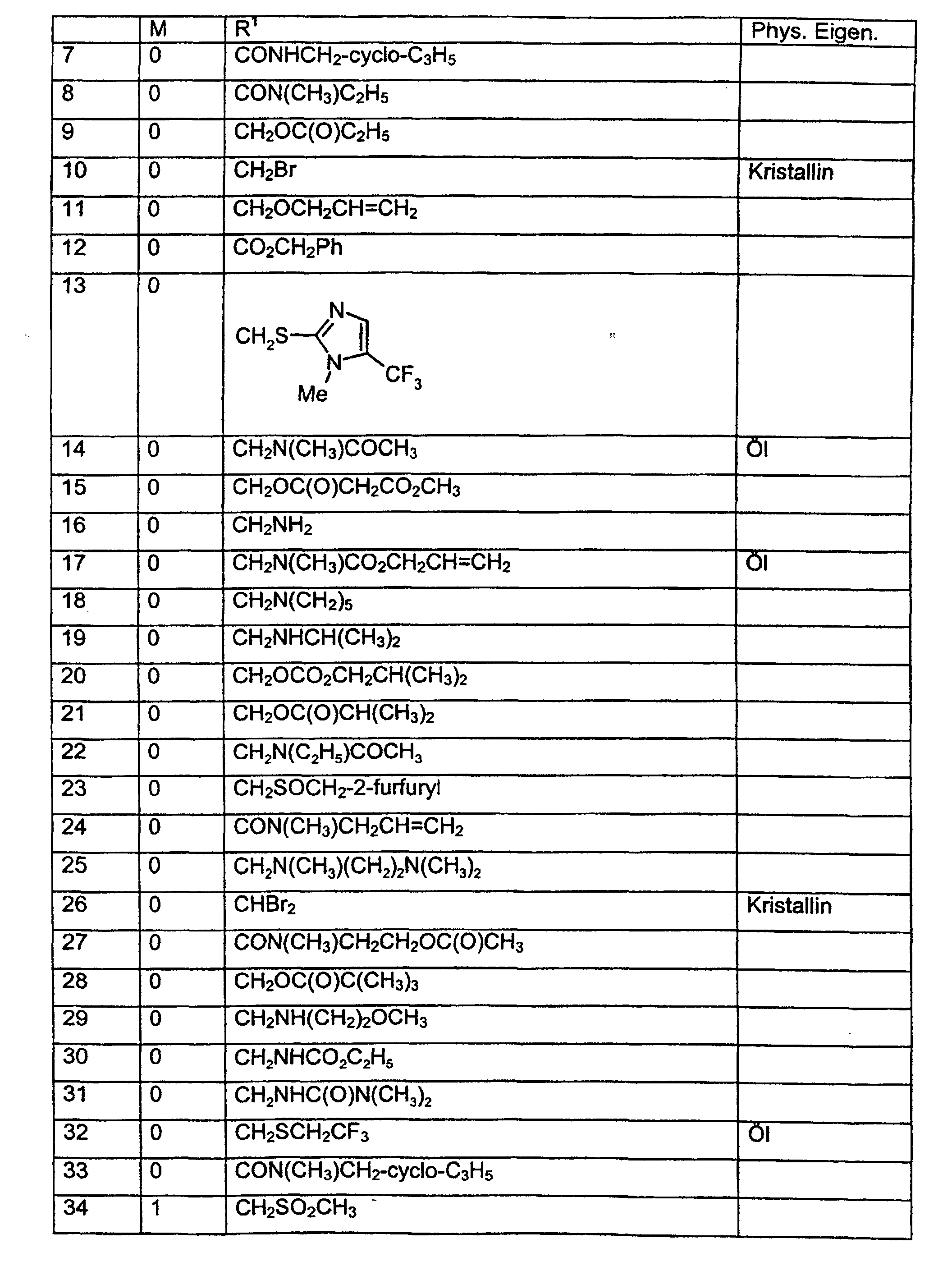

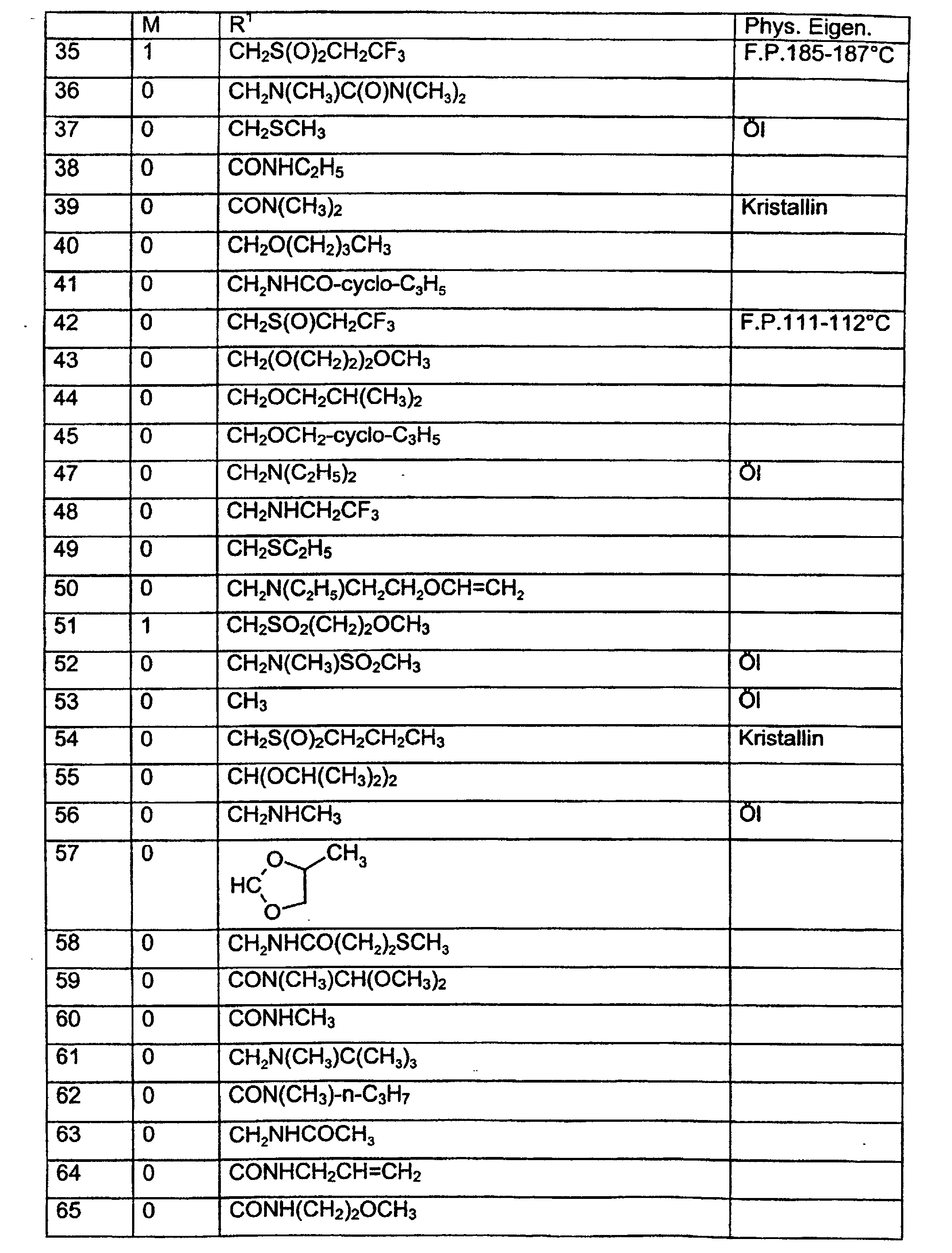

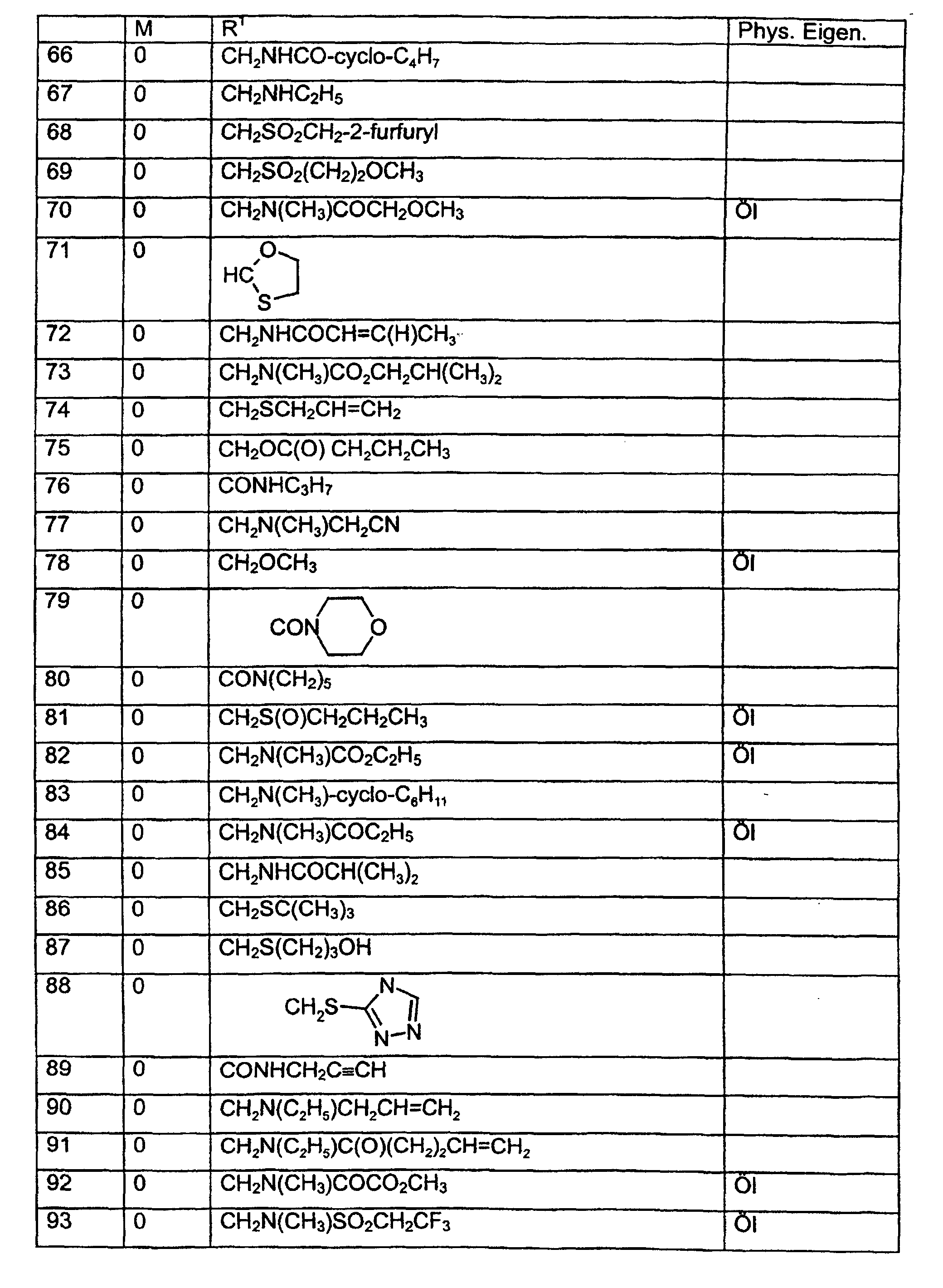

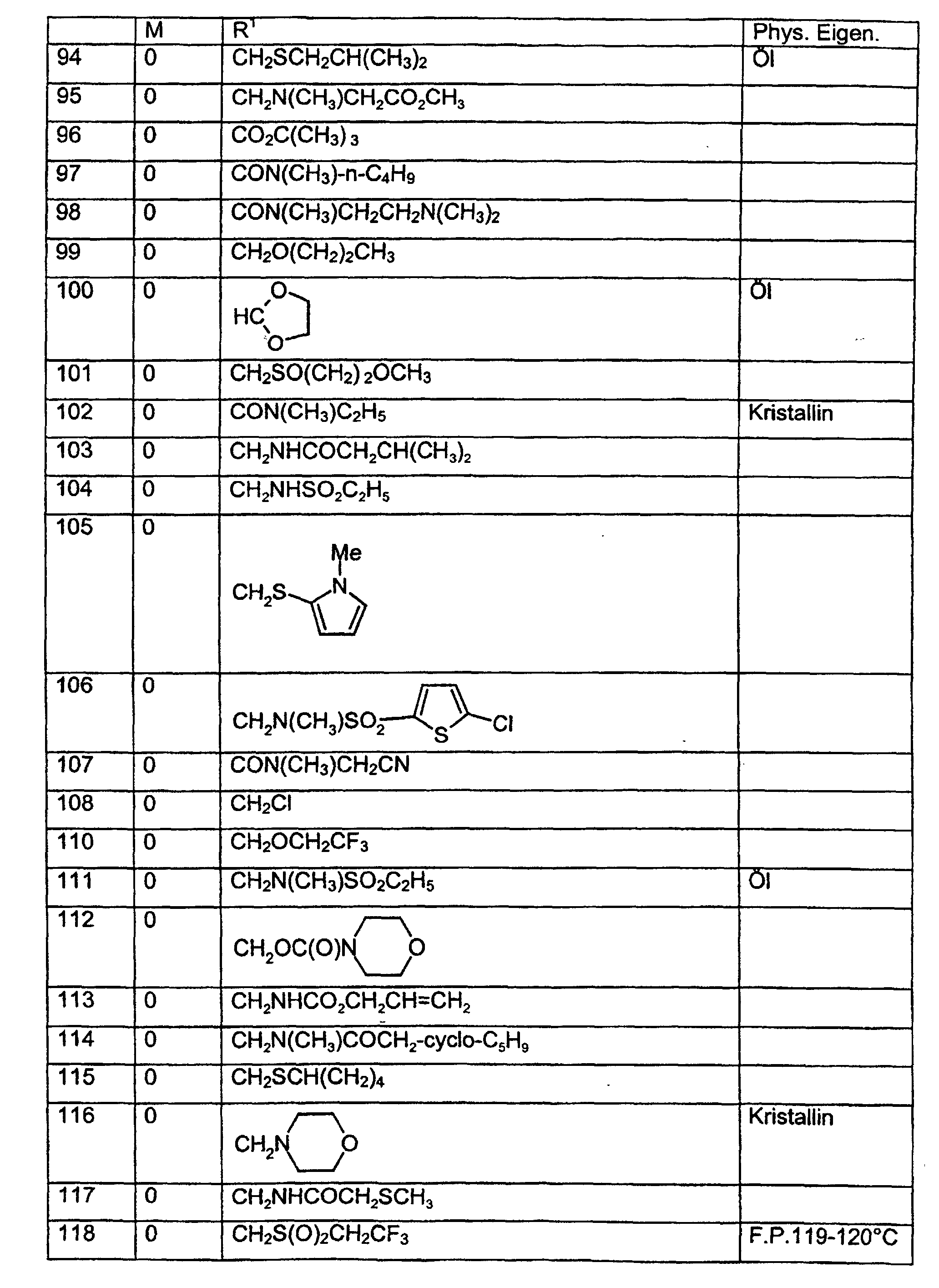

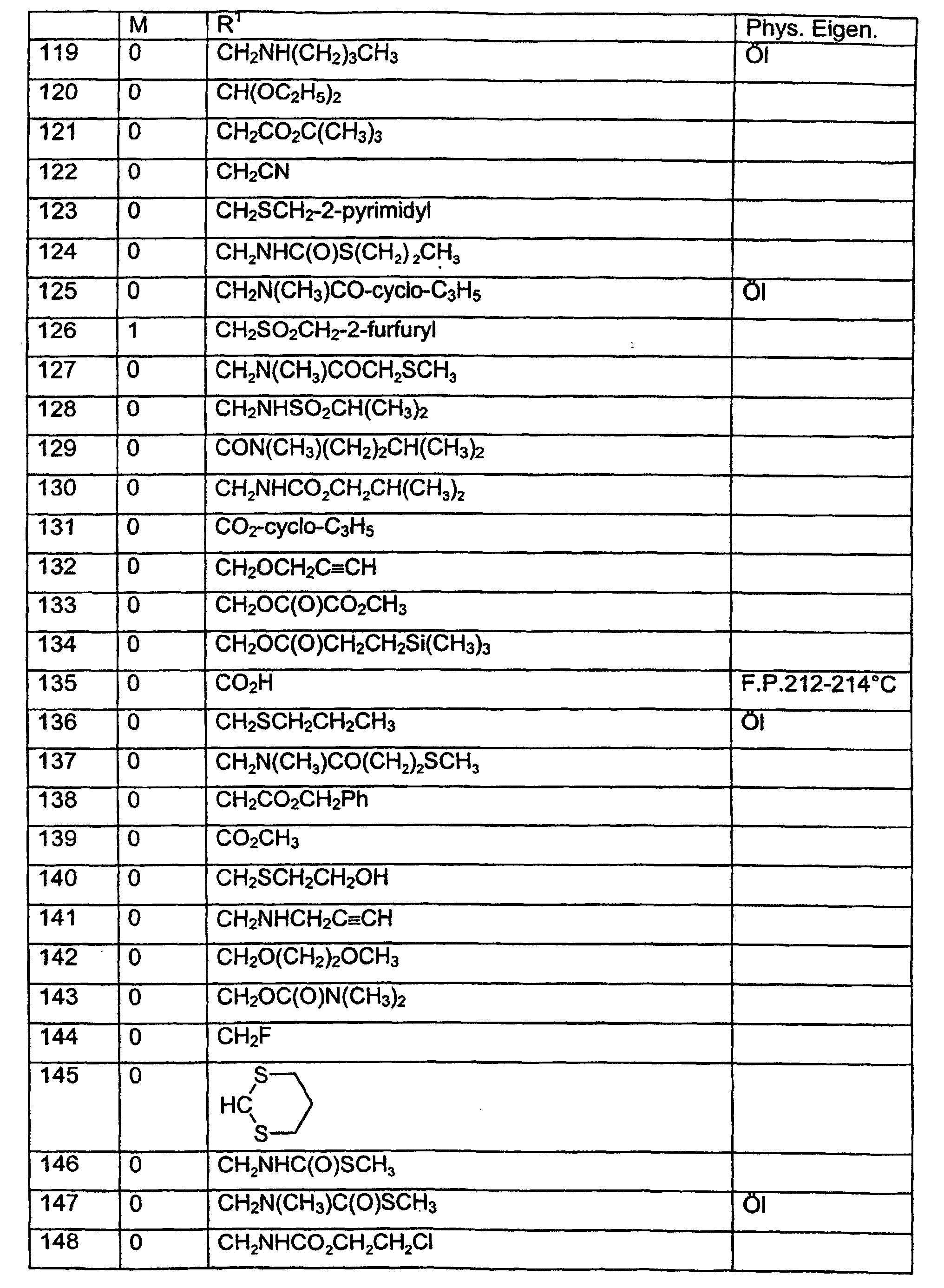

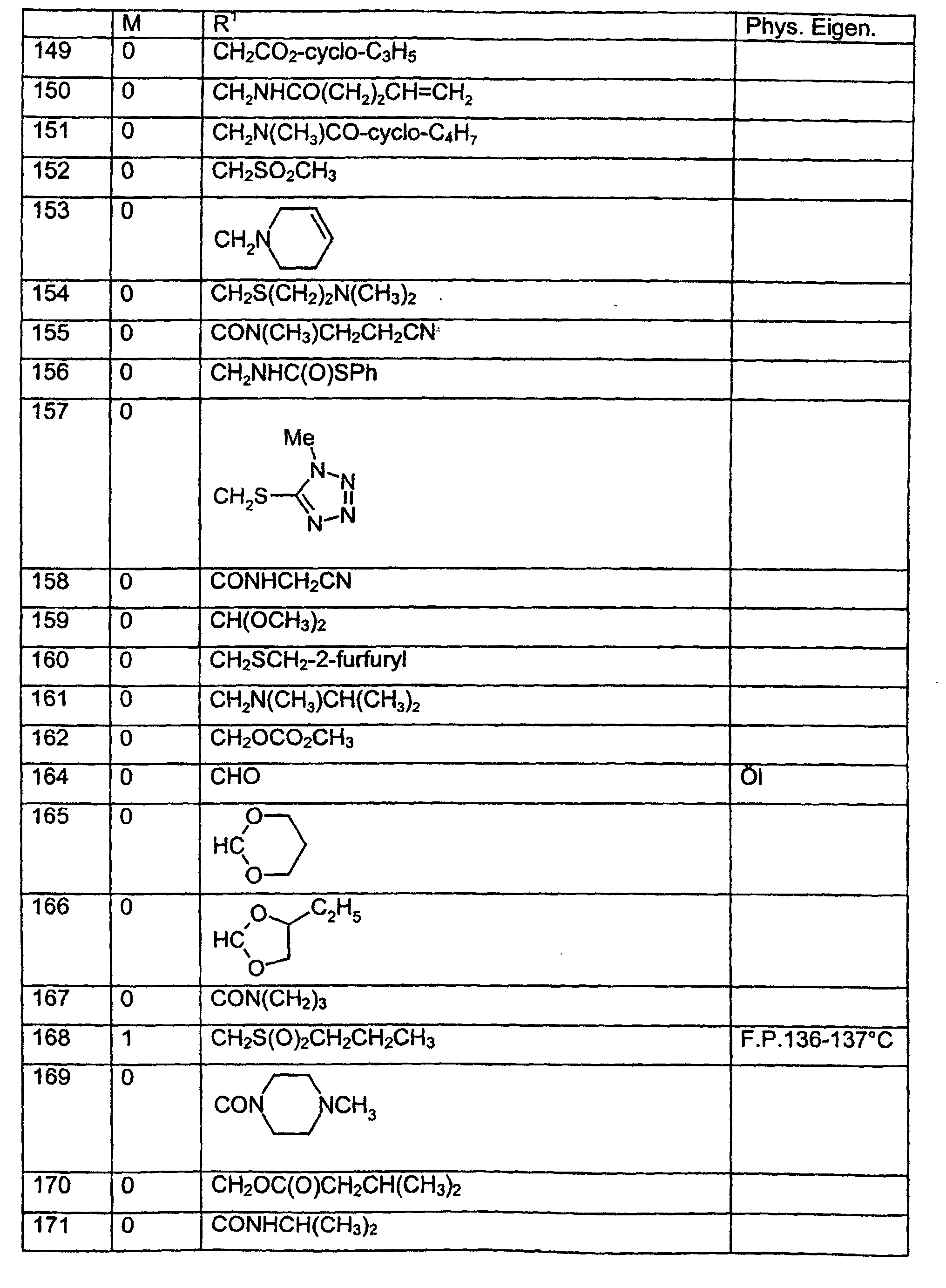

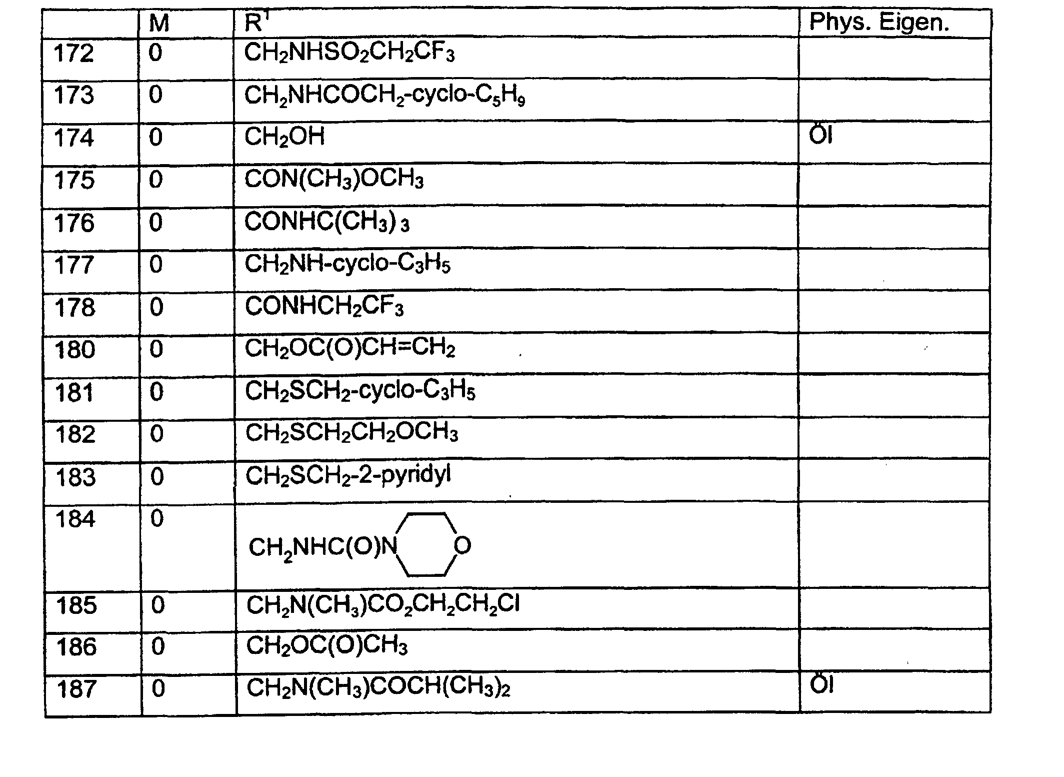

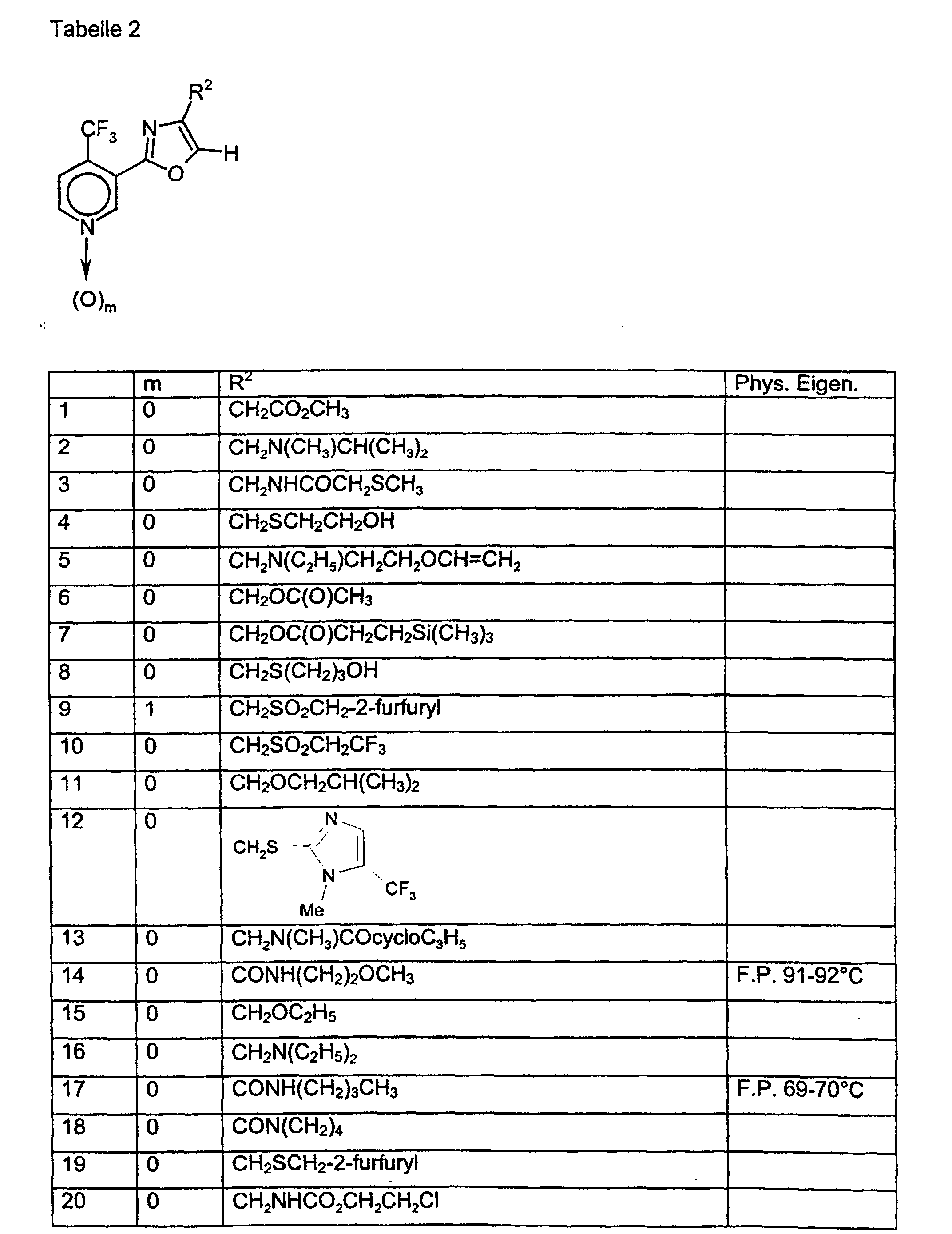

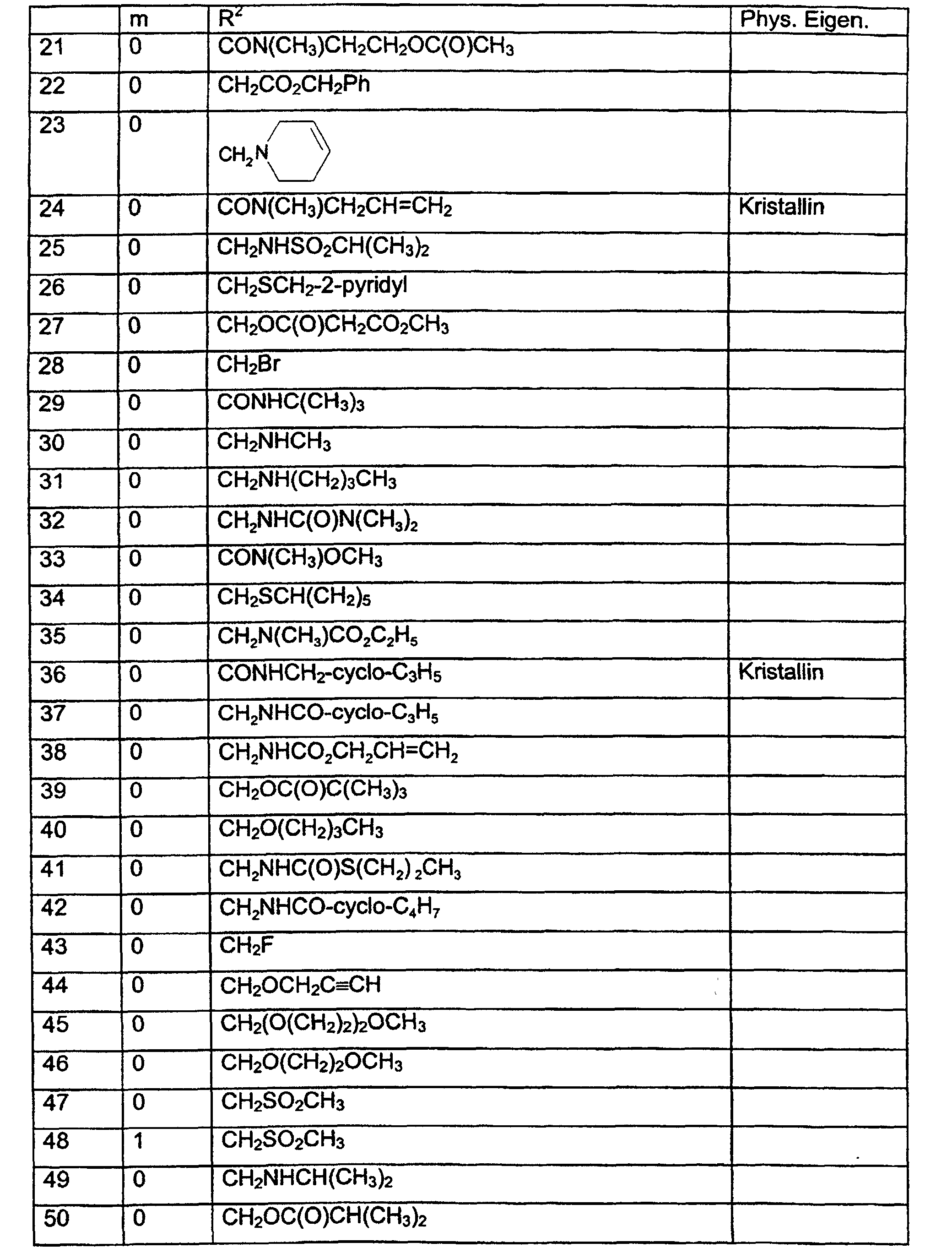

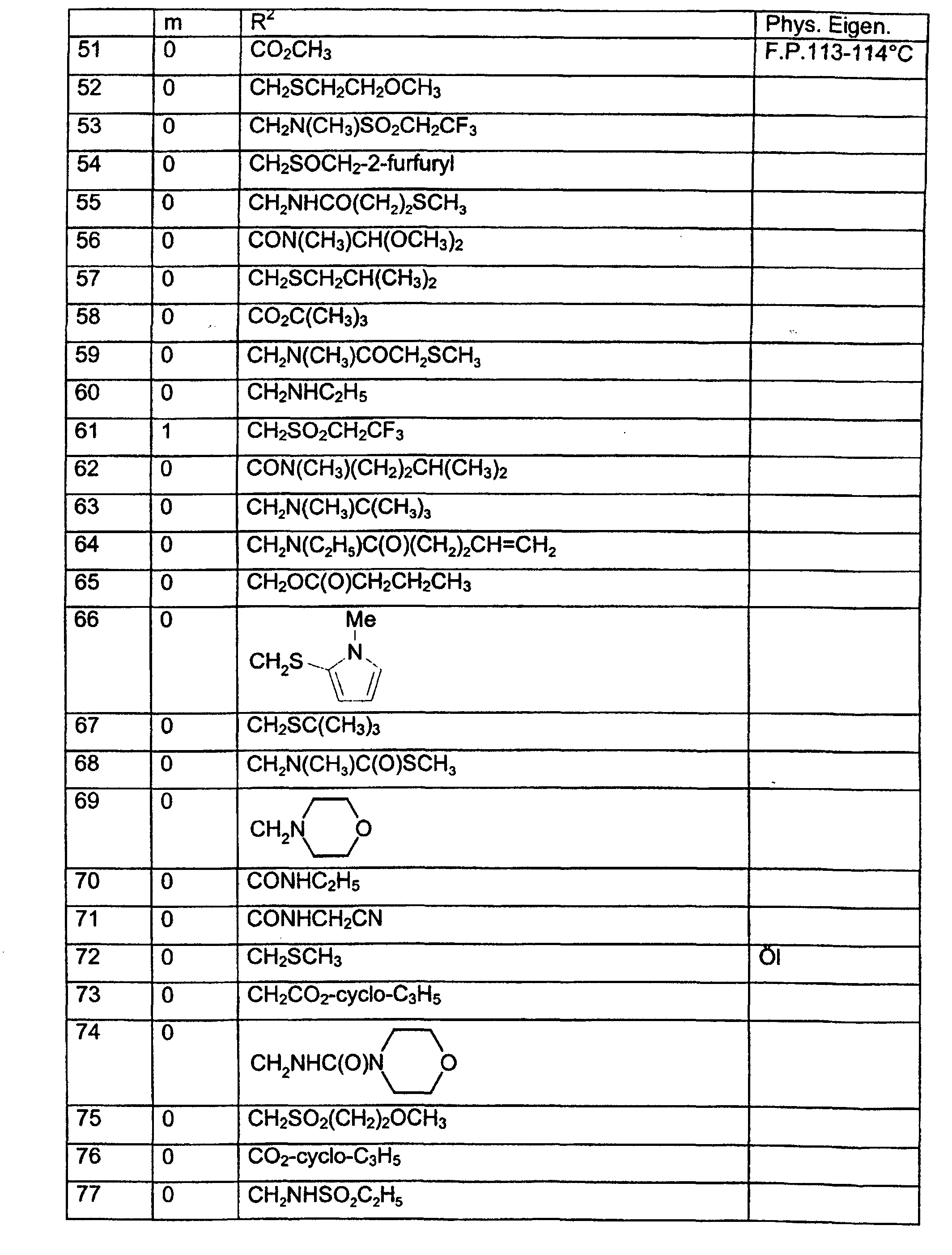

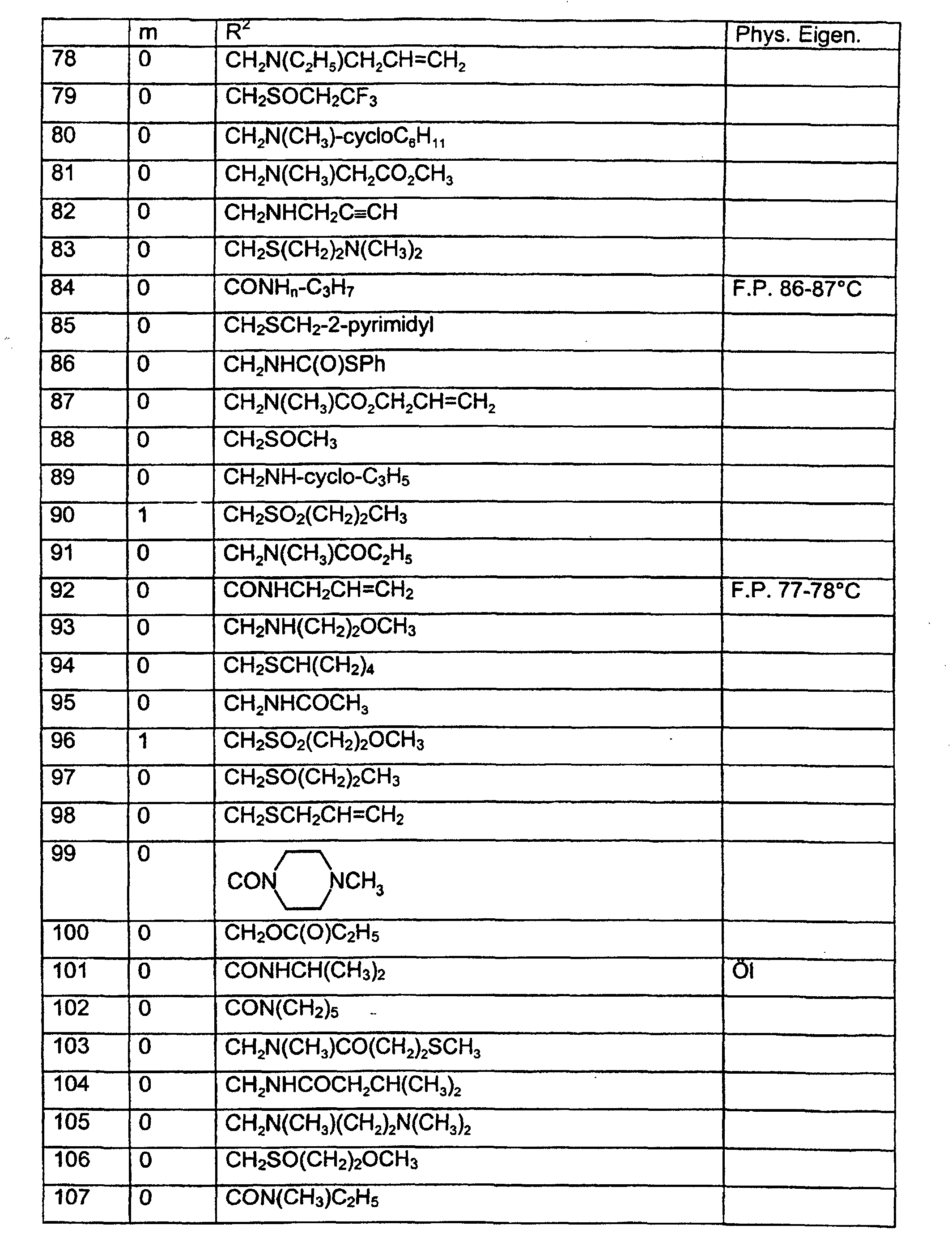

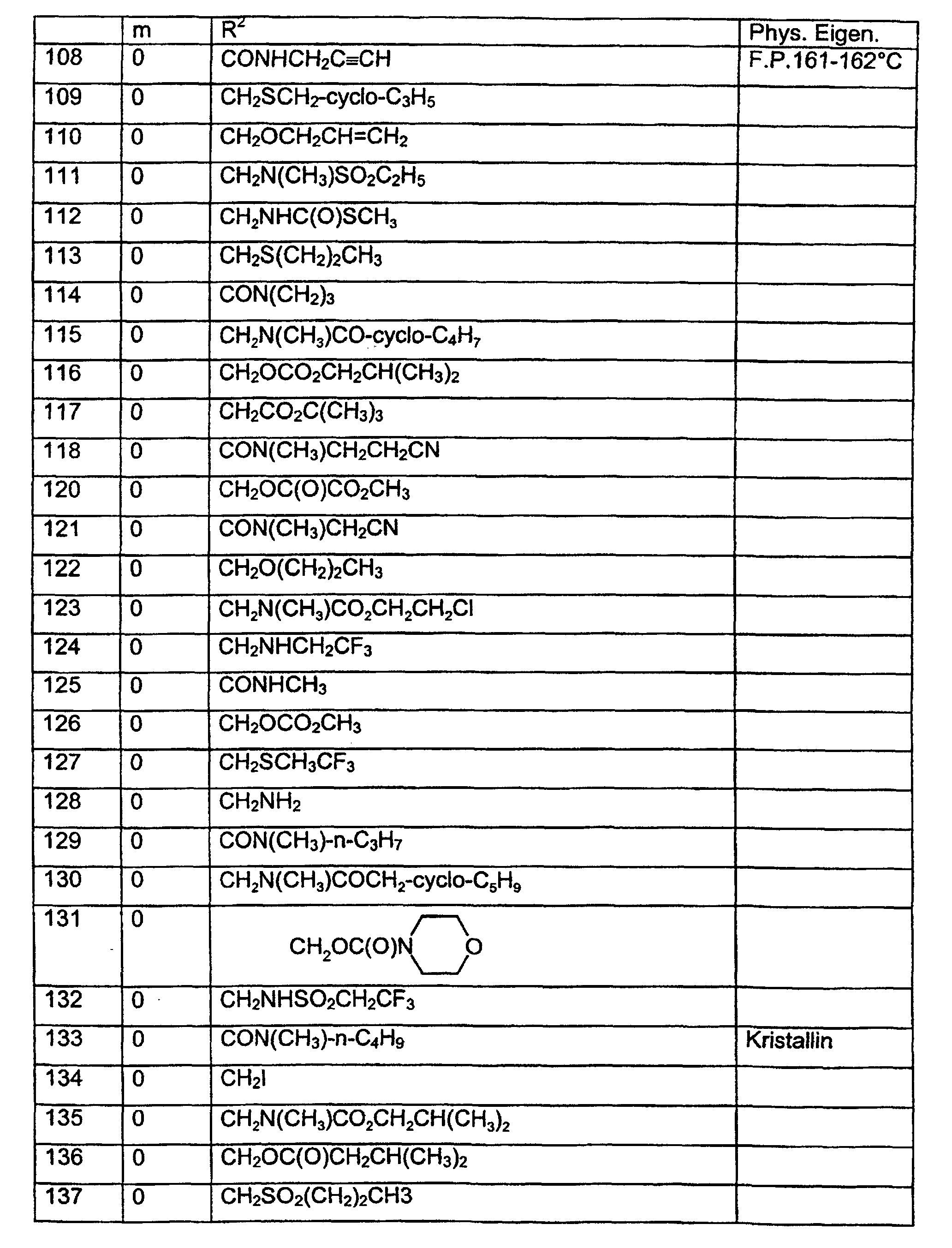

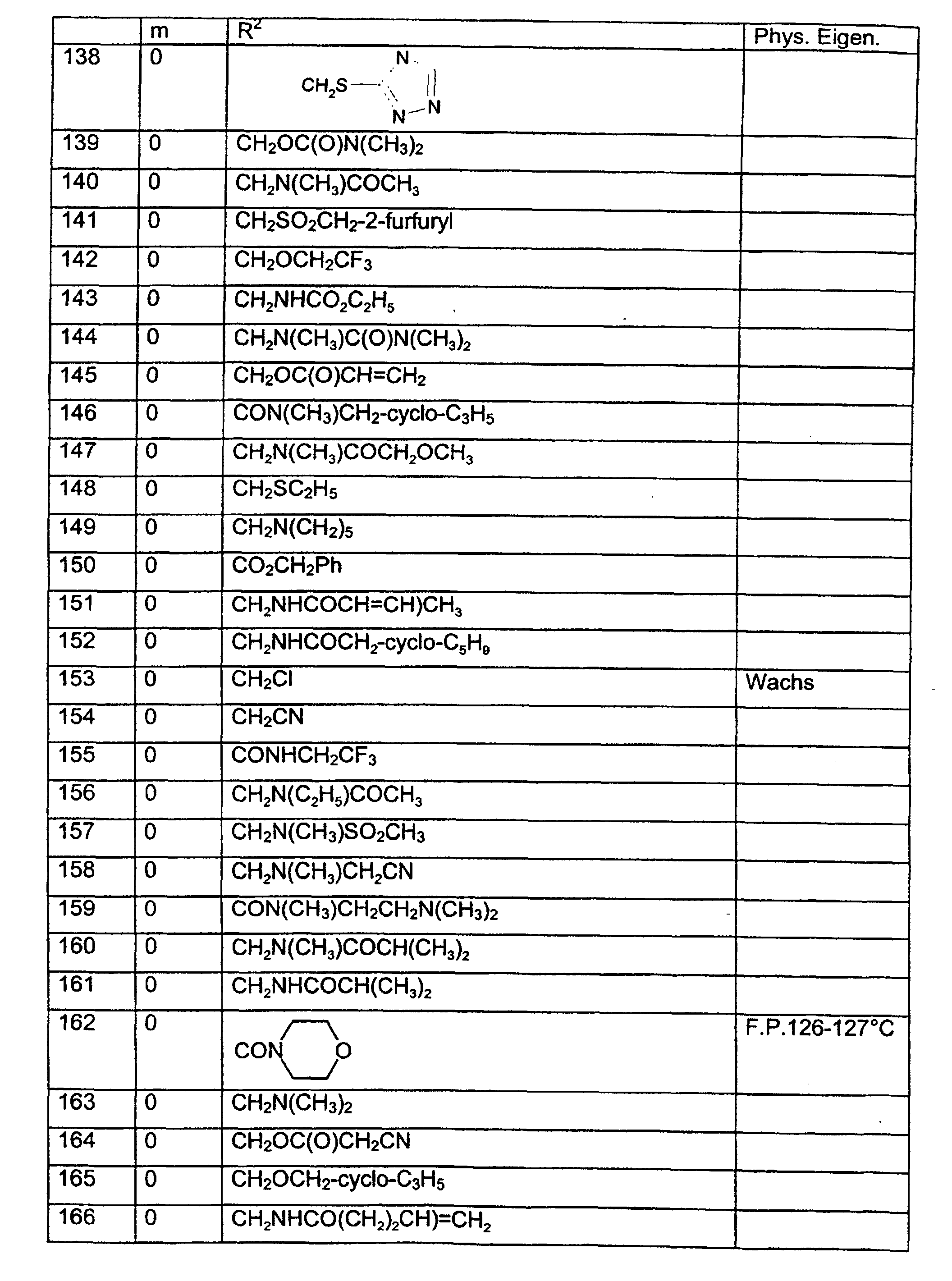

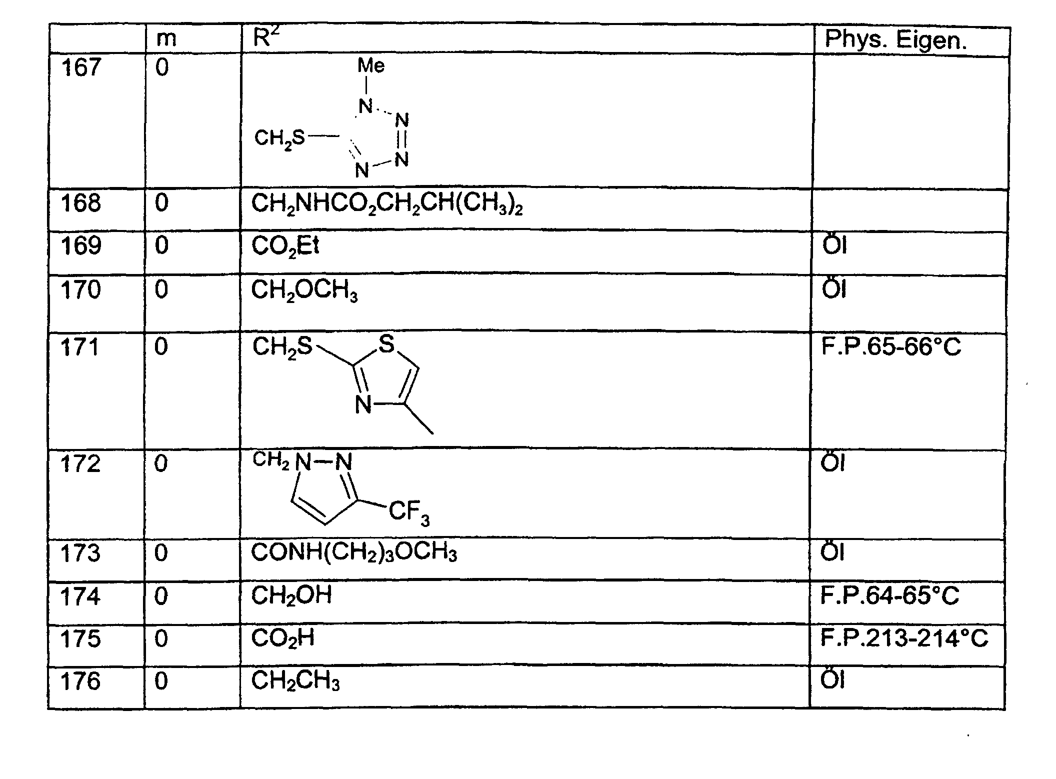

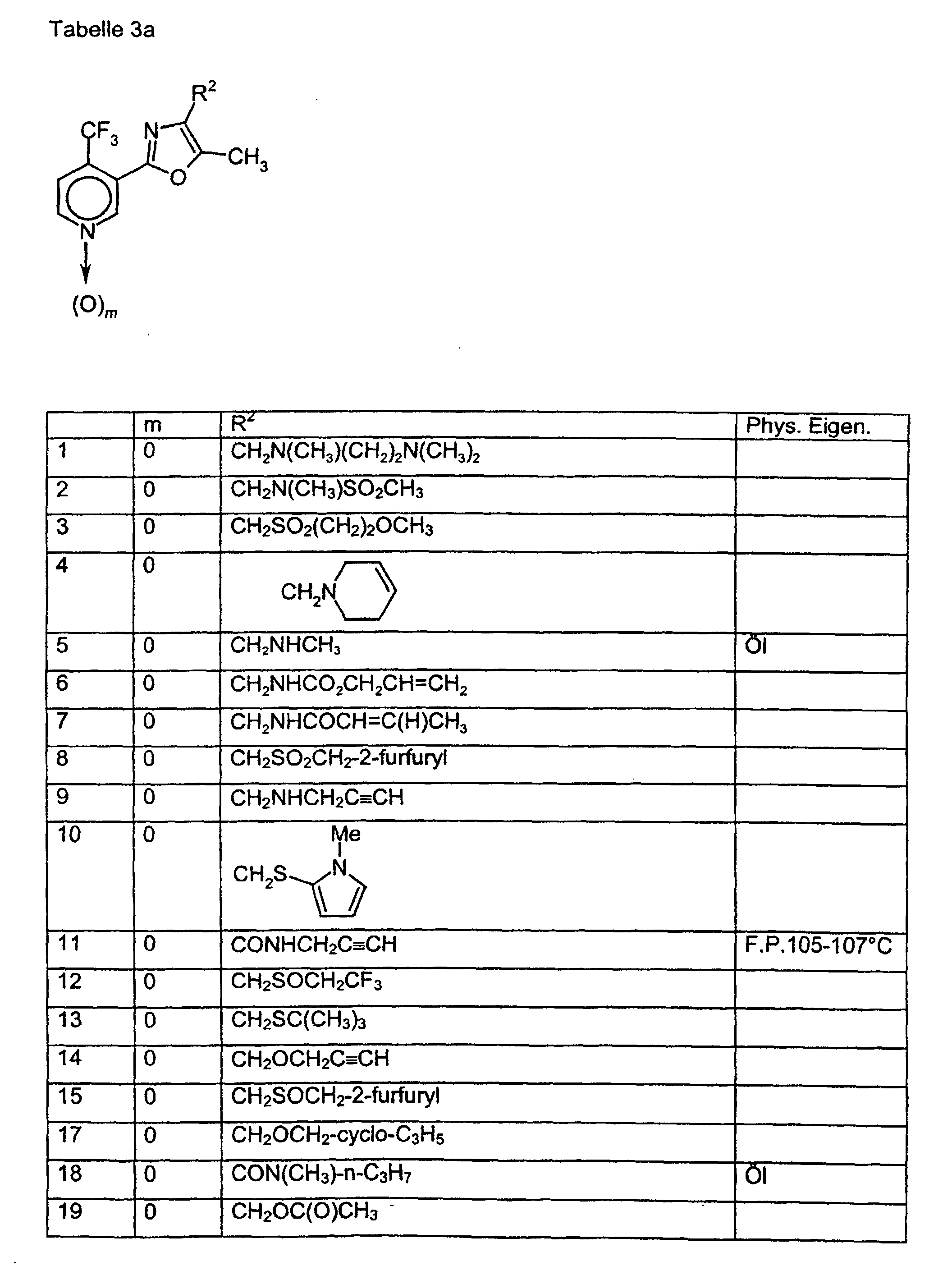

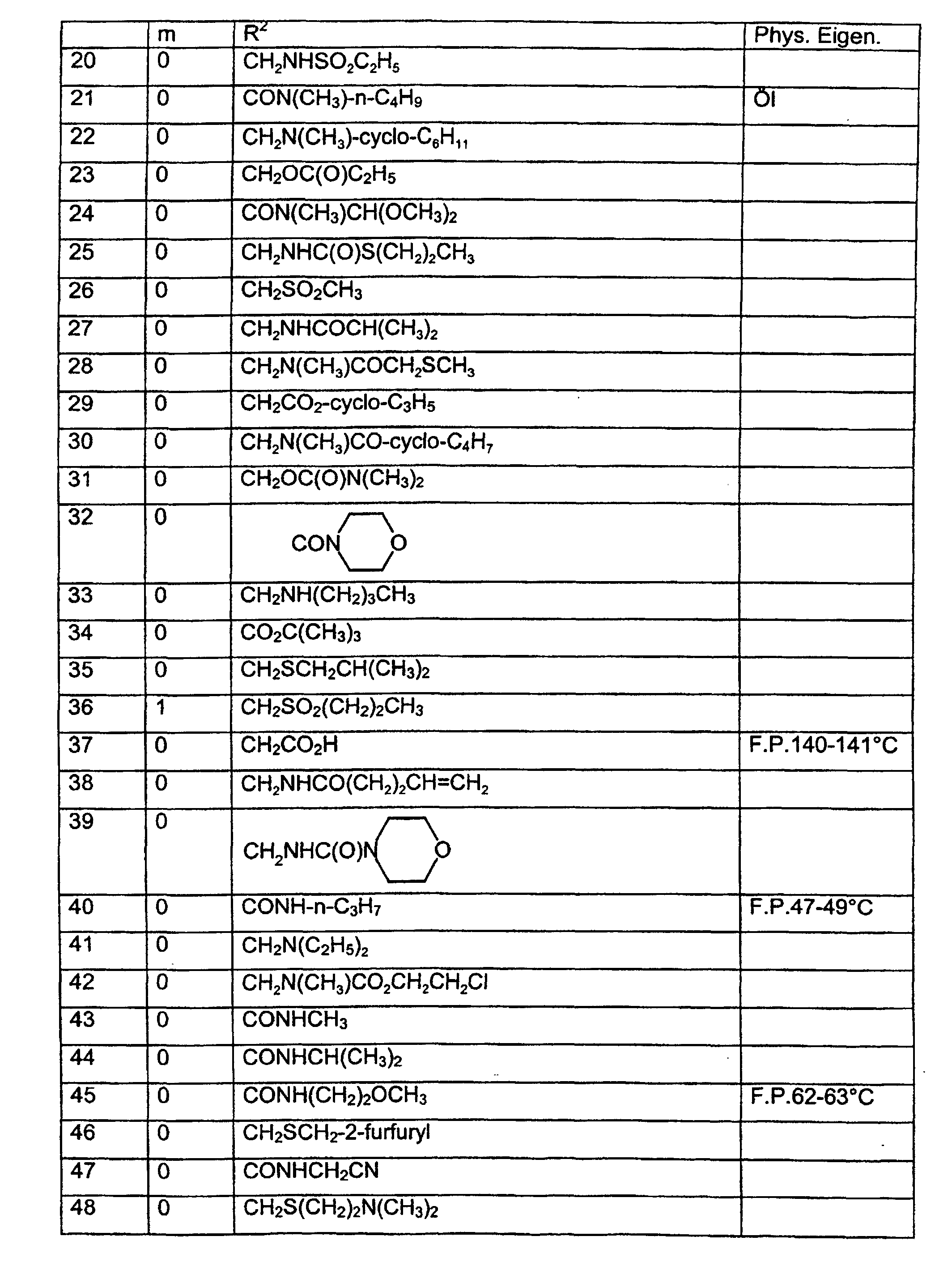

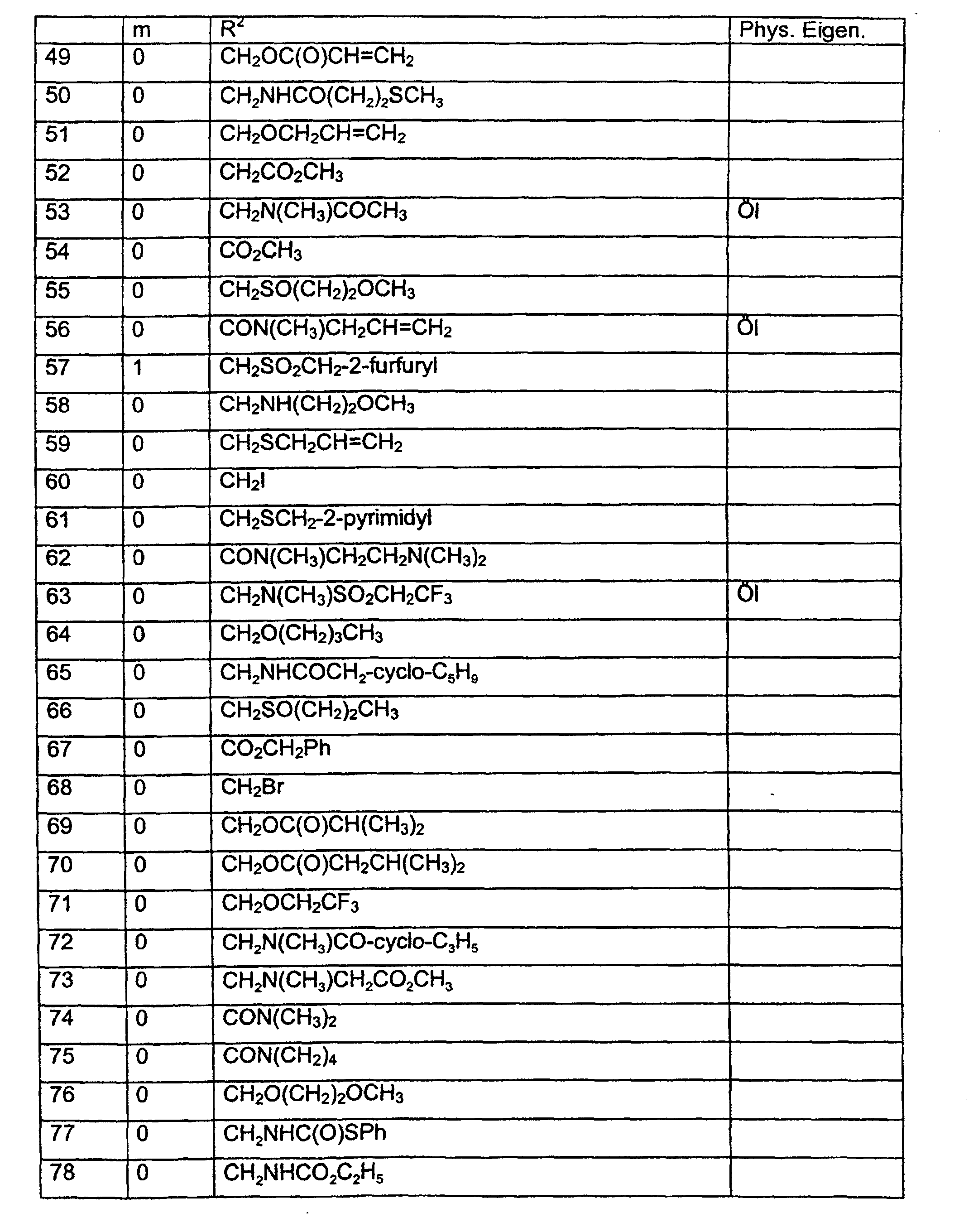

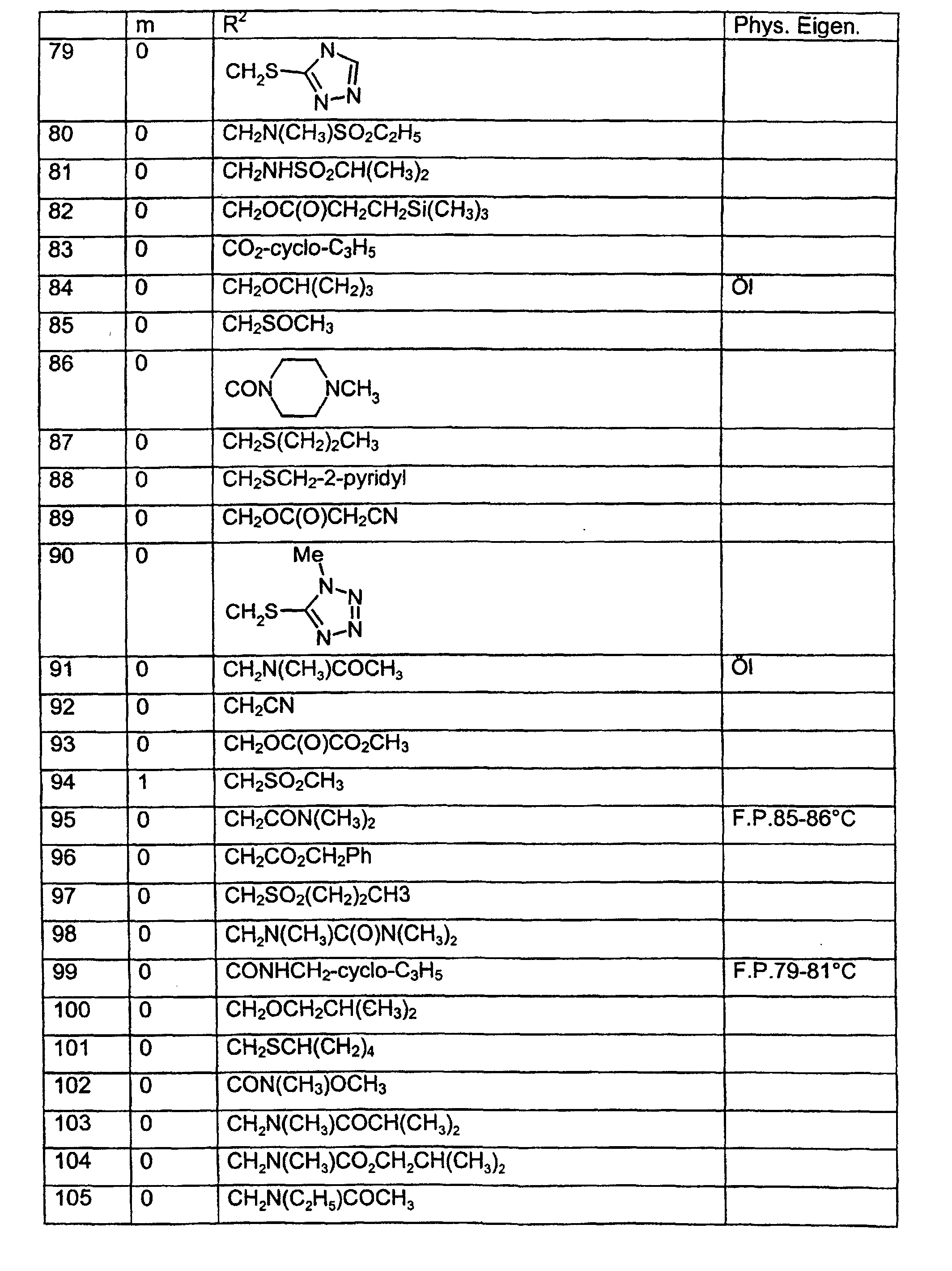

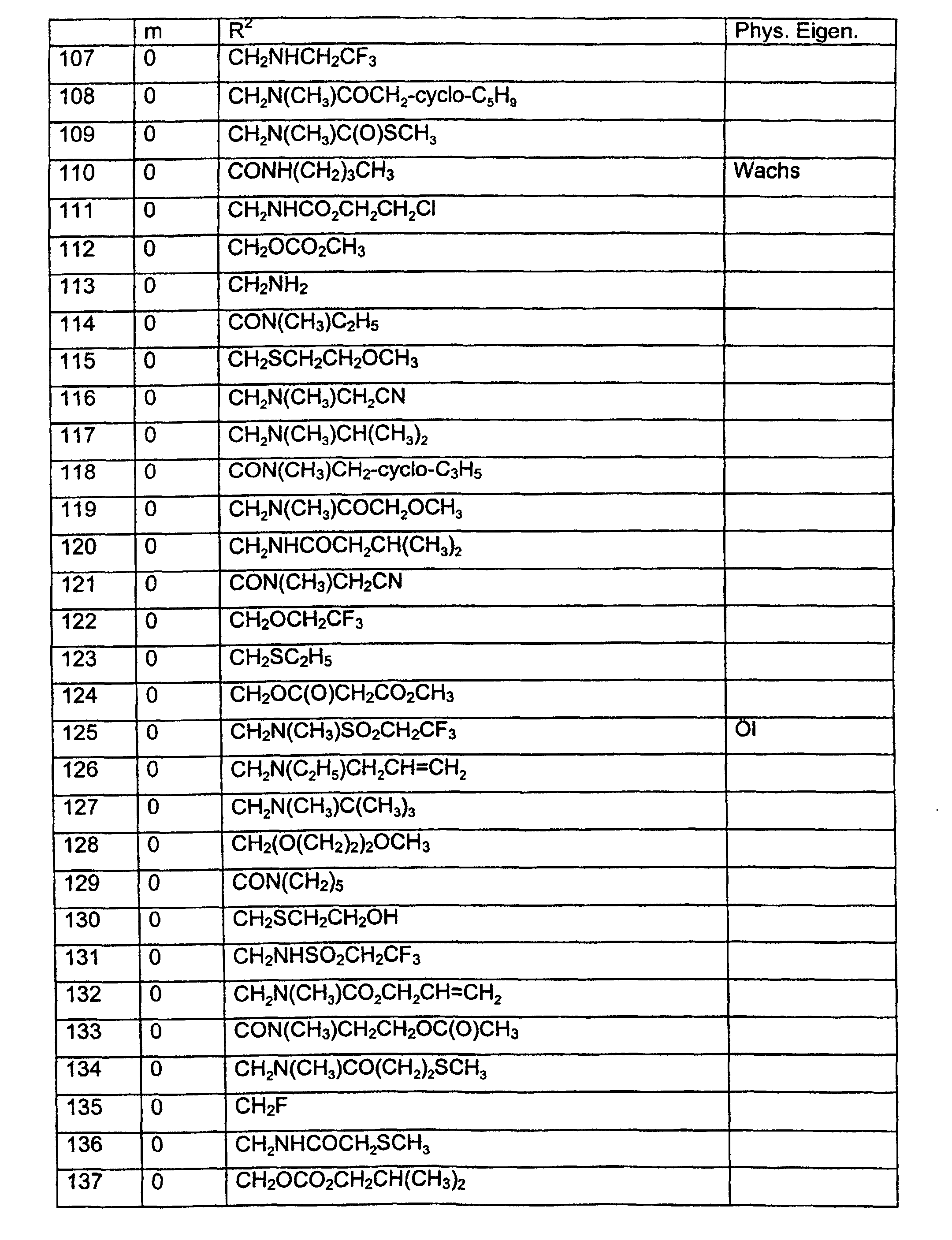

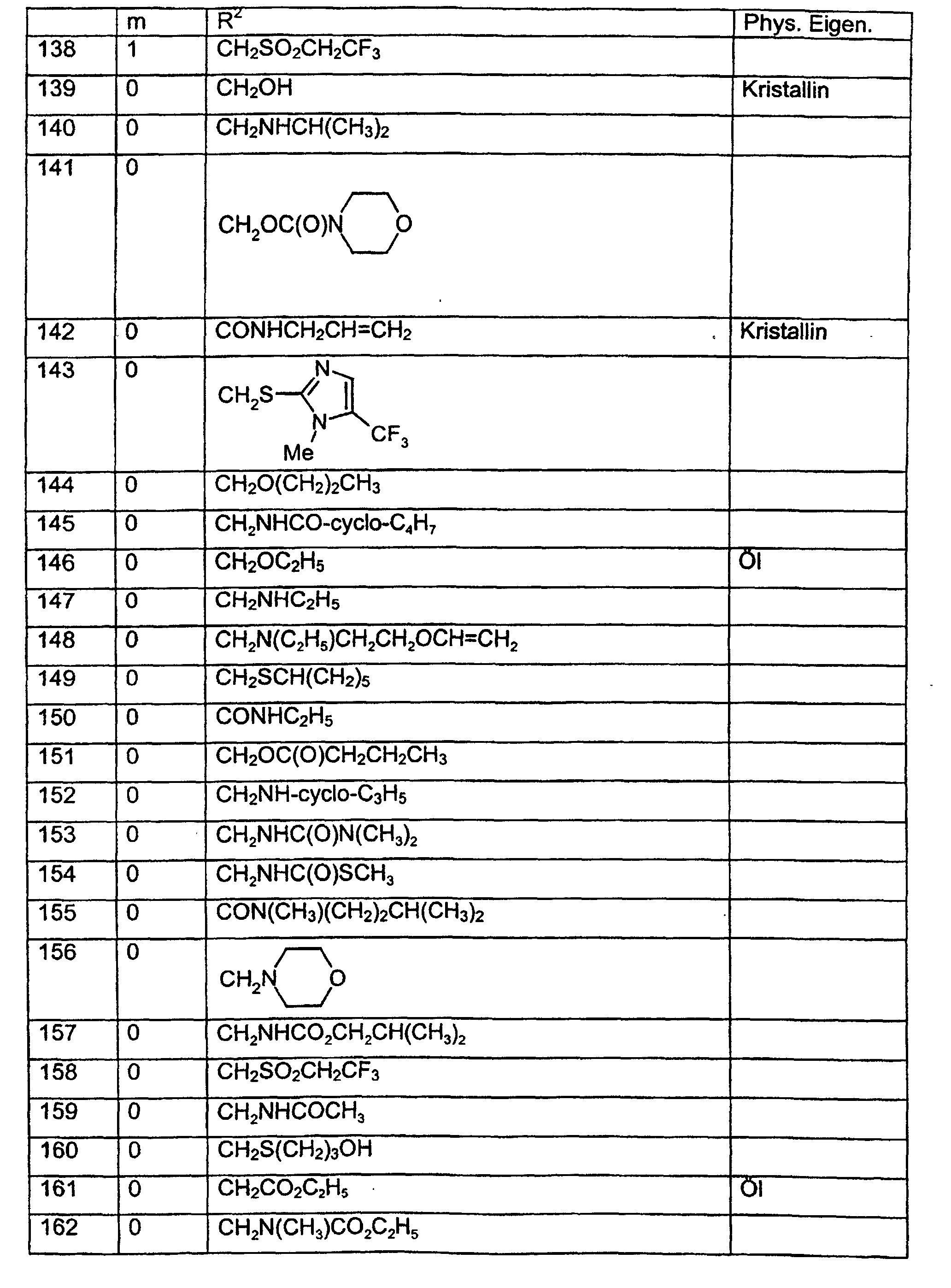

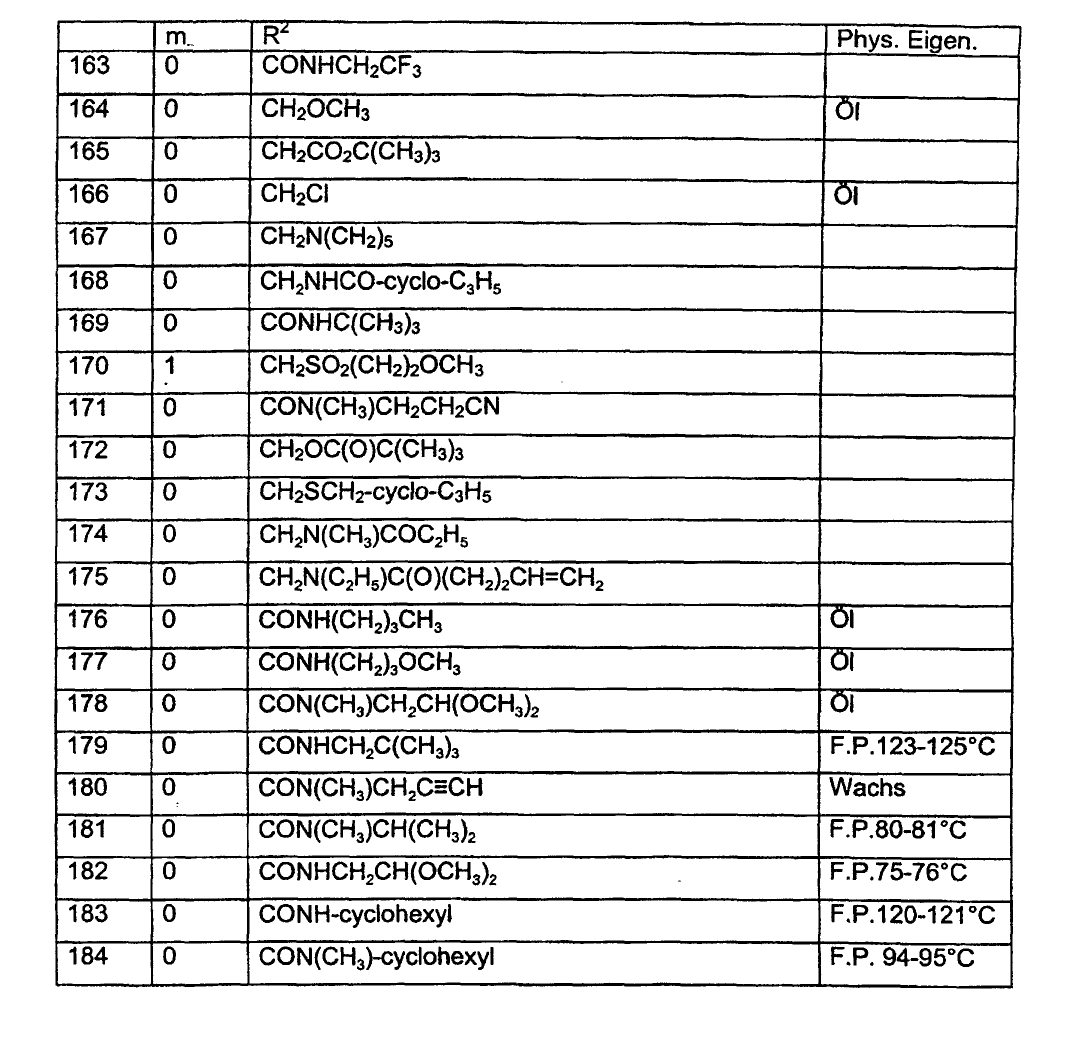

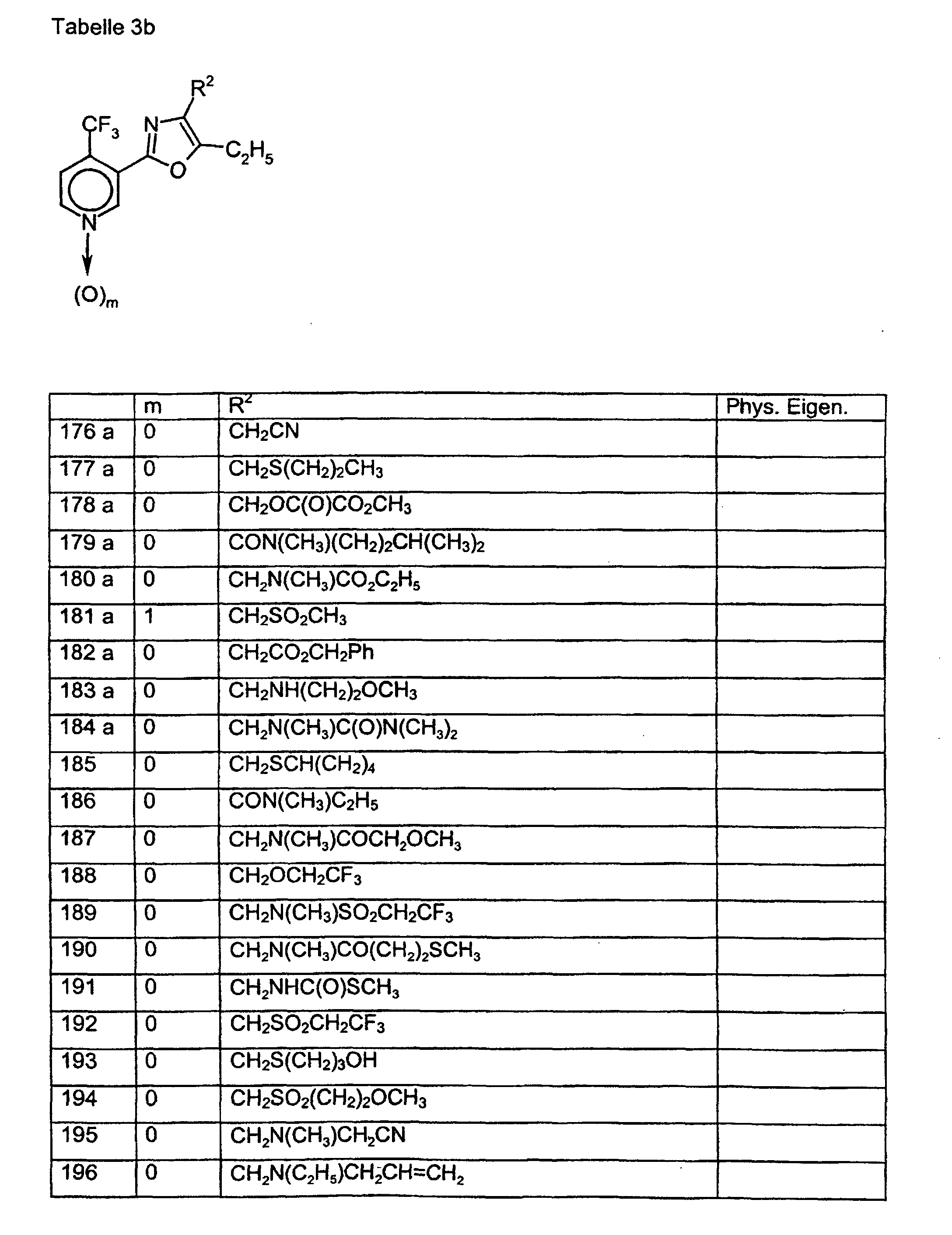

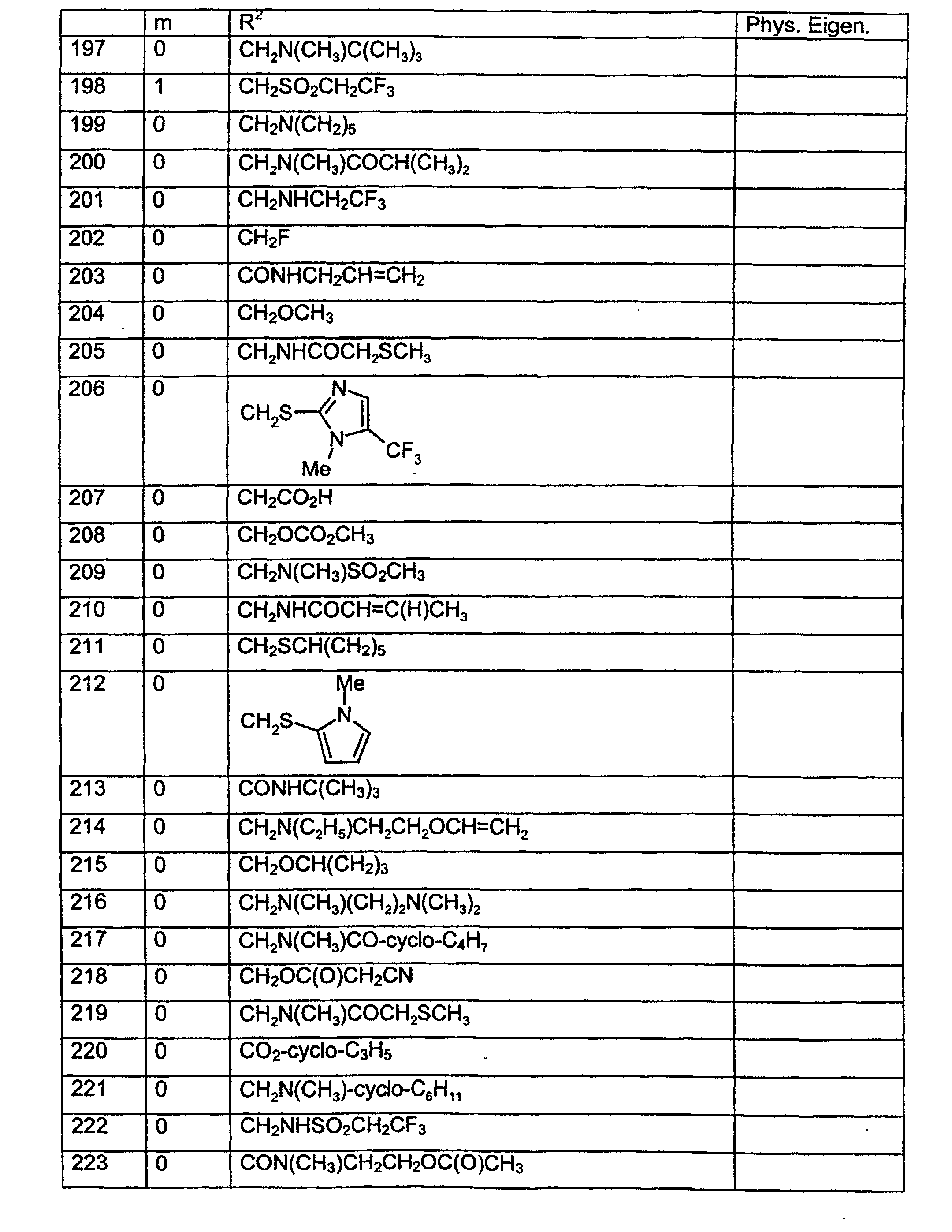

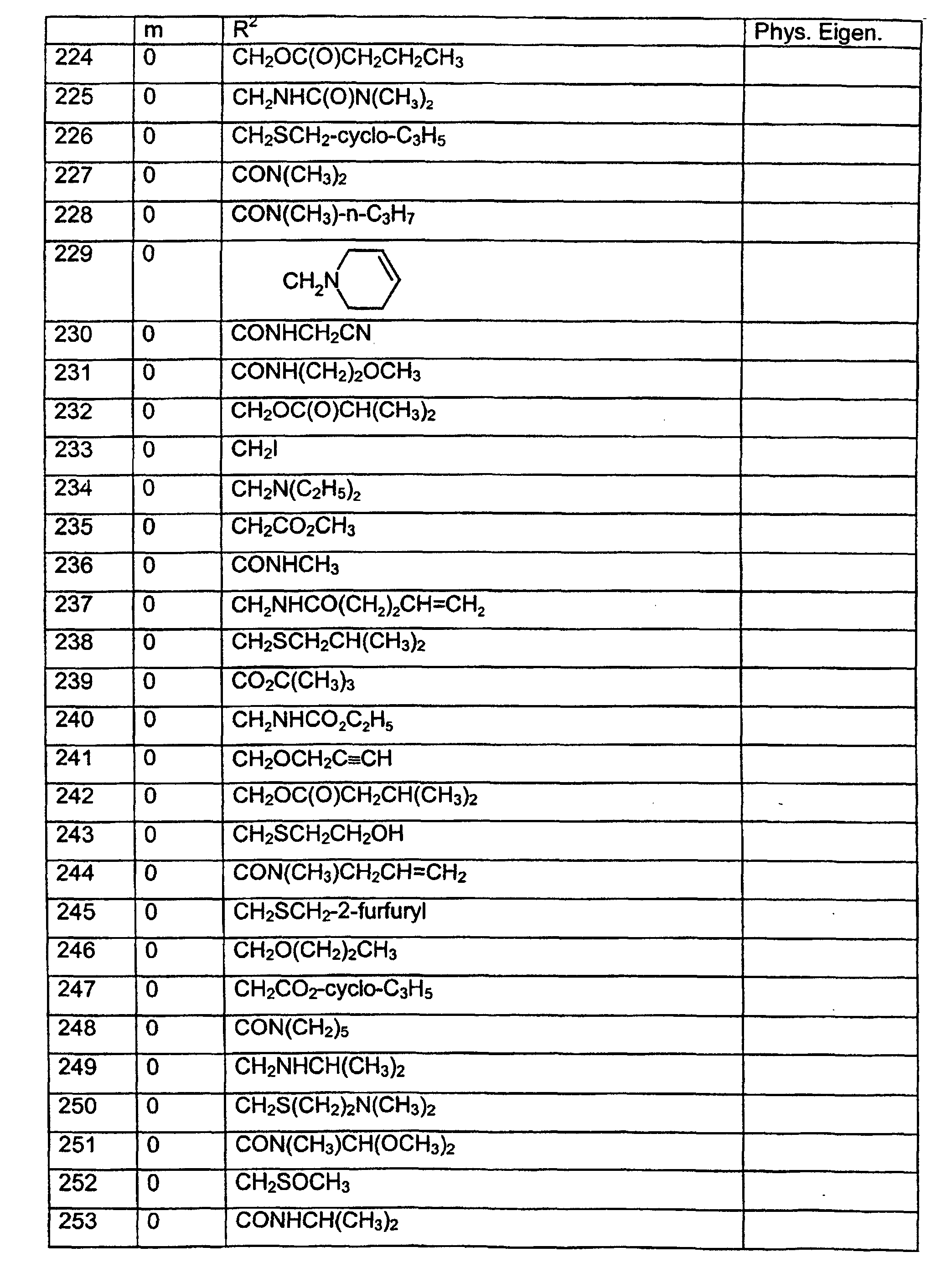

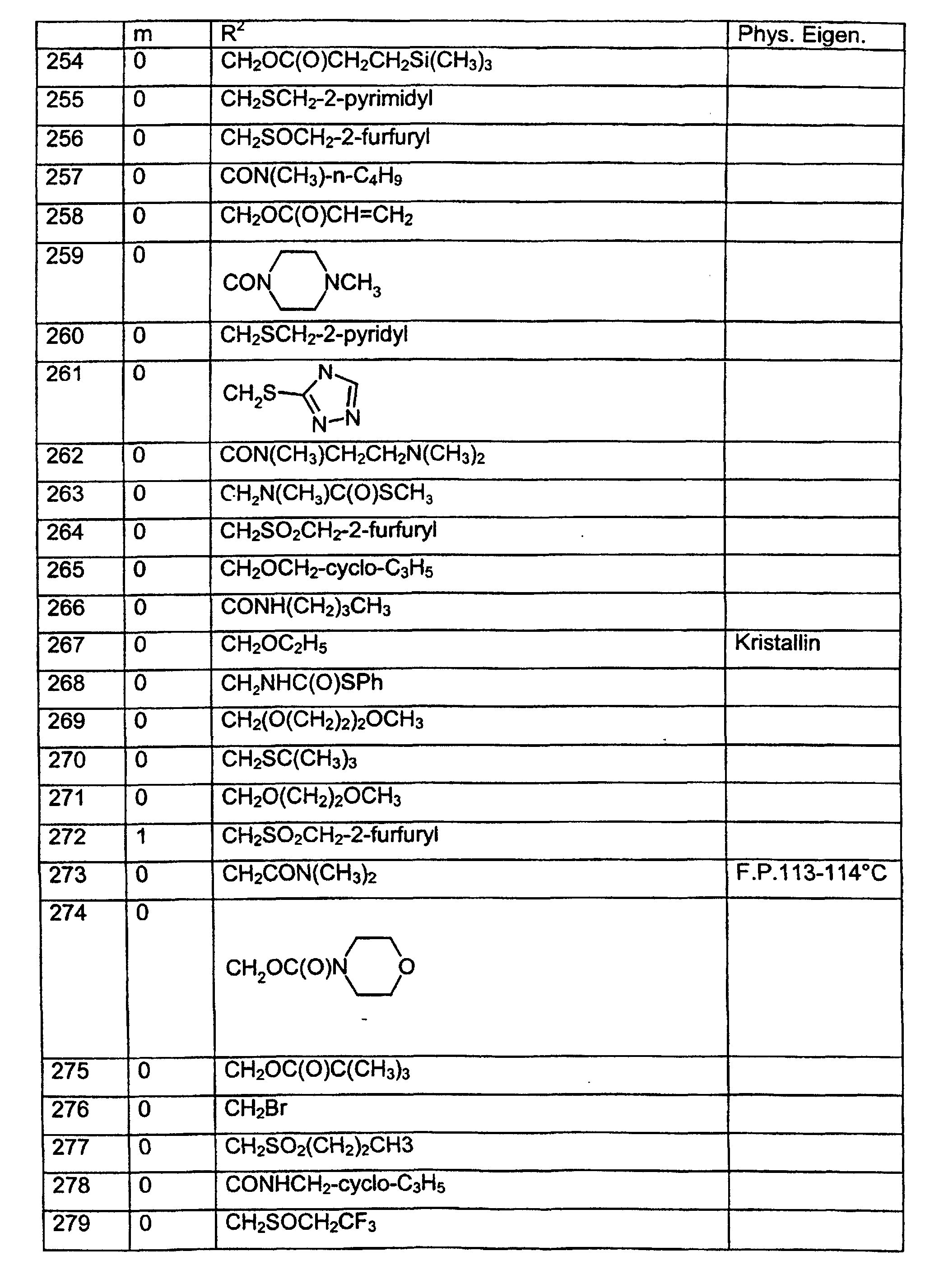

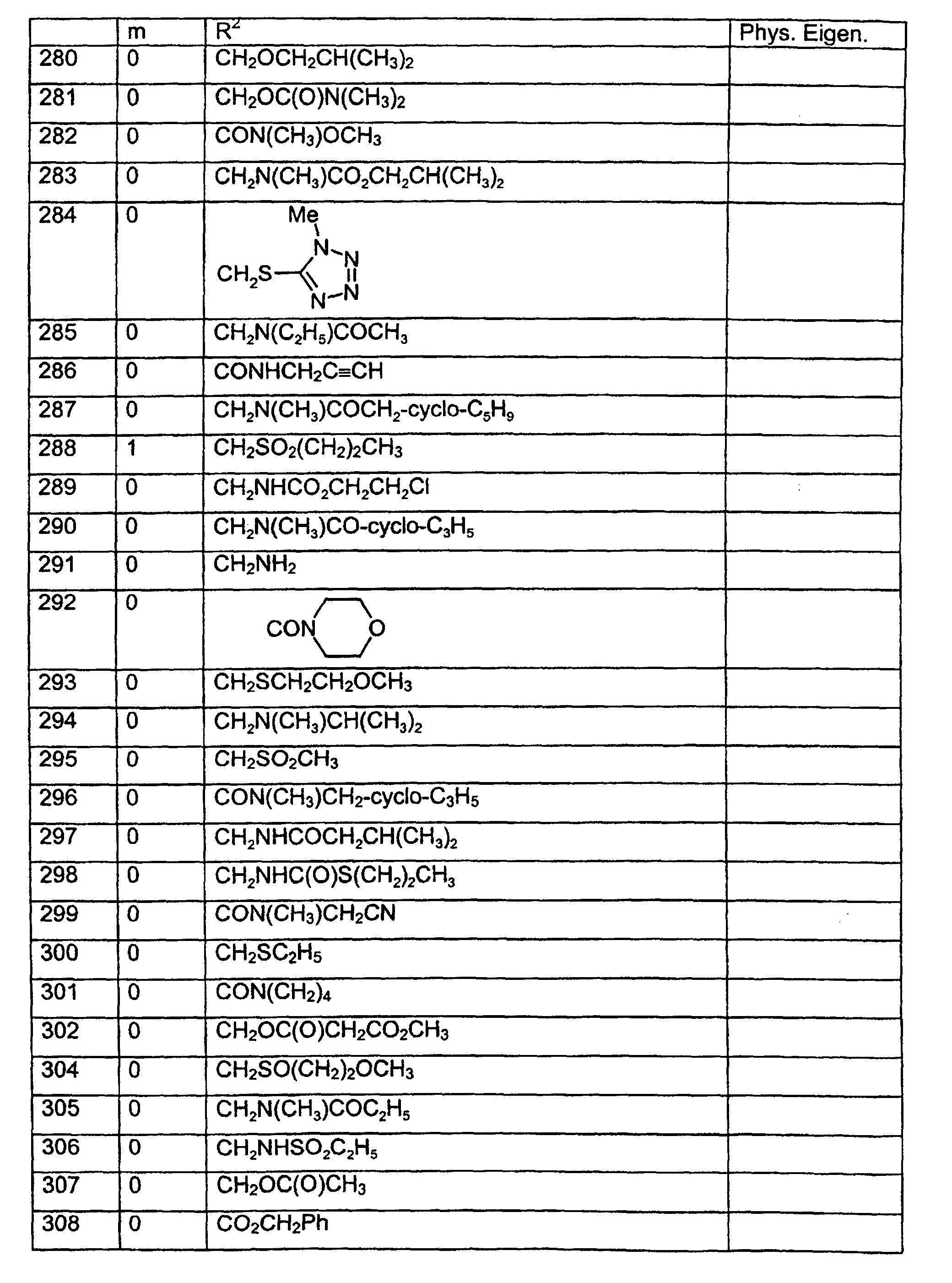

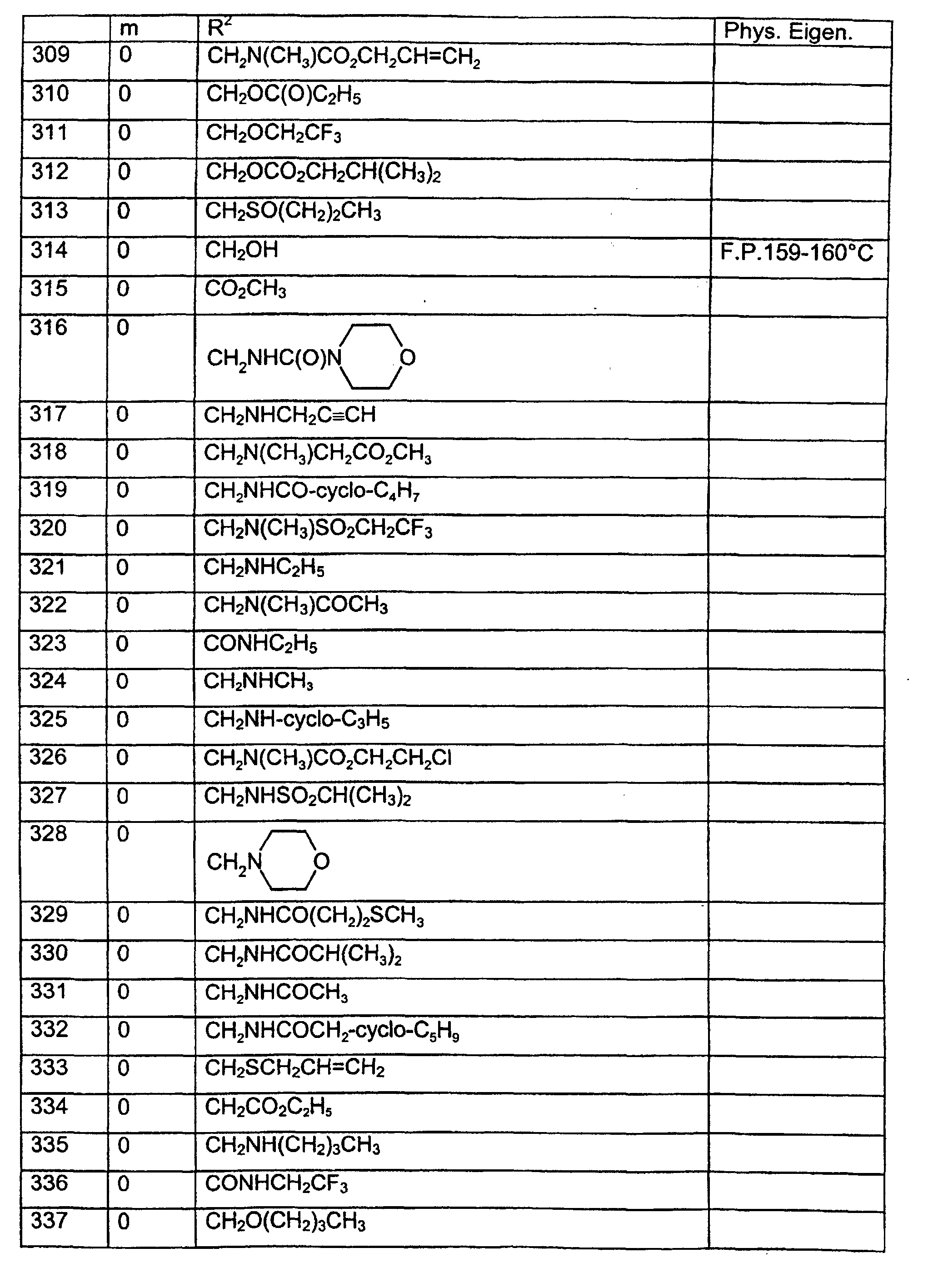

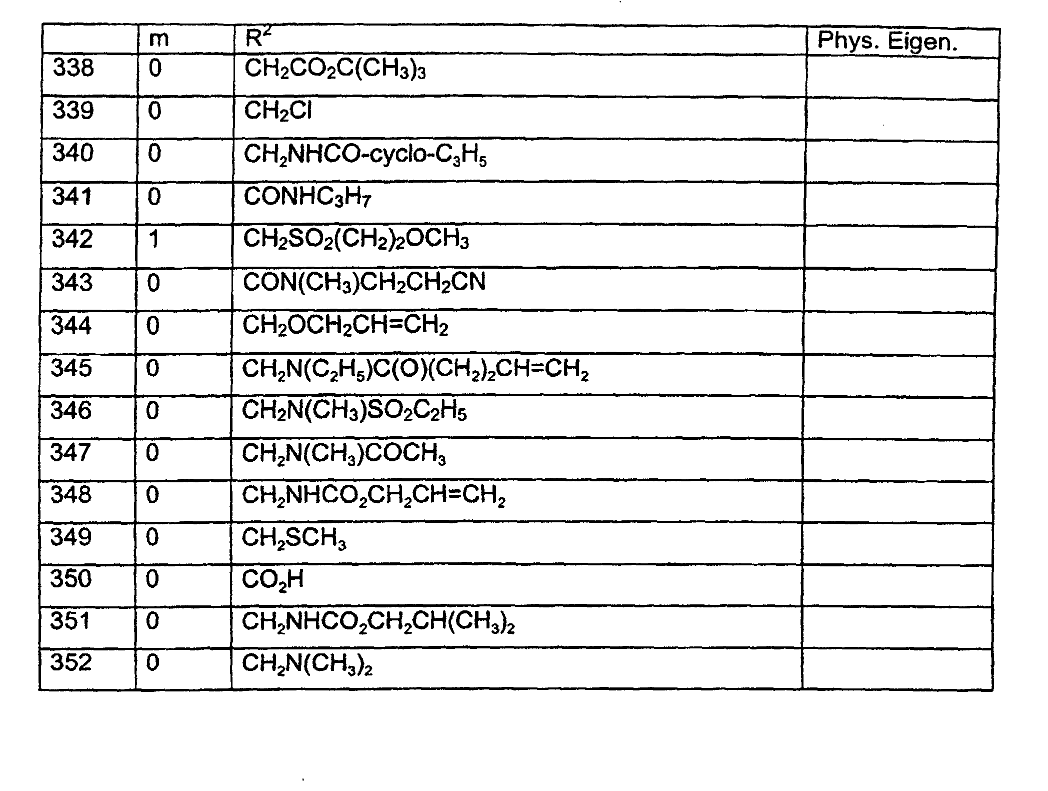

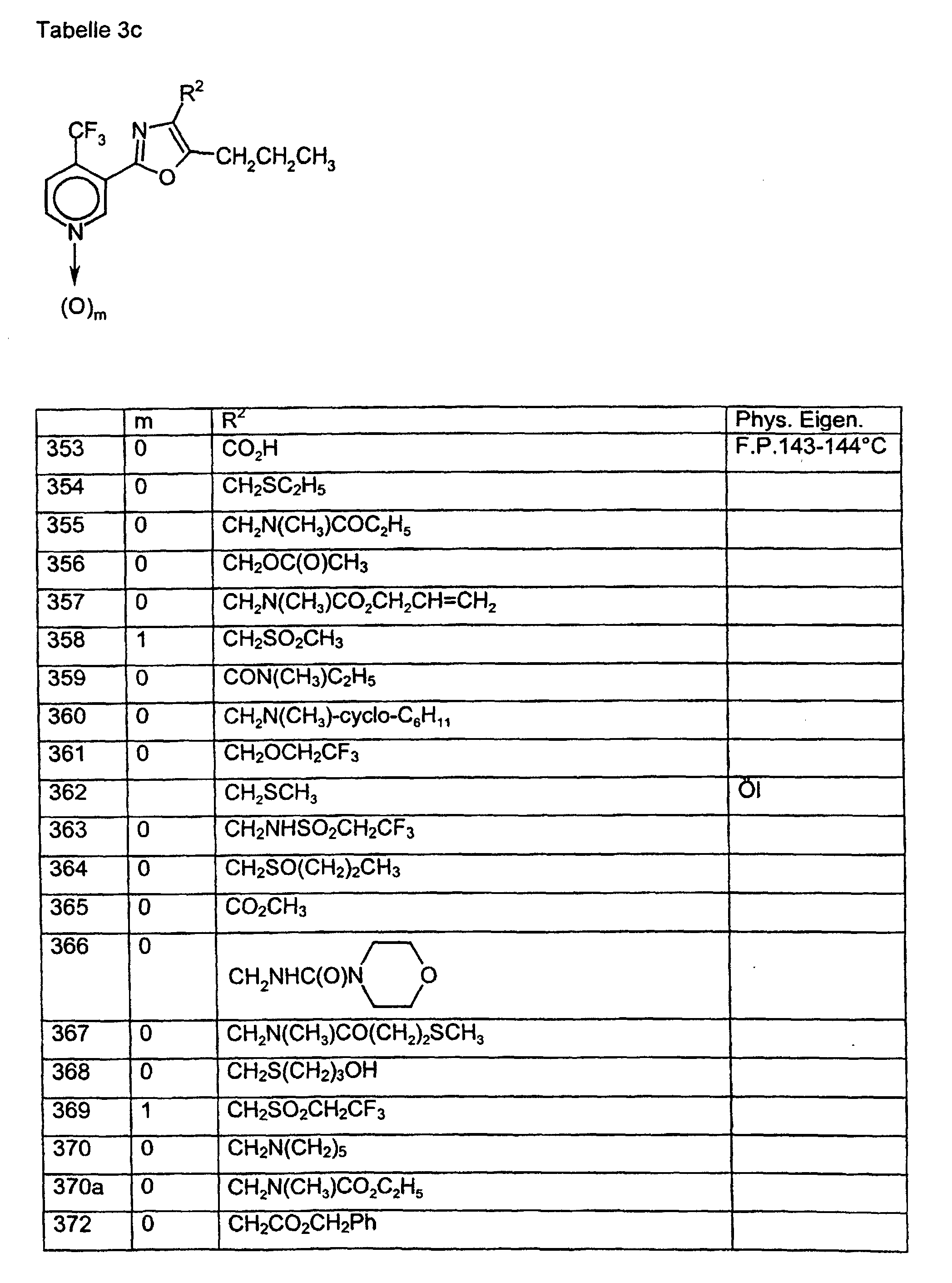

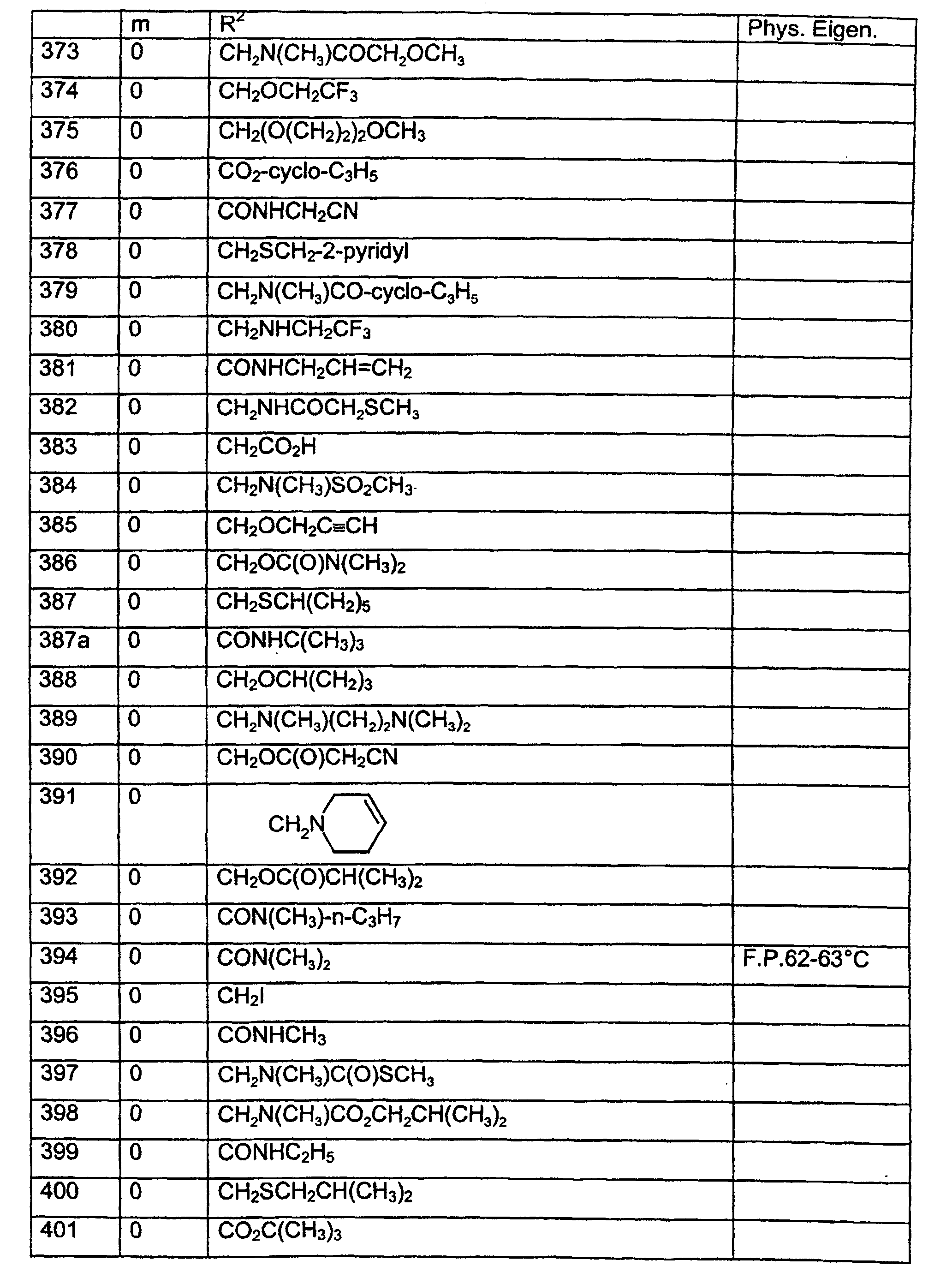

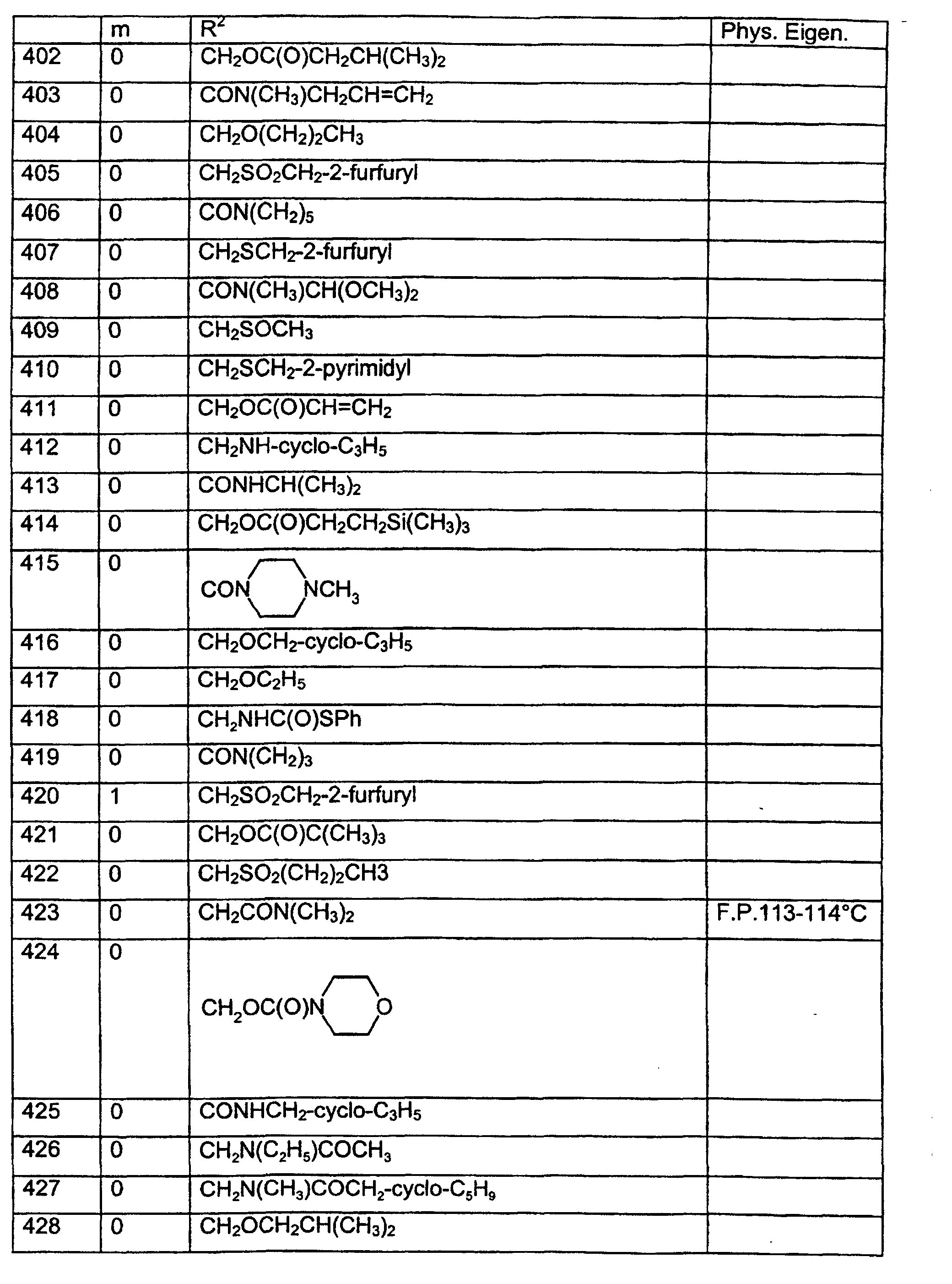

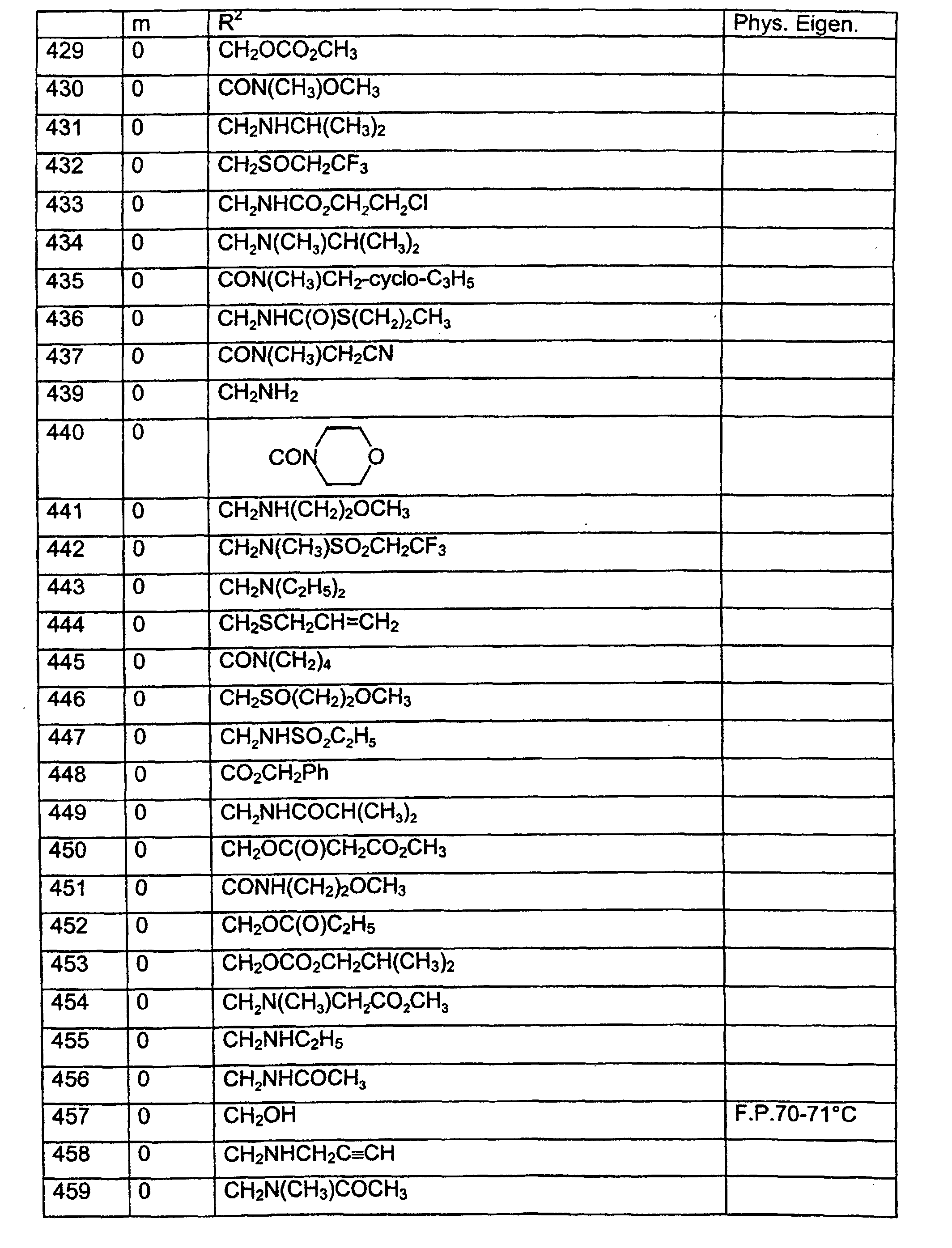

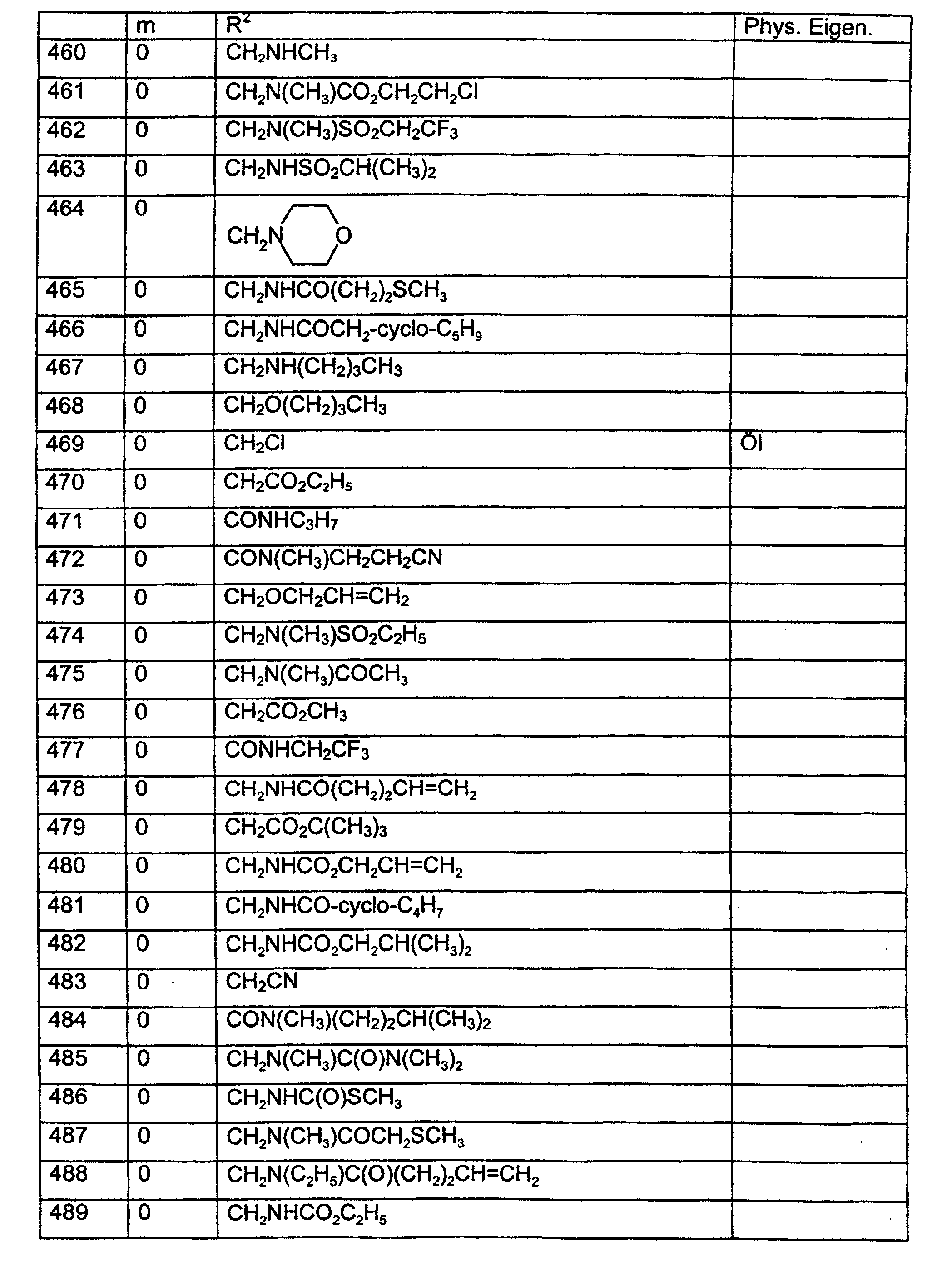

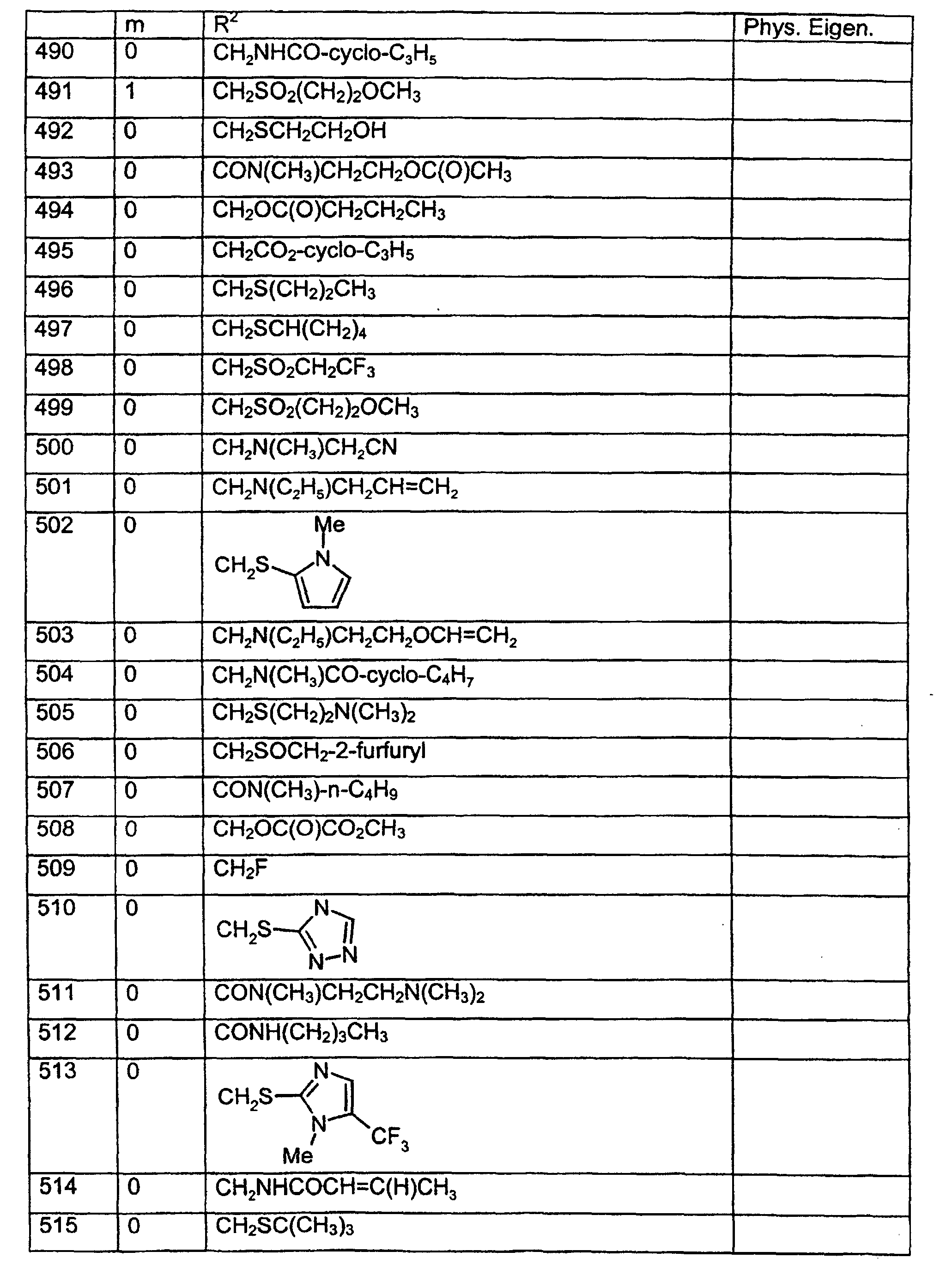

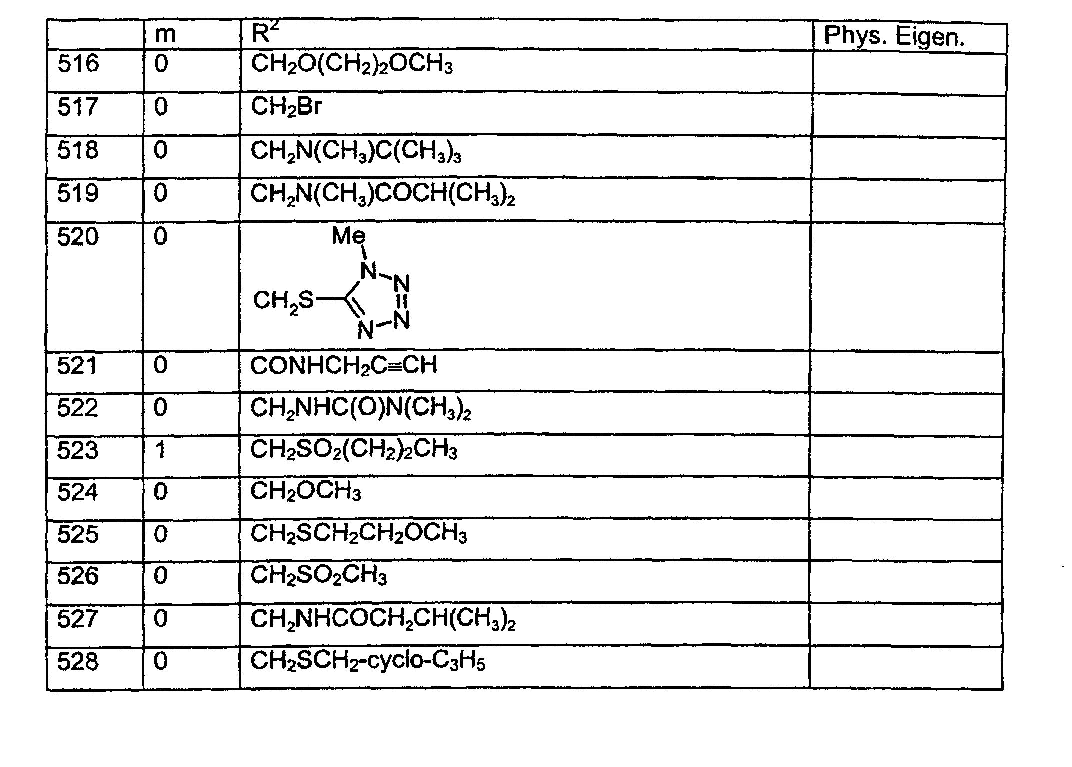

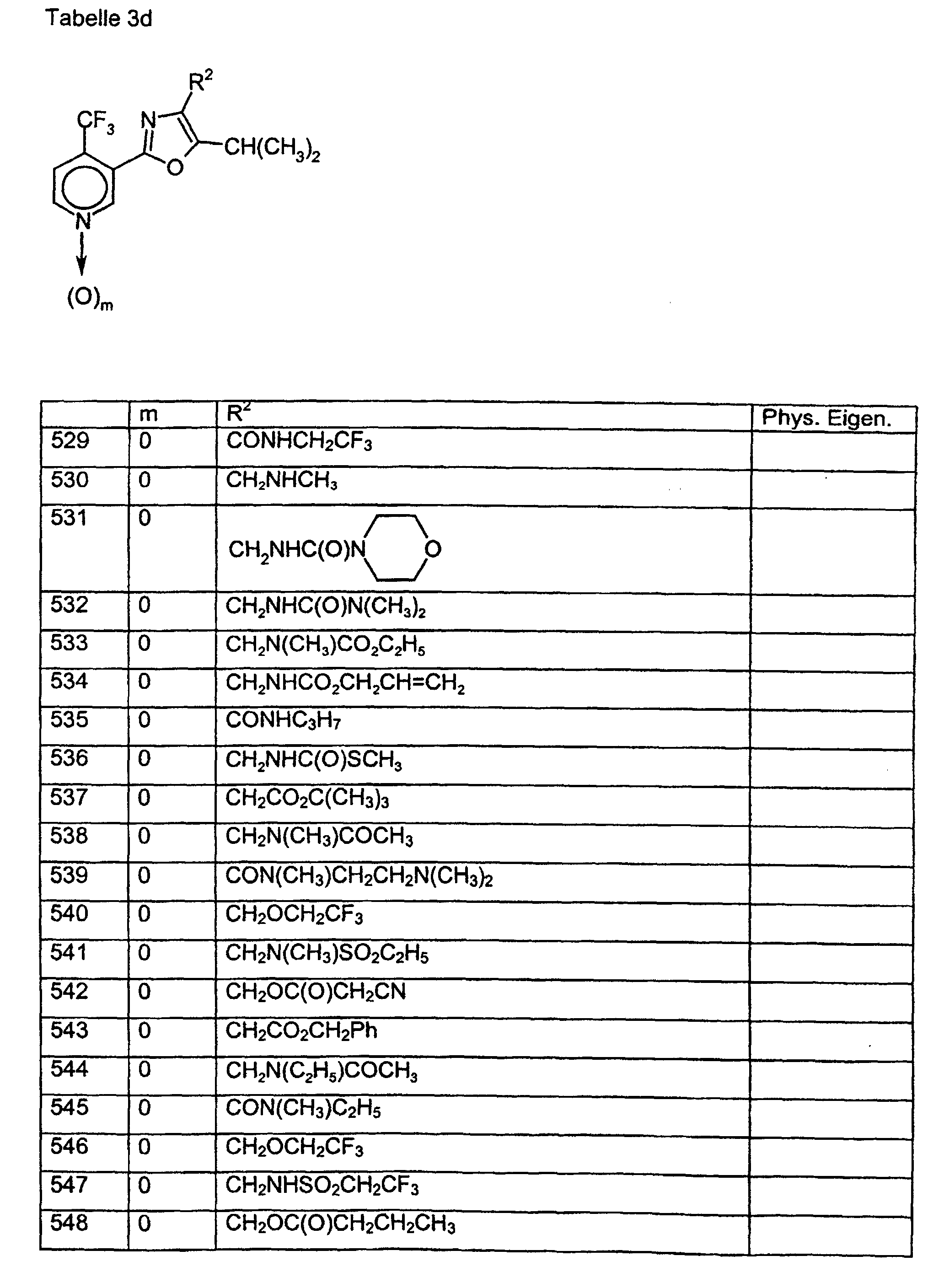

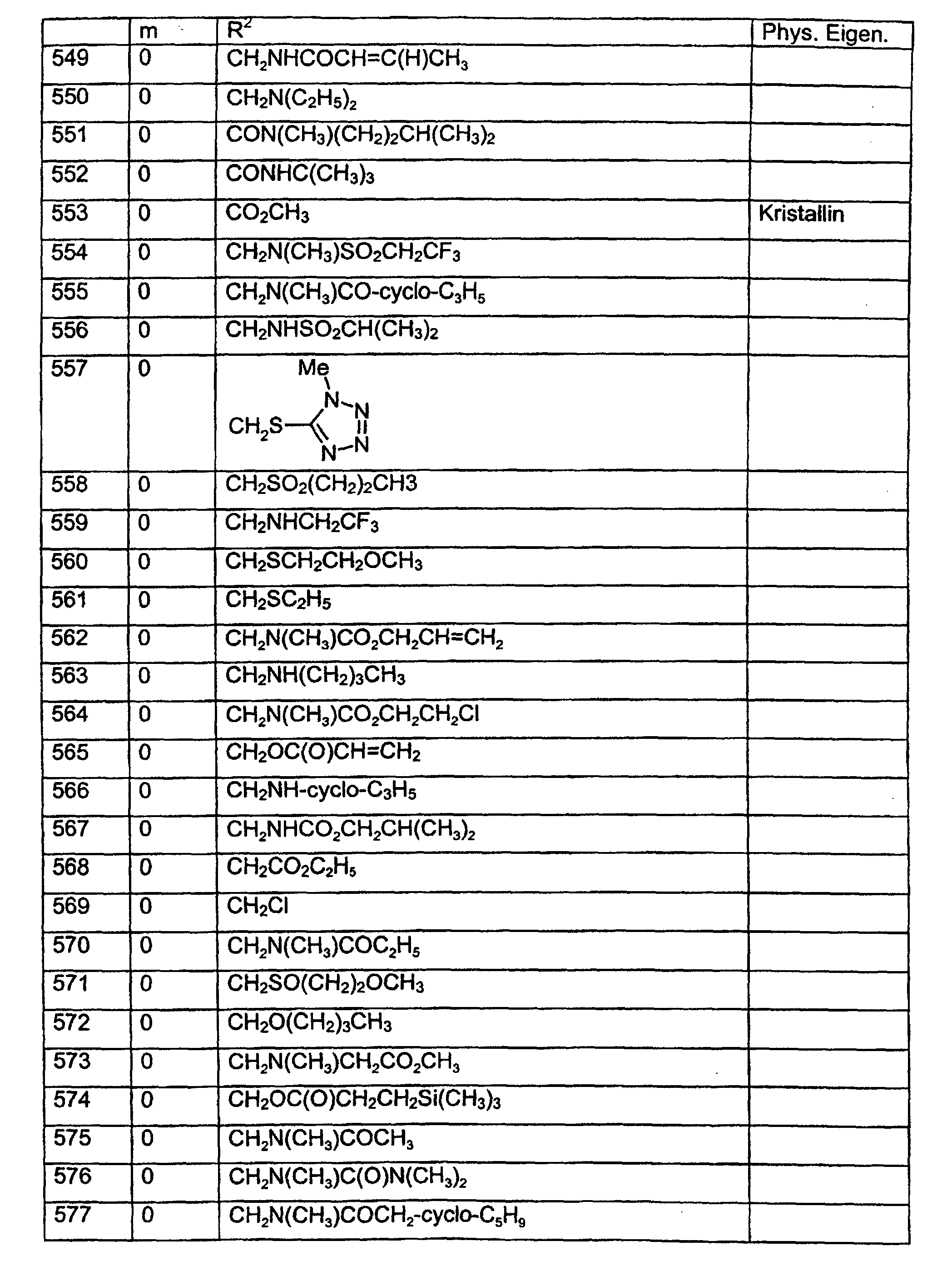

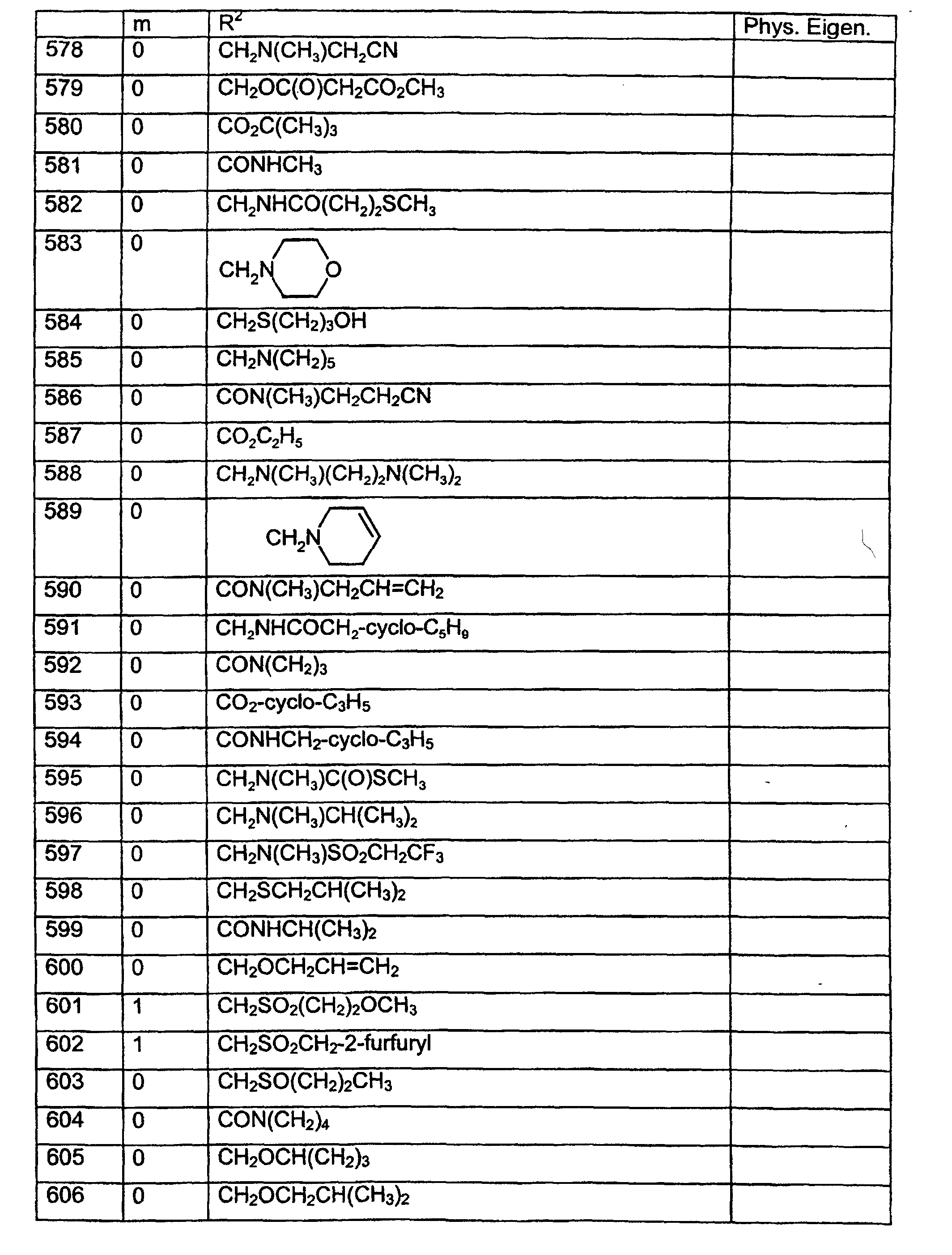

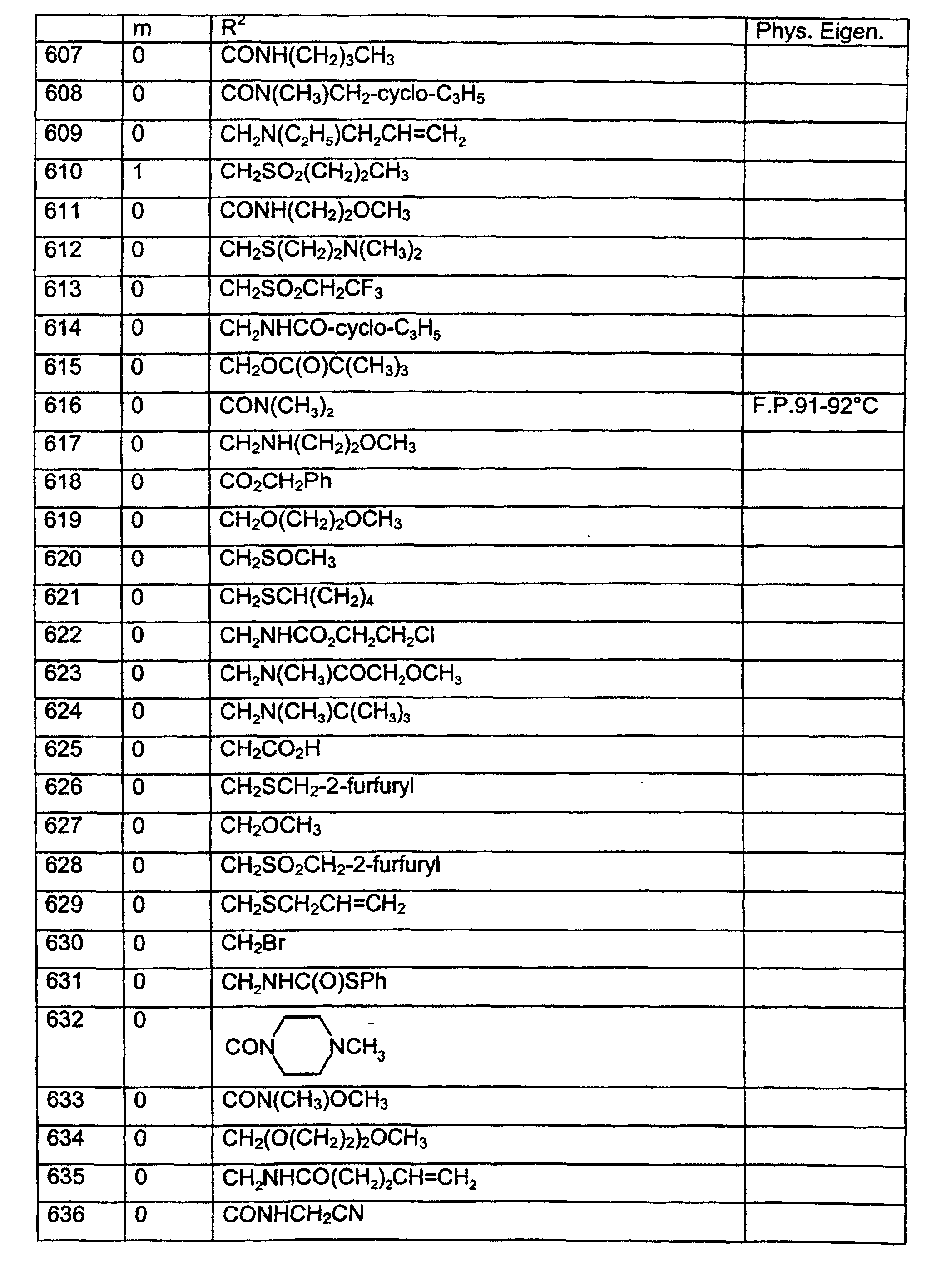

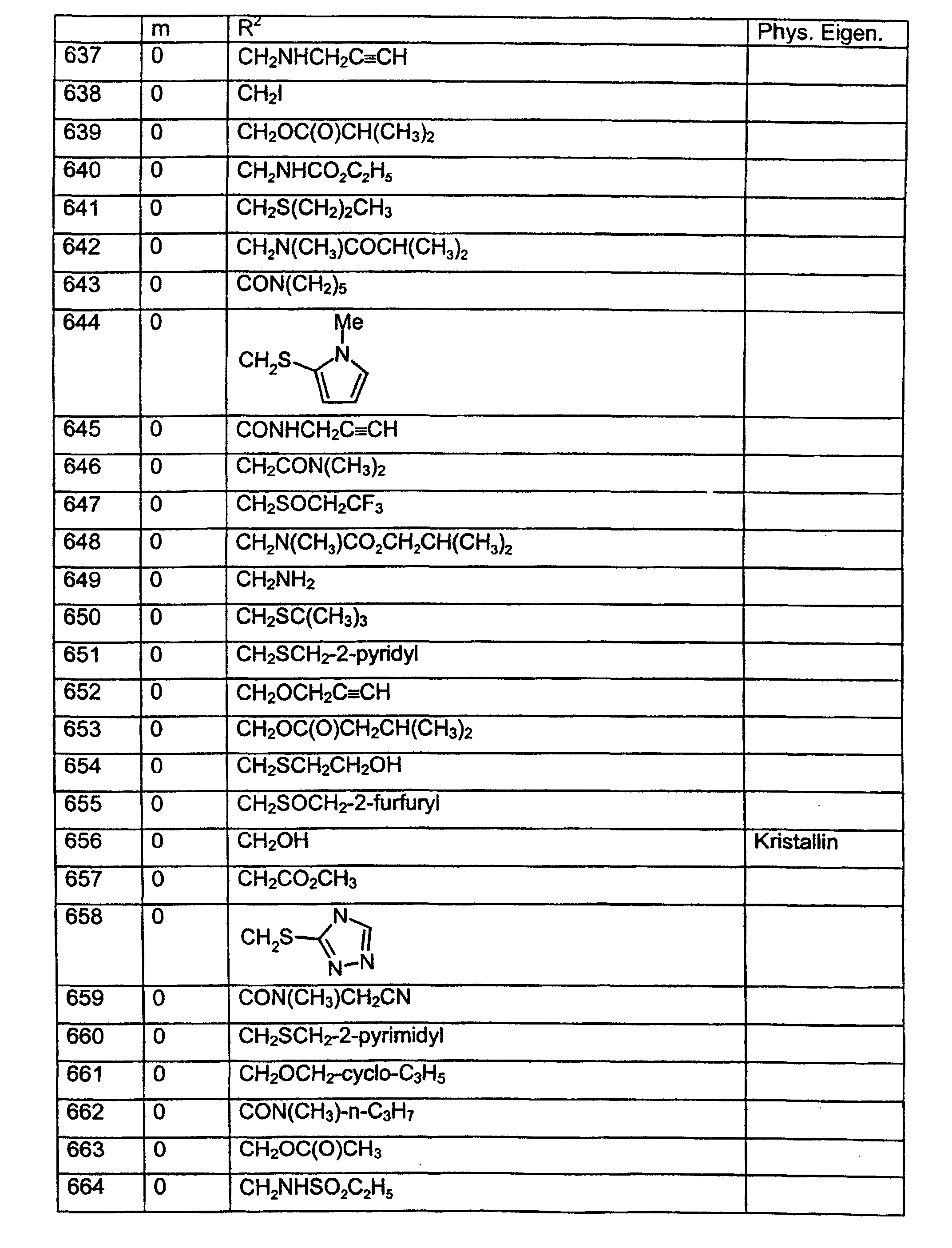

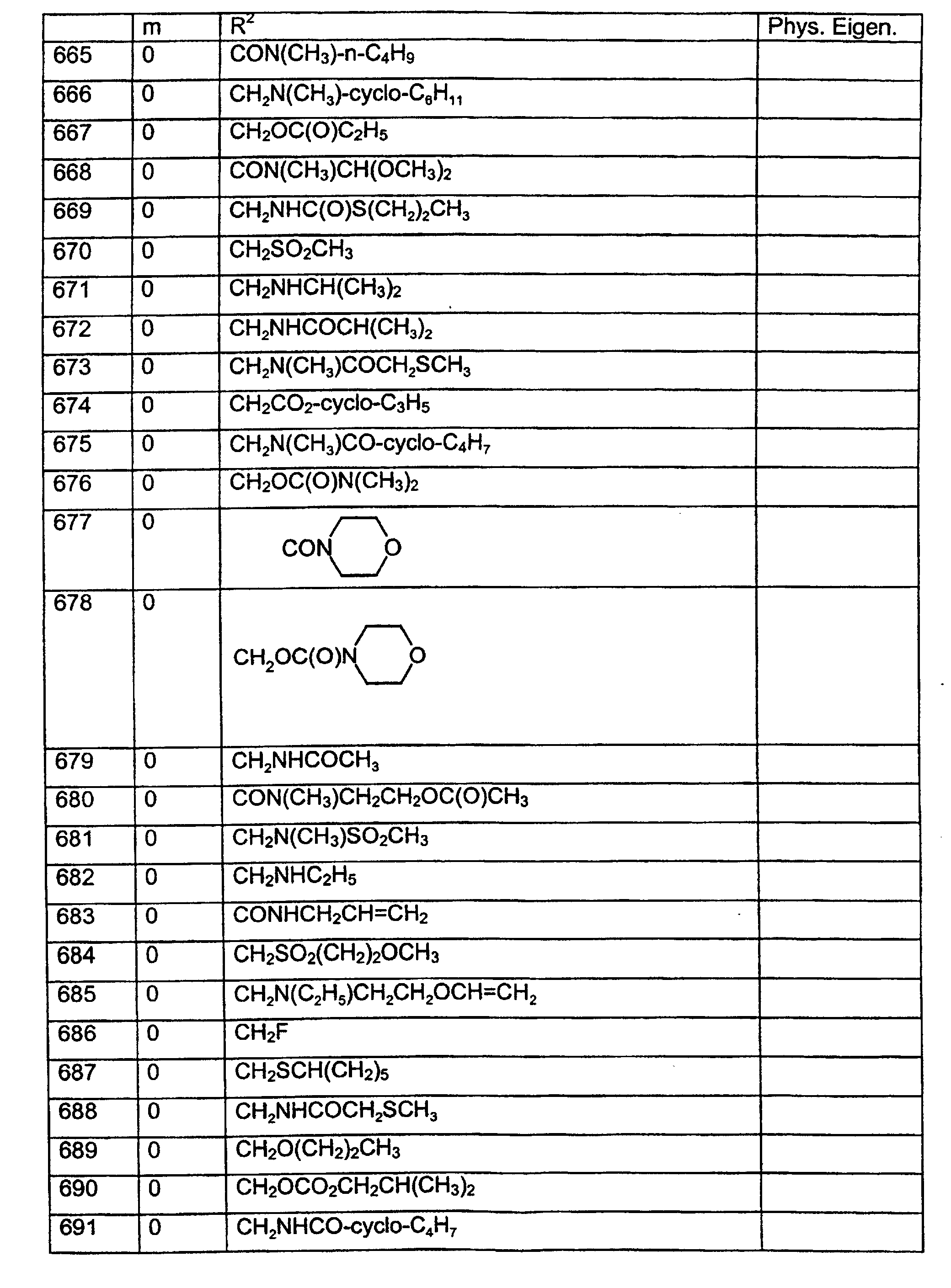

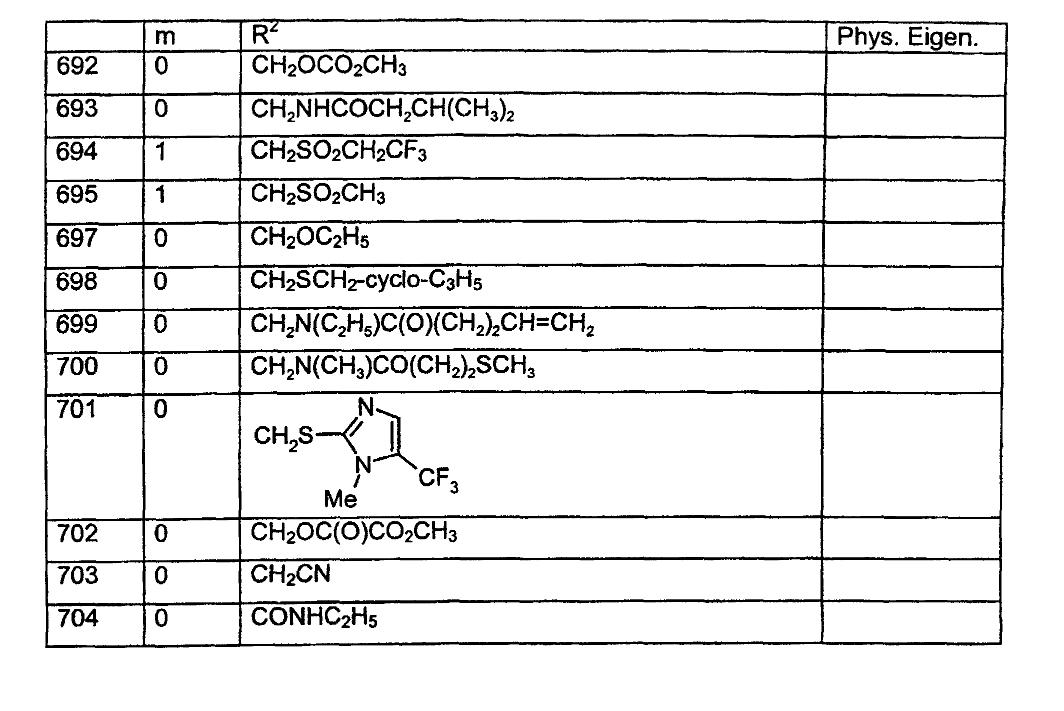

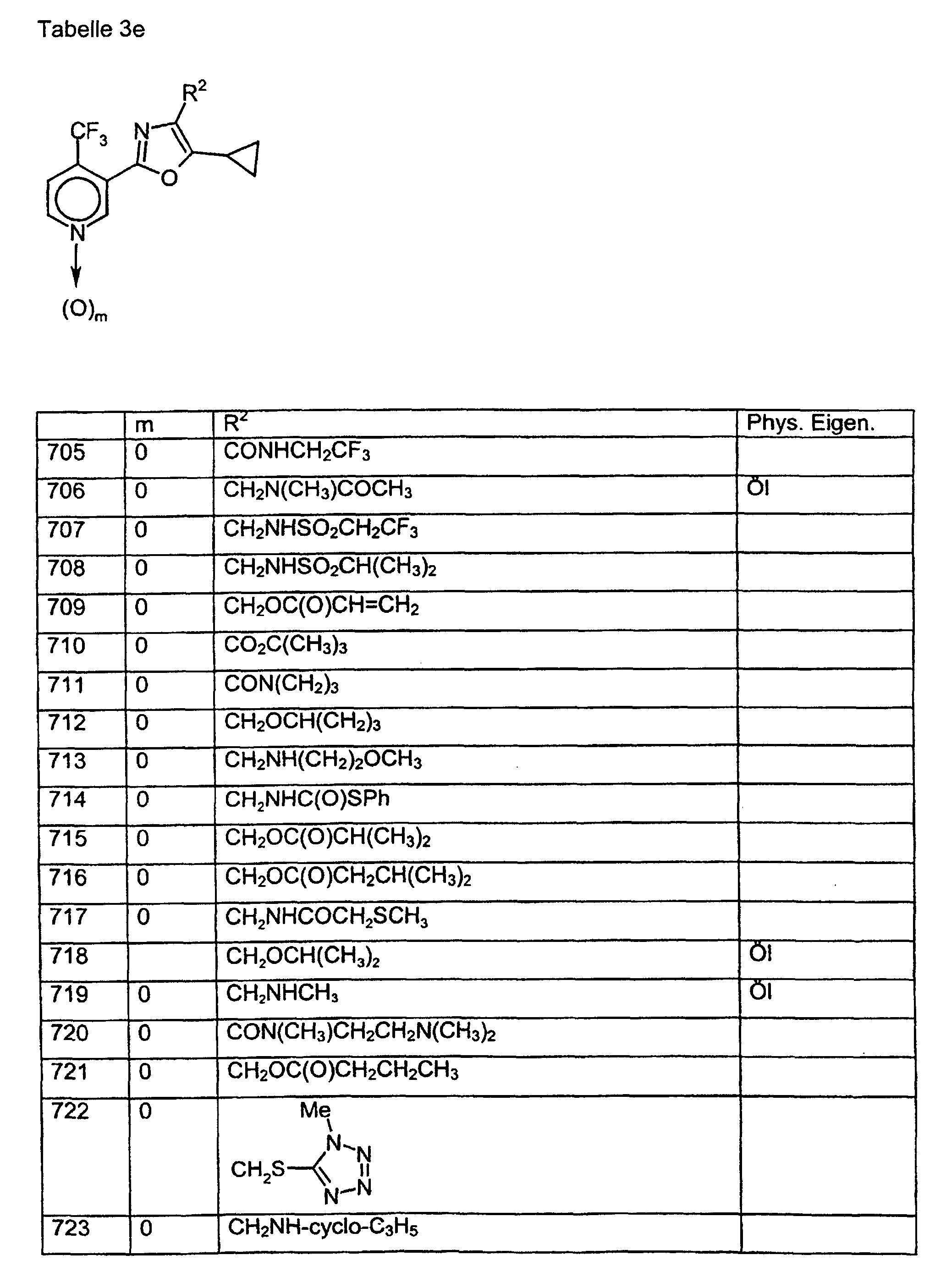

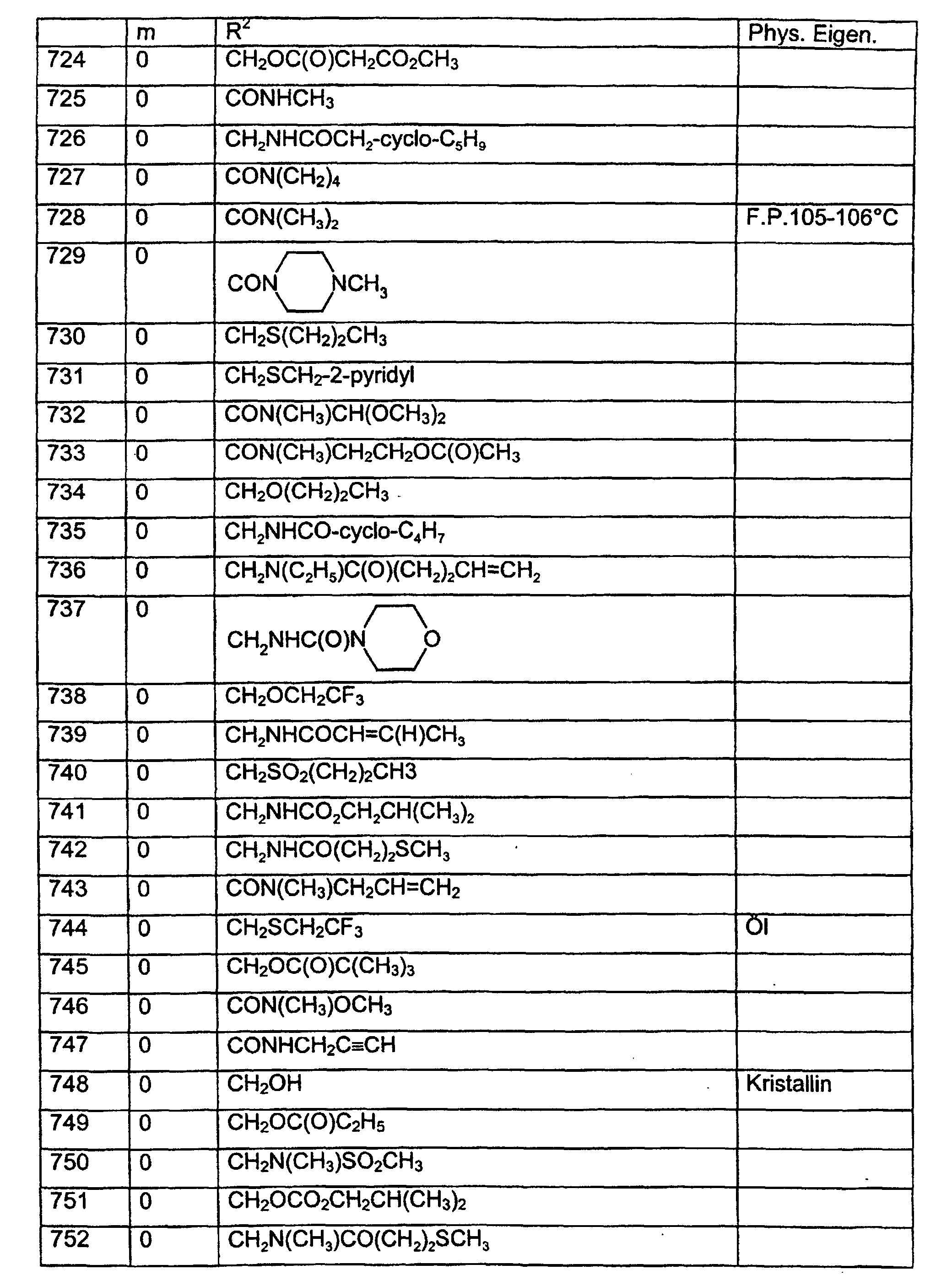

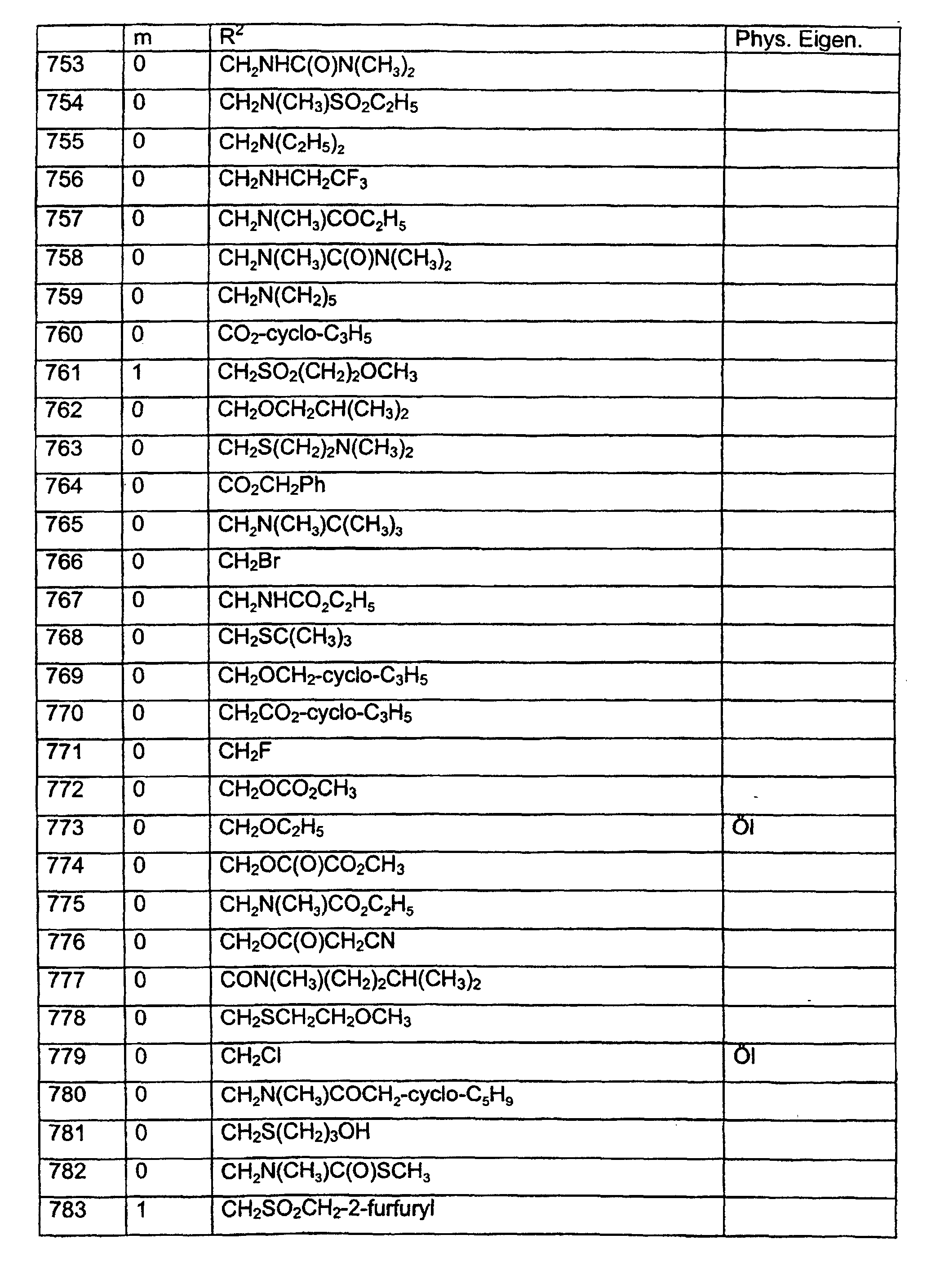

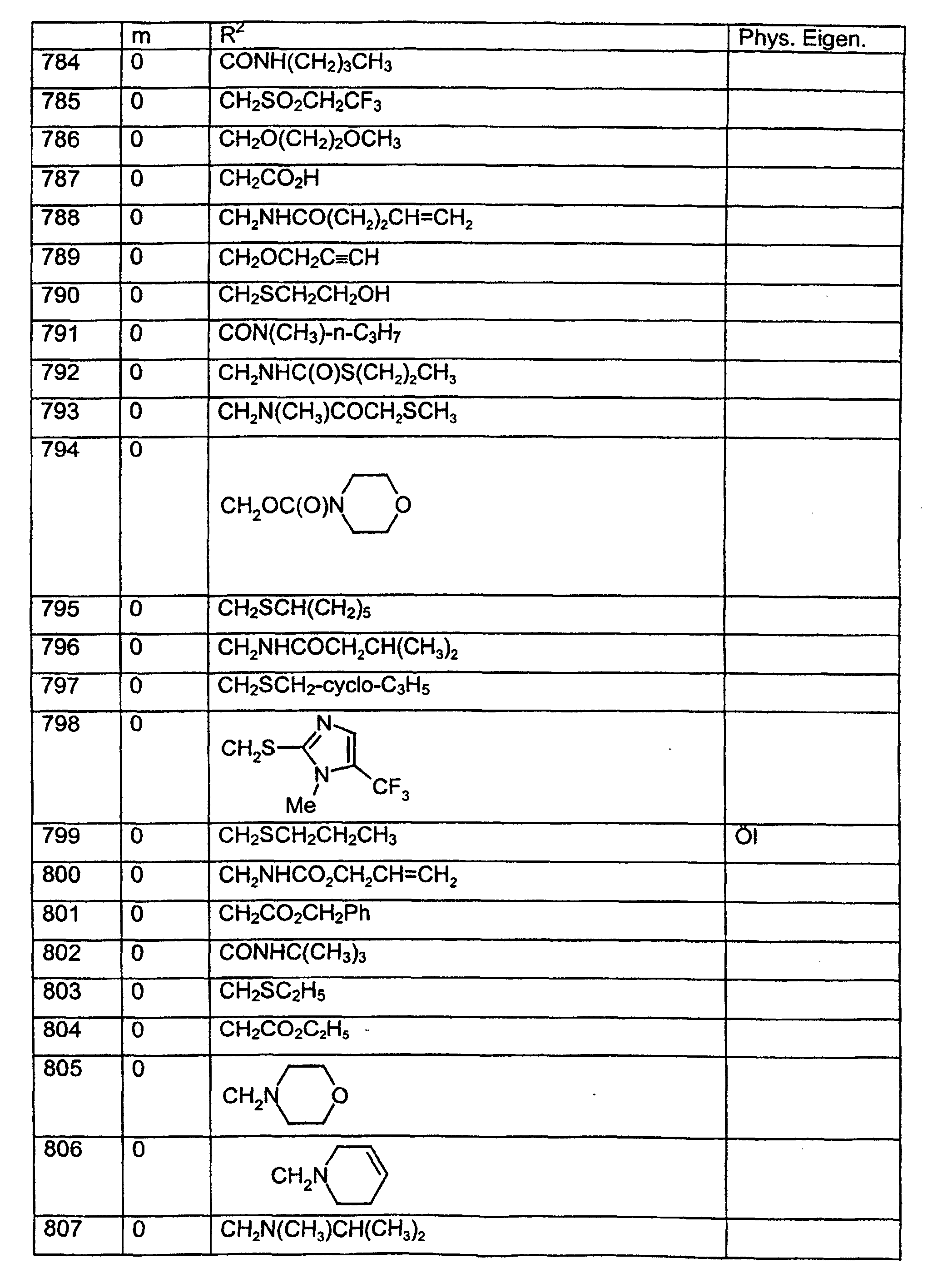

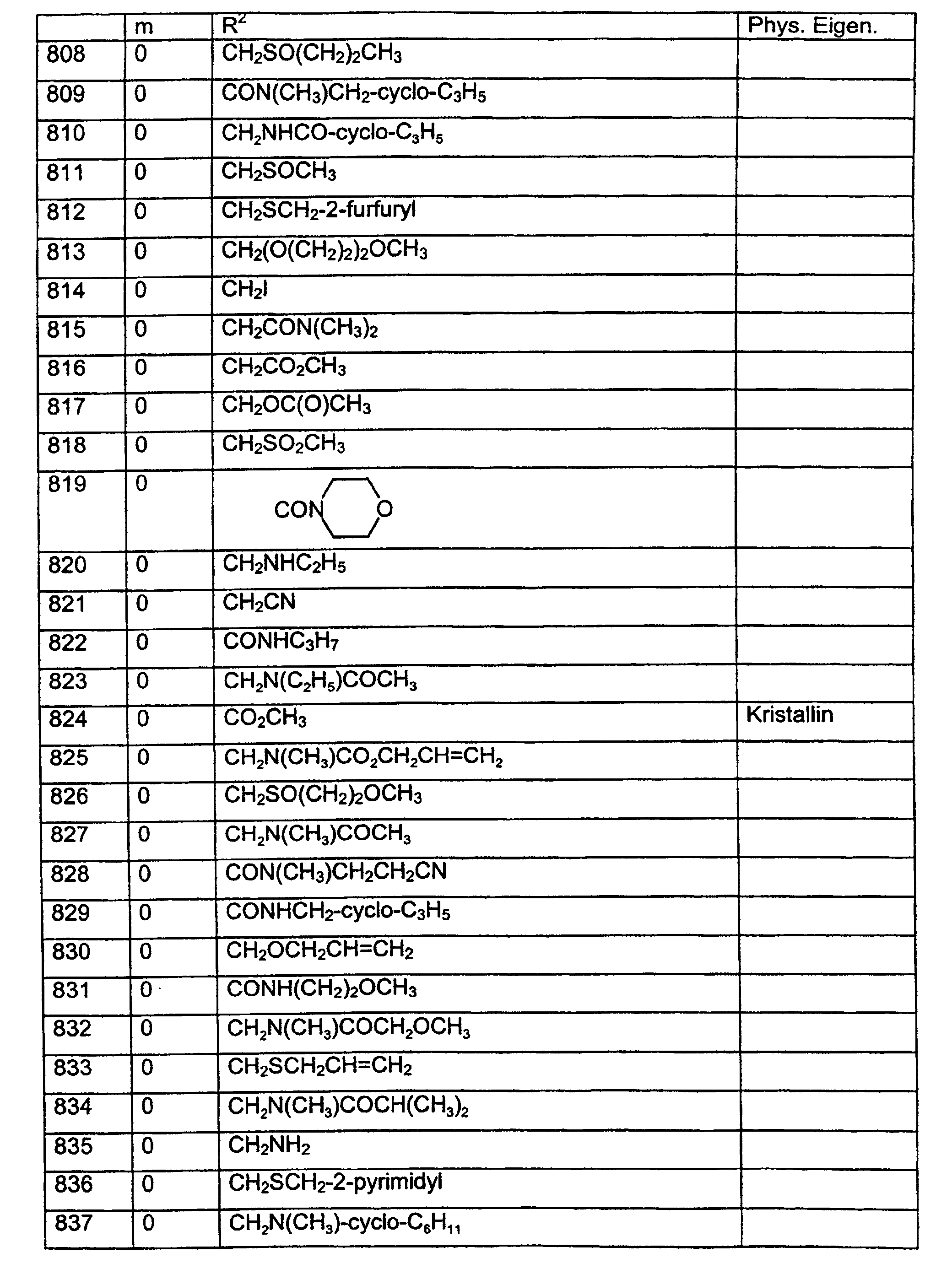

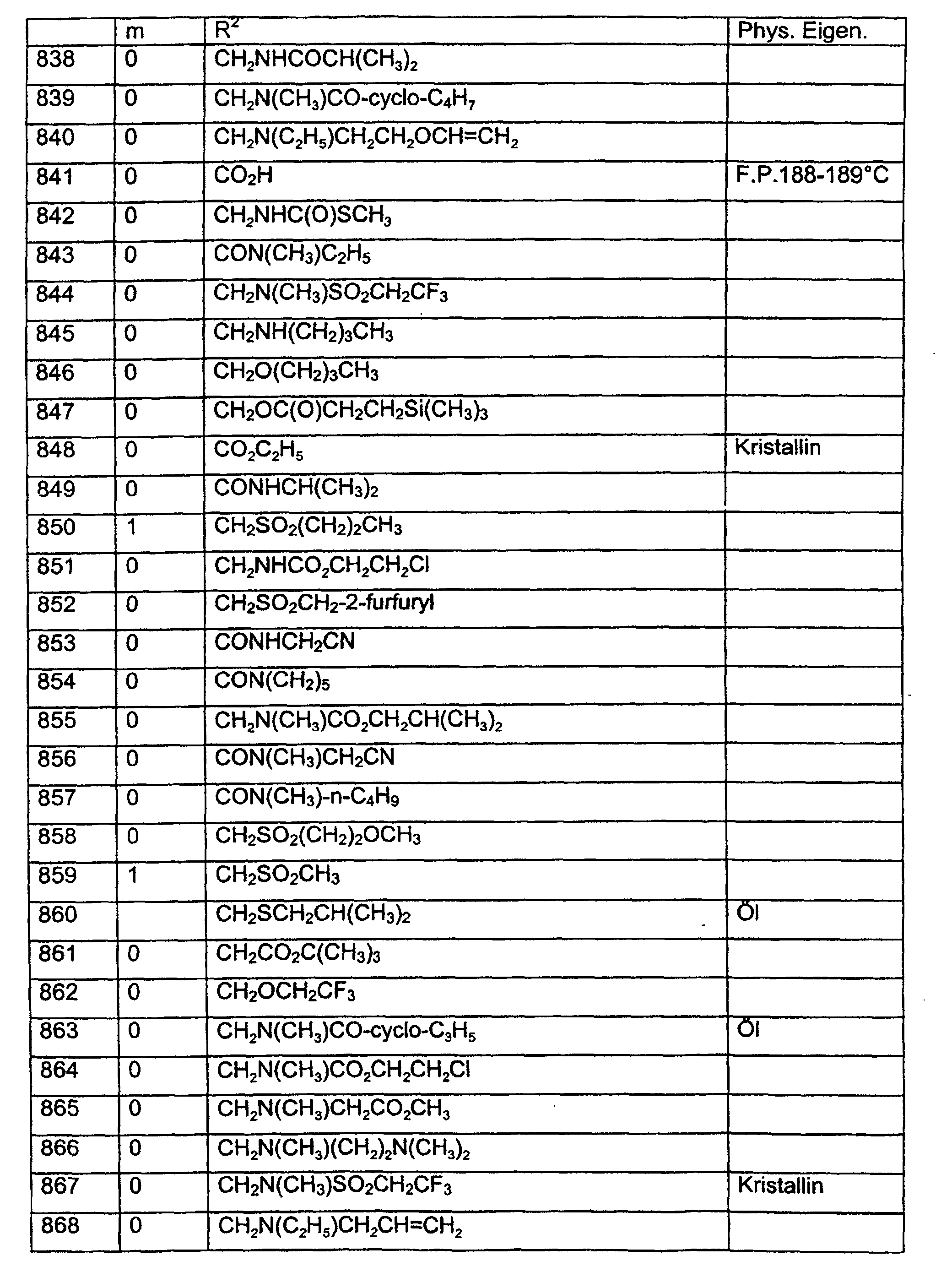

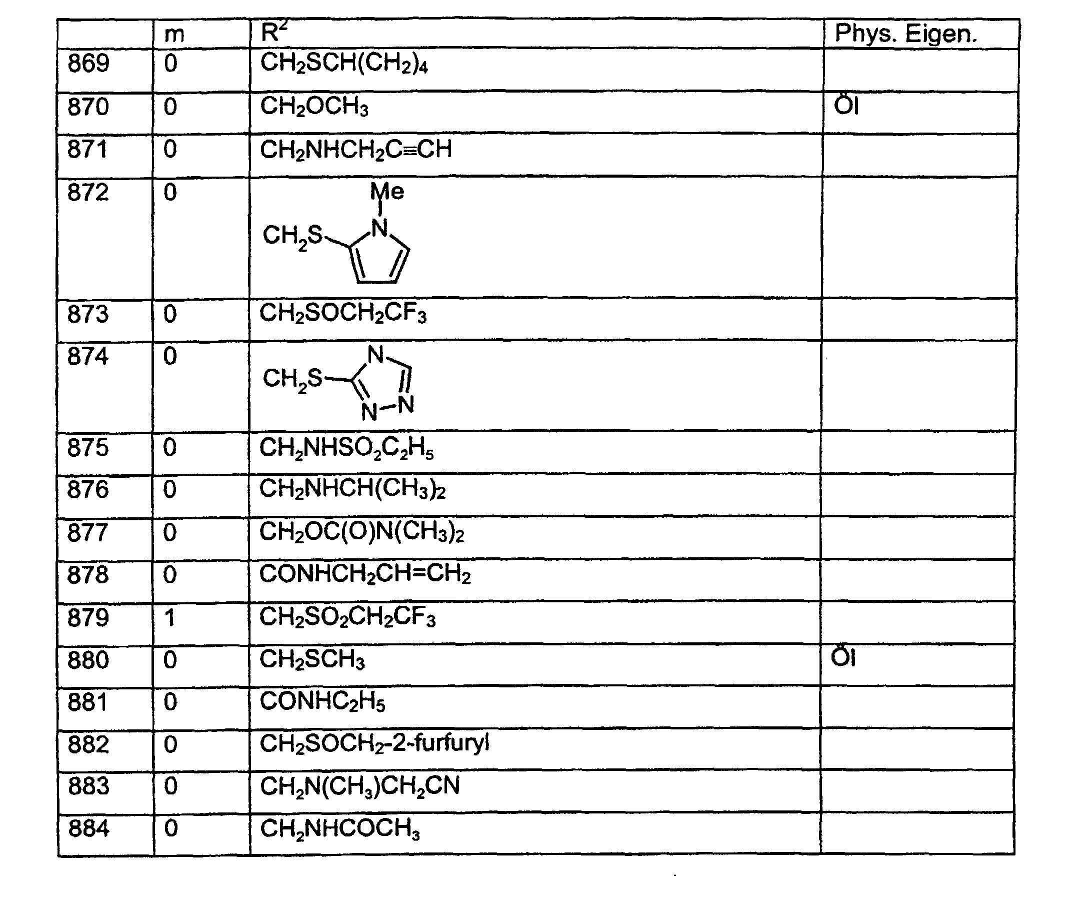

### B. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.
b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat stellt man her, indem man 40 Gew.-teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.
d) Ein emulgierbares Konzentrat läßt sich herstellen aus 15 Gew.-Teilen Wirkstoff, 75 Gew.-Teilen Cyclohexan als Lösungsmittel und 10 Gew.-Teilen oxethyliertem Nonylphenol (10 EO) als Emulgator.
e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

### C. Biologische Beispiele

### Beispiel 1

Angekeimte Ackerbohnen-Samen (Vicia faba) mit Keimwurzeln wurden in mit Leitungswasser gefüllte Braunglasfläschchen übertragen und anschließend mit ca. 100 schwarzen Bohnenblattläusen (Aphis fabae) belegt. Pflanzen und Blattläuse wurden dann für 5 Sekunden in eine wäßrige Lösung des zu prüfenden und formulierten Präparates getaucht. Nach dem Abtropfen wurden Pflanze und Tiere in einer Klimakammer gelagert (16 Stunden Licht/Tag, 25 °C, 40-60 % RF(relative Feuchtigkeit)). Nach 3 und 6 Tagen Lagerung wurde die Wirkung des Präparates auf die Blattläuse bestimmt. Bei einer Konzentration von 300 ppm (bezogen auf den Gehalt an Wirkstoff) bewirkten die Präparate gemäß Beispiele Nr. 1/37, 1/78, 1/136, 1/94, 1/174, 1/14, 1/125, 1/187, 1/17, 1/84, 1/52, 1/82, 1/70, 3/74, 1/54, 1/81, 1/42, 3/161, 1/186 eine 90-100 %ige Mortalität der Blattläuse. (Die Numerierung der aktiven Verbindungen gibt Tabelle/Nr. in der Tabelle an).

### Beispiel 2

Die Blätter von 12 Reispflanzen mit einer Halmlänge von 8 cm wurden für 5 Sekunden in eine wäßrige Lösung des zu prüfenden und formulierten Präparates getaucht. Nach dem Abtropfen wurden die so behandelten Reispflanzen in eine Petrischale gelegt und mit ca. 20 Larven (L3-Stadium) der Reiszikadenart Nilaparvata lugens besetzt. Nach dem Verschließen der Petrischale wurde diese in einer Klimakammer gelagert (16 Stunden Licht/Tag, 25 °C, 40-60 % RF). Nach 6 Tagen Lagerung wurde die Mortalität der Zikadenlarven bestimmt. Bei einer Konzentration von 300 ppm (bezogen auf den Gehalt an Wirkstoff) bewirken die Präparate gemäß Beispiele Nr. 1/53, 1/26, 1/164, 1/174, 1/82, 1/42 eine 90-100%ige Mortalität.

### Beispiel 3

Angekeimte Ackerbohnen-Samen (Vicia faba) mit Keimwurzeln wurden in mit Leitungswasser gefüllte Braunglasfläschchen übertragen. Vier Mililiter einer wäßrigen Lösung des zu prüfenden und formulierten Präparates wurden in das Braunglasfläschchen hineinpipettiert. Anschließend wurde die Ackerbohne mit ca. 100 schwarzen Bohnenblattläusen (Aphis fabae) stark belegt. Pflanze und Tiere wurden dann in einer Klimakammer gelagert (16 Stunden Licht/Tag, 25 °C, 40-60 % RF). Nach 3 und 6 Tagen Lagerung wurde die wurzelsystemische Wirkung des Präparates auf die Blattläuse bestimmt. Bei einer Konzentration von 30 ppm (bezogen auf den Gehalt an Wirkstoff) bewirkten die Präparate gemäß Beispiele Nr. 1/53, 1/26, 1/37, 1/78, 1/136, 1/56, 1/94, 1/174, 1/14, 1/187, 1/84, 1/52, 1182, 1/70, 1/32, 3/74, 1/54, 1/81, 1/42, 3/227, 3/161 eine 90-100 %ige Mortalität der Blattläuse durch wurzelsystemische Wirksamkeit.

## Patentansprüche

1. 4-Trifluormethyl-3-oxazolylderivate der Formel (I) 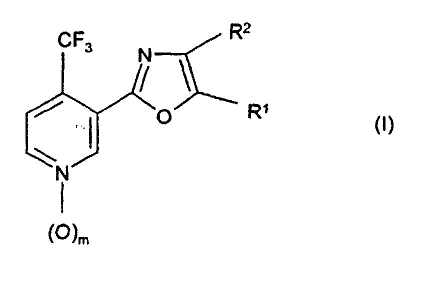 wobei die Symbole und Indizes folgende Bedeutungen haben:
m ist 0 oder 1;
R¹ und R² sind
a) H, CH₃, -C₂H₅, -CH₂-CH₂-CH₃, -CH(CH₃)₂ oder Cyclopropyl oder
b) -CH₃, -CH₂XR³, -CHY, -CO₂R⁴ oder -CONR⁵R⁶,
wobei jeweils einer der Reste R¹, R² aus der Gruppe a und der andere aus der Gruppe b ist;
X ist O, S, SO, SO₂ oder NR⁷;
Y ist O, Br₂, ((C₁-C₄)-Alkoxy)₂, ((C₁-C₄)-Alkytthio)₂, V-(CH₂)_{2 oder 3}-V, mit V = O, S, wobei ein H-Atom gegebenenfalls durch (C₁-C₄)-Alkyl ersetzt ist;
R³ ist R⁸, COR⁹, CO₂R¹⁰, CONR¹¹R¹² oder, falls X O oder NR⁷ ist, SO₂R¹³;
R⁴, R⁵, R⁶, R⁷, R⁸, R⁹, R¹⁰, R¹¹, R¹², R¹³ sind gleich oder verschieden unabhängig voneinander H, (C₁-C₁₀)-Alkyl, (C₂-C₁₀)-Alkenyl, (C₂-C₁₀)-Alkinyl, (C₃-C₈)-Cycloalkyl, (C₄-C₈)-Cycloalkenyl, (C₆-C₈)-Cycloalkinyl, Aryl oder Heterocyclyl, wobei jede der acht letztgenannten Gruppen gegebenenfalls ein oder mehrfach substituiert ist, und wobei gegebenenfalls jeweils R⁵ und R⁶ sowie R¹¹ und R¹² zusammen-(CH₂)₄-, -(CH₂)₅-, -(CH₂)₂-O-(CH₂)₂- oder -(CH₂)₂-NR⁴-(CH₂)₂- sind;
mit der Maßgabe, daß die Verbindungen, in denen
R¹ = CO₂C₂H₅ und R² = H,
R¹ = H und R² = CH₂NHC₆H₅,
R¹ = CH₃ und R² = CO₂H,
R¹ = CH₃ und R² = CO₂C₂H₅,
R¹ = CH₃ und R² = CON(CH₃)₂,
R¹ = CH(CH₃)₂ und R² = CO₂H,
R¹ = CH(CH₃)₂ und R² = CO₂C₂H₅
bedeuten, ausgenommen sind.

2. 4-Trifluormethyl-3-oxazolylpyridine gemäß Anspruch 1, **dadurch gekennzeichnet, daß** R¹ H und R² einen Rest aus der Gruppe b bedeutet.

3. 4-Trifluormethyl-3-oxazolylpyridine nach Anspruch 1, **dadurch gekennzeichnet, daß** R¹ einen Rest aus der Gruppe b und R² H bedeutet.

4. 4-Trifluormethyl-3-oxazolylpyridine nach Anspruch 1, **dadurch gekennzeichnet, daß** R¹ einen Rest aus der Gruppe a, außer Wasserstoff, und R² einen Rest aus der Gruppe b bedeutet.

5. Verfahren zur Herstellung von Verbindungen der allgemeinen Formel (I) nach einem oder mehreren der Ansprüche 1 bis 4, **dadurch gekennzeichnet**, Verbindungen der Formel (II) wobei R¹ und R² die in der Formel (I) in Anspruch 1 angegebenen Bedeutungen haben, mit einem wasserentziehenden Reagenz, wie anorganische Säurechloride, anorganische Säuren und Anhydride, 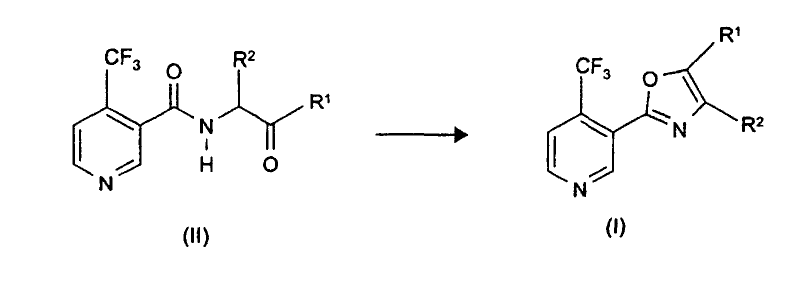 umgesetzt werden.

6. Mittel mit insektizider, akarizider und/oder nematizider Wirkung, **gekennzeichnet durch** einen Gehalt an mindestens einer Verbindung der Formel (I) nach einem oder mehreren der Ansprüche 1 bis 4.

7. Mittel mit insektizider, akarizider und/oder nematizider Wirkung nach Anspruch 6 in Mischung mit Träger- und/oder oberflächenaktiven Stoffen.

8. Mittel nach Anspruch 6 oder 7, **dadurch gekennzeichnet, daß** es einen weiteren Wirkstoff aus der Gruppe Akarizide, Fungizide, Herbizide, Insektizide, Nematizide oder wachstumsregulierende Stoffe enthält.

9. Verwendung einer Verbindung nach einem oder mehreren der Ansprüche 1 bis 4 oder eines Mittels nach Anspruch 6 oder 7 zur Herstellung eines Tierarzneimittels.

10. Verfahren zur Bekämpfung von Schadinsekten, Acarina und Nematoden, **dadurch gekennzeichnet, daß** man eine wirksame Menge einer Verbindung nach einem oder mehreren der Ansprüche 1 bis 4 oder eines Mittels nach einem oder mehreren der Ansprüche 6 bis 8 auf den Ort der gewünschten Wirkung appliziert.

11. Verfahren zum Schutz von Nutzpflanzen vor der unerwünschten Einwirkung durch Schadinsekten, Acarina und Nematoden, **dadurch gekennzeichnet, daß** mindestens eine der Verbindungen nach einem oder mehreren der Ansprüche 1 bis 4 oder ein Mittel nach einem oder mehreren der Ansprüche 6 bis 8 zur Behandlung des Nutzpflanzen-Saatgutes verwendet wird.

12. Verwendung von Verbindungen nach einem oder mehreren der Ansprüche 1 bis 4 oder eines Mittels nach einem oder mehreren der Ansprüche 6 bis 8 zur Bekämpfung von Schadinsekten, Acarina und Nematoden.

## Claims

1. A 4-trifluoromethyl-3-oxazolyl derivative of the formula (I) 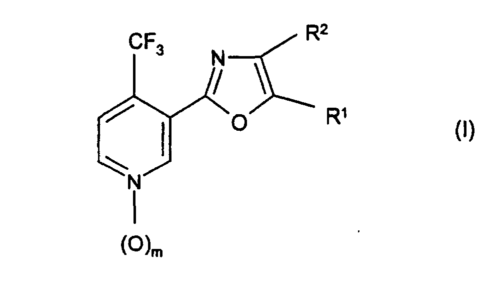 where the symbols and indices are as defined below:
m is 0 or 1;
R¹ and R² are
a) H, CH₃, -C₂H₅, -CH₂-CH₂-CH₃, -CH(CH₃)₂ or cyclopropyl or
b) -CH₃, -CH₂XR³, -CHY, -CO₂R⁴ or -CONR⁵R⁶,
where in each case one of the radicals R¹,R² is of the group a and the other is of the group b;
X is O, S, SO, SO₂ or NR⁷;
Y is O, Br₂, ((C₁-C₄)-alkoxy)₂, ((C₁-C₄)-alkylthio)₂, V-(CH₂)₂ₒᵣ₃-V, where V=O, S, where an H atom is optionally replaced by (C₁-C₄) alkyl;
R³ is R⁸, COR⁹, CO₂R¹⁰, CONR¹¹R¹² or, if X is O or NR⁷, is SO₂R¹³; R⁴,R⁵,R⁶,R⁷,R⁸,R⁹,R¹⁰,R¹¹,R¹²,R¹³ are identical or different and are independently of one another H, (C₁-C₁₀)-alkyl, (C₂-C₁₀)-alkenyl, (C₂-C₁₀)-alkynyl, (C₃-C₈)-cycloalkyl, (C₄-C₈)-cycloalkenyl, (C₆-C₈)-cycloalkynyl, aryl or heterocyclyl, where each of the eight last-mentioned groups is unsubstituted or mono-or polysubstituted, and where, if appropriate, in each case R⁵ and R⁶ and R¹¹ and R¹² together are -(CH₂)₄-, -(CH₂)₅-, -(CH₂)₂-O-(CH₂)₂- or -(CH₂)₂-NR⁴-(CH₂)₂-;
with the proviso, that the compounds in which
R¹ = CO₂C₂H₅ and R² = H,
R¹ = H and R² = CH₂ NHC₆H₅,
R¹ = CH₃ and R² = CO₂H,
R¹ = CH₃ and R² = CO₂C₂H₅,
R¹ = CH₃ and R² = CON(CH₃)₂,
R¹ = CH(CH₃)₂ and R² = CO₂H,
R¹ = CH(CH₃)₂ and R² = CO₂C₂H₅
are not included.

2. A 4-trifluoromethyl-3-oxazolylpyridine as claimed in claim 1, wherein R¹ is H and R² is a radical of the group b.

3. A 4-trifluoromethyl-3-oxazolylpyridine as claimed in claim 1, wherein R¹ is a radical of the group b and R² is H.

4. A 4-trifluoromethyl-3-oxazolylpyridine as claimed in claim 1, wherein R¹ is a radical of the group a, with the exception of hydrogen, and R² is a radical of the group b.

5. A process for preparing compounds of the formula (I) as claimed in one or more of claims 1 to 4, which comprises reacting compounds of formula (II) where R¹ and R² are as defined in the formula (I) in claim 1 with a dehydrating agent, such as inorganic acid chlorides, inorganic acids and anhydrides.

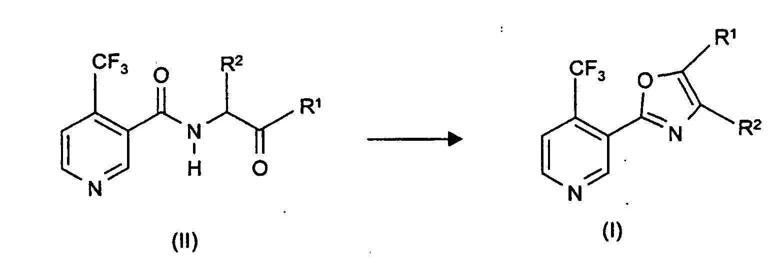

6. A composition having insecticidal, acaricidal and/or nematicidal action, which comprises at least one compound of the formula (I) as claimed in one or more of claims 1 to 4.

7. The composition having insecticidal, acaricidal and/or nematicidal action as claimed in claim 6 as a mixture with carrier substances and/or surface-active substances.

8. The composition as claimed in claim 6 or 7, which comprises a further active compound from the group of the acaricides, fungicides, herbicides, insecticides, nematicides or growth-regulating substances.

9. The use of a compound as claimed in one or more of claims 1 to 4 or a composition as claimed in claim 6 or 7 for preparing a drug for veterinary use.

10. A method for controlling harmful insects, acarids and nematodes, which comprises applying an effective amount of a compound as claimed in one or more of claims 1 to 4 or a composition as claimed in one or more of claims 6 to 8 to the site of the desired action.

11. A method for protecting useful plants against the undesirable action of harmful insects, acarids and nematodes, which comprises using at least one of the compounds as claimed in one or more of claims 1 to 4 or one composition as claimed in one or more of claims 6 to 8 for treating the seed of the useful plants.

12. The use of a compound as claimed in one or more of claims 1 to 4 or a composition as claimed in one or more of claims 6 or 8 for controlling harmful insects, acarids and nematodes.

## Revendications

1. Dérivés de 4-trifluorométhyl-3-oxazolyle de formule (I) 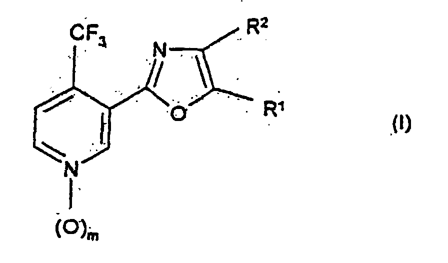 où les symboles et indices ont les significations suivantes :
m vaut 0 ou 1,
R¹ et R² représentent
a) un atome d'hydrogène, des groupes CH₃, -C₂H₅, -CH₂-CH₂-CH₃, -CH(CH₃)₂ ou cyclopropyle
ou
b) des groupes -CH₃, -CH₂XR³, -CHY, -CO₂R⁴ ou -CONR⁵R⁶,
où chaque fois un des restes R¹, R² est pris dans le groupe a et l'autre dans le groupe b ;
X représente un atome d'oxygène, un atome de soufre, des groupes SO, SO₂ ou NR⁷ ;
Y représente un atome d'oxygène, des groupes Br₂, (alkoxy en C₁-C₄)₂, (alkylthio en C₁-C₄)₂, V-(CH₂)_{2 ou 3}-V, avec V = O, S, où le cas échéant un atome d'hydrogène est remplacé par un groupe alkyle en C₁-C₄ ;
R³ représente des groupes R⁸, COR⁹, CO₂R¹⁰, CONR¹¹R¹² ou, dans le cas où X représente un atome d'oxygène ou un groupe NR⁷, représente un groupe SO₂R¹³;
R⁴, R⁵, R⁶, R⁷ R⁸, R⁹, R¹⁰, R¹¹, R¹², R¹³
représentent, identiques ou différents, indépendamment l'un de l'autre, un atome d'hydrogène, des groupes alkyle en C₁-C₁₀, alcényle en C₂-C₁₀, alcynyle en C₂-C₁₀, cycloalkyle en C₃-C₈, cycloalcényle en C₄-C₈, cycloalcynyle en C₆-C₈, aryle ou hétérocyclyle, où chacun des huit derniers groupes cités est substitué le cas échéant une ou plusieurs fois et où, le cas échéant à chaque fois R⁵ et R⁶ ainsi que R¹¹ et R¹² représentent conjointement un groupe -(CH₂)₄-, -(CH₂)₅-, -(CH₂)₂-O-(CH₂)₂- ou -(CH₂)₂-NR⁴-(CH₂)₂- ;
sous réserve que les composés, dans lesquels
R¹ = CO₂C₂H₅ et R² = H,
R¹ = H et R² = CH₂NHC₆H₅,
R¹ = CH₃ et R² = CO₂H,
R¹ = CH₃ et R² = CO₂C₂H₅,
R¹ = CH₃ et R² = CON(CH₃)₂,
R¹ = CH(CH₃)₂ et R² = CO₂H,
R¹ = CH(CH₃)₂ et R² = CO₂C₂H₅,
soient exclus.

2. 4-Trifluoro-3-oxazolylpyridine selon la revendication 1, **caractérisée en ce que** R¹ représente un atome d'hydrogène et R² un reste du groupe b.

3. 4-Trifluoro-3-oxazolylpyridine selon la revendication 1, **caractérisée en ce que** R¹ représente un reste du groupe b et R² un atome d'hydrogène.

4. 4-Trifluoro-3-oxazolylpyridine selon la revendication 1, **caractérisée en ce que** R¹ représente un reste du groupe a, à l'exception d'un atome d'hydrogène et R² représente un reste du groupe b.

5. Procédé pour la préparation de composés de formule générale (I) selon une ou plusieurs des revendications 1 à 4, **caractérisé en ce que** les composés de formule (II), où R¹ et R² possèdent les significations indiquées à la formule (I) à la revendication 1 réagissent avec un réactif déshydratant, comme des chlorures d'acides inorganiques, des acides inorganiques et des anhydrides.

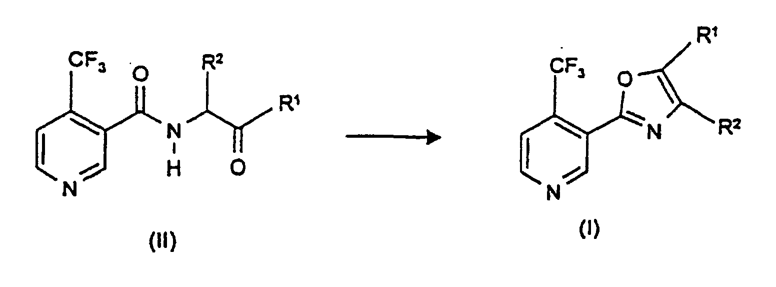

6. Agent avec un effet insecticide, acaricide et/ou nématicide, **caractérisé par** une teneur en au moins un composé de formule (I) selon une ou plusieurs des revendications 1 à 4.

7. Agent avec un effet insecticide, acaricide et/ou nématicide selon la revendication 6 en mélange avec des substances véhicules et/ou agents de surface.

8. Agent selon la revendication 6 ou 7, **caractérisé en ce qu'**il contient une autre substance active prise dans le groupe des substances acaricides, fongicides, herbicides, insecticides, nématicides ou de croissance.

9. Utilisation d'un composé selon une ou plusieurs des revendications 1 à 4 ou d'un agent selon la revendication 6 ou 7 pour la préparation d'un produit pharmaceutique vétérinaire.

10. Procédé pour la lutte contre des insectes ravageurs, des acariens et des nématodes, **caractérisé en ce que** l'on applique une quantité efficace d'un composé selon une ou plusieurs des revendications 1 à 4 ou d'un agent selon une ou plusieurs des revendications 6 à 8 à l'endroit de l'effet désiré.

11. Procédé pour la protection des plantes utiles avant l'effet non désiré des insectes ravageurs, des acariens et des nématodes, **caractérisé en ce que** l'on utilise au moins un des composés selon une ou plusieurs des revendications 1 à 4 ou un agent selon une ou plusieurs des revendications 6 à 8 pour le traitement de la semence des plantes utiles.

12. Utilisation des composés selon une ou plusieurs des revendications 1 à 4 ou d'un agent selon une ou plusieurs des revendications 6 à 8 pour la lutte contre les insectes parasites, les acariens et les nématodes.
